# EUROPEAN PATENT APPLICATION

(11) **EP 4 628 592 A1**
(43) Date of publication of application: **08.10.2025**
(21) Application number: 23903481.2
(22) Date of filing: 11.12.2023
(51) Int. Cl.: C12P 21/00, C12N 15/09, C40B 40/10

(54) **PEPTIDE PRODUCTION METHOD COMPRISING STEP FOR TRANSLATING AMINO ACID HAVING OXYGEN ATOM AT SPECIFIC POSITION**

(30) Priority: 12.12.2022 JP 2022198158
(71) Applicant: CHUGAI SEIYAKU KABUSHIKI KAISHA, Tokyo 115-8543 (JP)
(72) Inventor: TANAKA Shota, Kamakura-shi, Kanagawa 247-8530 (JP); SHINOHARA Shojiro, Kamakura-shi, Kanagawa 247-8530 (JP); NAKANO Kazuhiko, Synapse, 138623 (SG)
(74) Representative: Vossius & Partner Patentanwälte Rechtsanwälte mbB
(86) International application number: PCT/JP2023/044244
(87) International publication number: WO 2024/128195

(57) **Abstract**

A method for producing a peptide, comprising a step of translating a nucleic acid that comprises a codon encoding a first unnatural amino acid and a codon encoding a second unnatural amino acid which are adjacent to each other, wherein at least one of the first unnatural amino acid and the second unnatural amino acid is an unnatural amino acid U1, and the unnatural amino acid U1 is an unnatural amino acid that meets the following requirement (A) and meets at least one selected from the group consisting of the following requirements (B) and (C): (A) a nitrogen atom constituting a main-chain amino group (nitrogen atom N1) and an oxygen atom other than an oxygen atom constituting a main-chain carboxy group (oxygen atom O1) are bound via two atoms to each other; (B) the oxygen atom O1 constitutes an alkoxy group or an aryloxy group; (C) the nitrogen atom N1 is bound to a linear or branched C₁-C₆ alkyl group.

## Description

### [Technical Field]

The present invention relates to a method for producing a peptide, comprising a step of translating an amino acid having an oxygen atom at a specific position.

### [Background Art]

There are many reports on the translation introduction of unnatural amino acids in cell-free systems, but many of them focus on the modification of various translation factors. It is difficult to efficiently translate unnatural amino acids using various translation factors that exist as translation devices for natural amino acids, and the preparation of various modified translation factors corresponding to the unnatural amino acids of interest has been tried so far.

In fact, it has been reported that ribosomal modifications improve the translation efficiency of D-amino acids or β-amino acids (see Non Patent Literatures 1 and 4). It has also been reported that EF-Tu modification allows the translation introduction of unnatural amino acids having a bulky aromatic ring at the side chain that cannot be translated with unmodified EF-Tu (see Non Patent Literature 2). In addition, it has been reported that the translation introduction of N-methylamino acid can be achieved by modification of aminoacyl tRNA synthetase (see Patent Literature 1).

It has also been reported that the continuous translation introduction of D-amino acids or β-amino acids has been achieved by using modified tRNA^{Pro1E2} (see, Non Patent Literatures 7, 8, 14 and 15, and Patent Literature 2). It has also been reported that modified tRNA^{Pro1E2} is effectively used for the continuous translation introduction of cyclic β-amino acids (see Non Patent Literature 10) and the single translation introduction of cyclic γ-amino acids or amino benzoic acids (see Non Patent Literatures 11 and 12).

In this way, there are reports on the single and continuous translation introductions of unnatural amino acids with the modification of various translation factors such as tRNA according to their respective purposes. On the other hand, it has been reported for some D-amino acids or β-amino acids that the continuous translation introduction is difficult even when natural translation factors are used but the single translation introduction is possible (see Non Patent Literatures 3, 5, and 6).

It has also been reported that linear γ-amino acids having a hydroxyl group at the β-position have excellent translation efficiency compared to linear γ-amino acids having no hydroxyl group at the β-position (see Non Patent Literature 13).

### [Citation List]

### [Patent Literature]

[Patent Literature 1] International Publication No. WO 2016/148044
[Patent Literature 2] International Publication No. WO 2019/077887

### [Non Patent Literature]

[Non Patent Literature 1] Biochemistry 2006, 45, 51, 15541
[Non Patent Literature 2] J. Am. Chem. Soc. 2007, 129, 46, 14458.
[Non Patent Literature 3] J. Am. Chem. Soc. 2013, 135, 5, 1830
[Non Patent Literature 4] Bioorganic & Medicinal Chemistry 21 (2013) 1088
[Non Patent Literature 5] Nucleic Acids Research, 2015, Vol. 43, No. 12, 5687
[Non Patent Literature 6] J. Am. Chem. Soc. 2016, 138, 6, 1962
[Non Patent Literature 7] Nucleic Acids Research, 2017, Vol. 45, No. 22, 12601
[Non Patent Literature 8] J. Am. Chem. Soc. 2018, 140, 12159
[Non Patent Literature 9] Int J Mol Sci. 2019, 20, 522
[Non Patent Literature 10] Nature Chemistry volume 12, 1081
[Non Patent Literature 11] J. Am. Chem. Soc. 2020, 142, 39, 16518
[Non Patent Literature 12] J. Am. Chem. Soc. 2020, 142, 11, 4965
[Non Patent Literature 13] ACS Chem. Biol. 2021, 16, 1325
[Non Patent Literature 14] Cell Chem. Biol. 24, 46-54 (2017)
[Non Patent Literature 15] Nature Communications volume 7, Article number: 11657 (2016)
[Non Patent Literature 16] ACS Chem. Biol. 2021, 16, 1011-1018

### [Summary of Invention]

### [Technical Problem]

Accordingly, an object of the present invention is to provide a method for producing a peptide that exhibits excellent translation efficiency with respect to the continuous translation introduction of unnatural amino acids.

### [Solution to Problem]

The present inventors have intensively studied, and, as a result, have found that the translation efficiency in the translation of a peptide having consecutive unnatural amino acids is improved when an amino acid having an oxygen atom at a specific position is translated.

The present invention is based on the finding and specifically relates to [1] to [123].
[1] A method for producing a peptide, comprising a step of translating a nucleic acid that comprises a codon encoding a first unnatural amino acid and a codon encoding a second unnatural amino acid which are adjacent to each other, wherein
   at least one of the first unnatural amino acid and the second unnatural amino acid is an unnatural amino acid U1, and the unnatural amino acid U1 is an unnatural amino acid that meets the following requirement (A) and meets at least one selected from the group consisting of the following requirements (B) and (C):
   (A) a nitrogen atom constituting a main-chain amino group (nitrogen atom N1) and an oxygen atom other than an oxygen atom constituting a main-chain carboxy group (oxygen atom O1) are bound via two atoms to each other;
   (B) the oxygen atom O1 constitutes an alkoxy group or an aryloxy group;
   (C) the nitrogen atom N1 is bound to a linear or branched C₁-C₆ alkyl group.
[2] The method according to [1], wherein the unnatural amino acid U1 meets the requirements (A) and (B).
[3] The method according to [1], wherein the unnatural amino acid U1 meets the requirements (A) and (C).
[4] The method according to [1], wherein the unnatural amino acid U1 meets the requirements (A), (B) and (C).
[5] The method according to any one of [1] to [4], wherein the oxygen atom O1 is present in a substituent substituted with the nitrogen atom N1.
[6] The method according to any one of [1] to [4], wherein the oxygen atom O1 is contained in a side chain of the unnatural amino acid U1.
[7] The method according to any one of [1] to [4], wherein in the unnatural amino acid U1, the nitrogen atom N1, a carbon atom constituting a main chain of the unnatural amino acid U1, and an atom constituting a side chain of the unnatural amino acid U1 together form a ring.
[8] The method according to any one of [1] to [7], wherein the oxygen atom O1, together with a linear or branched C₁-C₆ alkyl group, a C₃-C₁₀ cycloalkyl group, a C₆-C₁₀ aryl group, a C₇-C₁₂ arylalkyl group, or a C₇-C₁₂ alkylaryl group, constitutes a corresponding C₁-C₆ alkoxy group, a corresponding C₃-C₁₀ cycloalkoxy group, a corresponding C₆-C₁₀ aryloxy group, a corresponding C₇-C₁₂ arylalkoxy group, or a corresponding C₇-C₁₂ alkylaryloxy group.
[9] The method according to any one of [1] to [7], wherein the oxygen atom O1, together with a methyl group, an ethyl group, a phenyl group, or a benzyl group, constitutes a corresponding methoxy group, a corresponding ethoxy group, a corresponding phenoxy group, or a corresponding benzyloxy group.
[10] The method according to any one of [1] to [7], wherein the oxygen atom O1, together with a methyl group or a phenyl group, constitutes a corresponding methoxy group or a corresponding phenoxy group.
[11] The method according to any one of [1] to [7], wherein the oxygen atom O1, together with a linear or branched C₁-C₆ alkyl group, constitutes a corresponding C₁-C₆ alkoxy group.
[12] The method according to any one of [1] to [7], wherein the oxygen atom O1, together with a methyl group or an ethyl group, constitutes a corresponding methoxy group or a corresponding ethoxy group.
[13] The method according to any one of [1] to [7], wherein the oxygen atom O1, together with a methyl group, constitutes a corresponding methoxy group.
[14] The method according to any one of [1] to [7], wherein the oxygen atom O1, together with a C₆-C₁₀ aryl group, constitutes a corresponding C₆-C₁₀ aryloxy group.
[15] The method according to any one of [1] to [7], wherein the oxygen atom O1, together with a phenyl group, constitutes a corresponding phenoxy group.
[16] The method according to any one of [1] to [7], wherein the oxygen atom O1 constitutes a hydroxyl group.
[17] The method according to any one of [1] to [16], wherein the nitrogen atom N1 is substituted with a methyl group or an ethyl group.
[18] The method according to any one of [1] to [16], wherein the nitrogen atom N1 is substituted with a methyl group.
[19] The method according to any one of [1] to [16], wherein the nitrogen atom N1 constitutes an unsubstituted amino group.
[20] The method according to any one of [1] to [19], wherein the unnatural amino acid U1 is an α-amino acid.
[21] A method for producing a peptide, comprising a step of translating a nucleic acid that comprises a codon encoding a first unnatural amino acid and a codon encoding a second unnatural amino acid which are adjacent to each other, wherein
   at least one of the first unnatural amino acid and the second unnatural amino acid is an unnatural amino acid U1, and the unnatural amino acid U1 is an unnatural amino acid represented by the following formula (I): 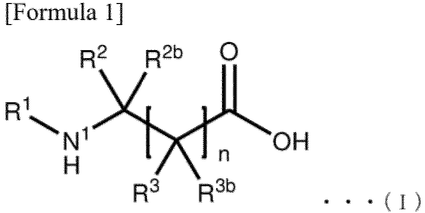 wherein n is an integer of 0 or more and 2 or less;
   R¹ is a group represented by formula (II), a hydrogen atom or a linear or branched C₁-C₆ alkyl group; 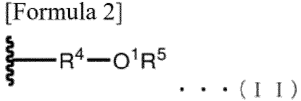
   R² and R³ are each independently (a) a group represented by the formula (II), (b) a hydrogen atom, or (c) a linear or branched C₁-C₄ alkyl group;
   R^{2d} and R^{3b} are each independently a hydrogen atom or a linear or branched C₁-C₄ alkyl group;
   R² and R^{2b} or R3 and R^{3b} may form a ring together with a carbon atom to which they are bound;
   any one or more of R¹, R², and R³ is a group represented by the formula (II);
   N¹ (nitrogen atom N1) in the formula (I) and O1 (oxygen atom O1) in the formula (II) are bound via two atoms to each other;
   when R¹ is a substituent represented by the formula (II), R⁴ and R² may form a ring together with a carbon atom to which R² is bound and N¹;
   when a plurality of R³ is present, they are each independently the same as or different from each other;
   when a plurality of R^{3b} is present, they are each independently the same as or different from each other;
   in the formula (II), 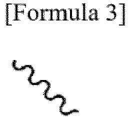 represents a binding position with N¹;
      R⁴ is a C₁-C₂ alkylene group;
      R⁵ is a hydrogen atom, a linear or branched C₁-C₆ alkyl group, a C₃-C₁₀ cycloalkyl group, a C₆-C₁₀ aryl group, a C₇-C₁₂ arylalkyl group, or a C₇-C₁₂ alkylaryl group; and
      when R² is a substituent represented by the formula (II), at least one of R¹ in the formula (I) and R⁵ in the formula (II) is not a hydrogen atom.
[22] The method according to [21], wherein the unnatural amino acid U1 is an unnatural amino acid represented by the following formula (III), (IV) or (V): 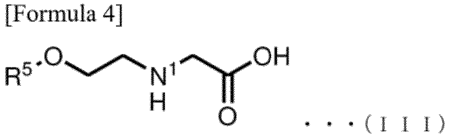 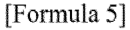 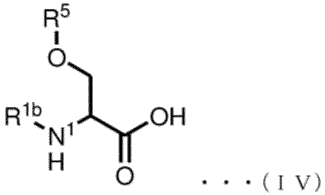 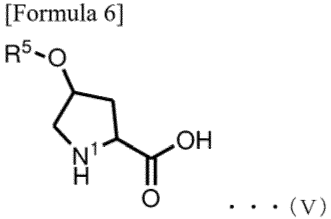 in the formulas (III), (IV) and (V), R^{1b} is a hydrogen atom or a linear or branched C₁-C₆ alkyl, and R⁵ has the same meaning as R⁵ in the formula (II).
[23] The method according to [22], wherein the unnatural amino acid U1 is an unnatural amino acid represented by the formula (III).
[24] The method according to [22], wherein the unnatural amino acid U1 is an unnatural amino acid represented by the formula (IV).
[25] The method according to [22], wherein the unnatural amino acid U1 is an unnatural amino acid represented by the formula (V).
[26] The method according to [21] or [22], wherein the unnatural amino acid U1 is an unnatural amino acid represented by the following formula (III), (IVb) or (Vb): 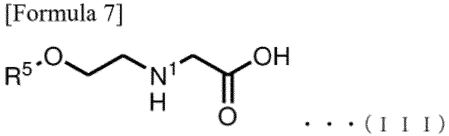 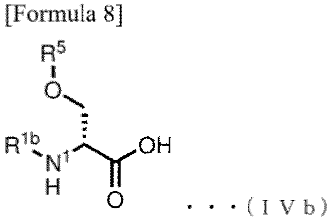 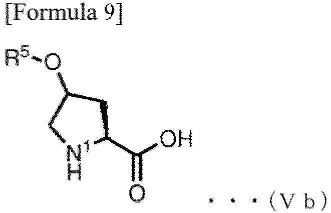 in the formulas (III), (IVb) and (Vb), R¹⁶ is a hydrogen atom or a linear or branched C₁-C₆ alkyl, and R⁵ has the same meaning as R⁵ in the formula (II).
[27] The method according to [26], wherein the unnatural amino acid U1 is an unnatural amino acid represented by the formula (IVb).
[28] The method according to [26], wherein the unnatural amino acid U1 is an unnatural amino acid represented by the formula (Vb).
[29] The method according to any one of [21] to [28], wherein, in the formula (II), R⁵ is a linear or branched C₁-C₆ alkyl group, a C₃-C₁₀ cycloalkyl group, a C₆-C₁₀ aryl group, a C₇-C₁₂ arylalkyl group, or a C₇-C₁₂ alkylaryl group.
[30] The method according to any one of [21] to [28], wherein in the formula (1), R¹ is a group represented by the formula (II), or a linear or branched C₁-C₆ alkyl group.
[31] The method according to any one of [21] to [28], wherein in the formula (I), R¹ is a group represented by the formula (II), or a linear or branched C₁-C₆ alkyl group, and
   in the formula (II), R⁵ is a linear or branched C₁-C₆ alkyl group, a C₃-C₁₀ cycloalkyl group, a C₆-C₁₀ aryl group, a C₇-C₁₂ arylalkyl group, or a C₇-C₁₂ alkylaryl group.
[32] The method according to any one of [21] to [31], wherein R¹ is a group represented by the formula (II).
[33] The method according to any one of [21] to [31], wherein R² is a group represented by the formula (II).
[34] The method according to any one of [21] to [31], wherein R¹ comprises a group represented by the formula (II), R⁴ and R² may form a ring together with a carbon atom to which R² is bound and N¹.
[35] The method according to any one of [21] to [34], wherein R⁵ is a linear or branched C₁-C₆ alkyl group, a C₆-C₁₀ aryl group, or a C₇-C₁₂ arylalkyl group.
[36] The method according to any one of [21] to [34], wherein R⁵ is a methyl group, an ethyl group, a phenyl group, or a benzyl group.
[37] The method according to any one of [21] to [34], wherein R⁵ is a methyl group or a phenyl group.
[38] The method according to any one of [21] to [34], wherein R⁵ is a linear or branched C₁-C₆ alkyl group.
[39] The method according to any one of [21] to [34], wherein R⁵ is a methyl group or an ethyl group.
[40] The method according to any one of [21] to [34], wherein R⁵ is a methyl group.
[41] The method according to any one of [21] to [34], wherein R⁵ is a C₆-C₁₀ aryl group.
[42] The method according to any one of [21] to [34], wherein R⁵ is a phenyl group.
[43] The method according to any one of [21] to [34], wherein R⁵ is a hydrogen atom.
[44] The method according to any one of [21] to [43], wherein R¹ and R^{1b} each are a methyl group or an ethyl group.
[45] The method according to any one of [21] to [43], wherein R¹ and R^{1b} each are a methyl group.
[46] The method according to any one of [21] to [43], wherein R¹ and R^{1b} each are a hydrogen atom.
[47] The method according to any one of [21] to [46], wherein n is 0.
[48] The method according to any one of [1] to [47], wherein both the first unnatural amino acid and the second unnatural amino acid are the unnatural amino acid U1.
[49] The method according to any one of [1] to [47], wherein the first unnatural amino acid is the unnatural amino acid U1, and the second unnatural amino acid is an unnatural amino acid that is different from the unnatural amino acid U1 (unnatural amino acid U2).
[50] The method according to any one of [1] to [49], wherein a codon encoding the first unnatural amino acid is translated before a codon encoding the second unnatural amino acid.
[51] The method according to any one of [1] to [49], wherein a codon encoding the first unnatural amino acid is translated after a codon encoding the second unnatural amino acid.
[52] The method according to any one of [1], [2], [5], [10], [17] to [19], [20], [49], and [50], wherein the first unnatural amino acid is the unnatural amino acid U1, and the second unnatural amino acid is the unnatural amino acid U2;
   the unnatural amino acid U1 meets the requirements (A) and (B); the oxygen atom O1 is present in a substituent substituted with the nitrogen atom N1; the oxygen atom O1 constitutes a methoxy group or a phenoxy group; and
   a codon encoding the first unnatural amino acid is translated before a codon encoding the second unnatural amino acid.
[53] The method according to any one of [1], [2], [6], [10], [19], [20], [49], and [50], wherein the first unnatural amino acid is the unnatural amino acid U1, and the second unnatural amino acid is the unnatural amino acid U2;
   the unnatural amino acid U1 meets the requirements (A) and (B); the oxygen atom O1 is contained in a side chain of the unnatural amino acid U1; the oxygen atom O1 constitutes a methoxy group or a phenoxy group; the nitrogen atom N1 constitutes an unsubstituted amino group; and
   a codon encoding the first unnatural amino acid is translated before a codon encoding the second unnatural amino acid.
[54] The method according to any one of [1], [3], [6], [16], [17], [20], [49], and [50], wherein the first unnatural amino acid is the unnatural amino acid U1, and the second unnatural amino acid is the unnatural amino acid U2;
   the unnatural amino acid U1 meets the requirements (A) and (C); the oxygen atom O1 is contained in a side chain of the unnatural amino acid U1; the oxygen atom O1 constitutes a hydroxyl group; the nitrogen atom N1 is substituted with a methyl group or an ethyl group; and
   a codon encoding the first unnatural amino acid is translated before a codon encoding the second unnatural amino acid.
[55] The method according to any one of [1], [2], [7], [10], [17] to [20], [49], and [50], wherein the first unnatural amino acid is the unnatural amino acid U1, and the second unnatural amino acid is the unnatural amino acid U2;
   the unnatural amino acid U1 meets the requirements (A) and (B); in the unnatural amino acid U1, the nitrogen atom N1, a carbon atom constituting a main chain of the unnatural amino acid U1, and an atom constituting a side chain of the unnatural amino acid U1 together form a ring; the oxygen atom O1 constitutes a methoxy group or a phenoxy group; and
   a codon encoding the first unnatural amino acid is translated before a codon encoding the second unnatural amino acid.
[56] A method for producing a peptide, comprising a step of translating a nucleic acid that comprises a codon encoding a first unnatural amino acid and a codon encoding a second unnatural amino acid which are adjacent to each other, wherein
   the first unnatural amino acid is the unnatural amino acid U1, and the second unnatural amino acid is the unnatural amino acid U2;
   the unnatural amino acid U1 is an α-amino acid; in the unnatural amino acid U1, a nitrogen atom constituting a main-chain amino group (nitrogen atom N1) and an oxygen atom other than an oxygen atom constituting a main-chain carboxy group (oxygen atom O1) are bound via two or three atoms to each other; the oxygen atom O1 is contained in a side chain of the unnatural amino acid U1; the oxygen atom O1 constitutes a hydroxyl group; the nitrogen atom N1 is substituted with a methyl group or an ethyl group; and
   a codon encoding the first unnatural amino acid is translated before a codon encoding the second unnatural amino acid.
[57] The method according to any one of [1], [2], [5], [10], [17] to [20], [49], and [51], wherein the first unnatural amino acid is the unnatural amino acid U1, and the second unnatural amino acid is the unnatural amino acid U2;
   the unnatural amino acid U1 meets the requirements (A) and (B); the oxygen atom O1 is present in a substituent substituted with the nitrogen atom N¹; the oxygen atom O1 constitutes a methoxy group or a phenoxy group; and
   a codon encoding the first unnatural amino acid is translated after a codon encoding the second unnatural amino acid.
[58] The method according to any one of [1], [2], [6], [10], [19], [20], [49], and [51], wherein the first unnatural amino acid is the unnatural amino acid U1, and the second unnatural amino acid is the unnatural amino acid U2;
   the unnatural amino acid U1 meets the requirements (A) and (B); the oxygen atom O1 is contained in a side chain of the unnatural amino acid U1; the oxygen atom O1 constitutes a methoxy group or a phenoxy group; the nitrogen atom N1 constitutes an unsubstituted amino group; and
   a codon encoding the first unnatural amino acid is translated after a codon encoding the second unnatural amino acid.
[59] The method according to any one of [1], [3], [6], [16], [17], [20], [49], and [51], wherein the first unnatural amino acid is the unnatural amino acid U1, and the second unnatural amino acid is the unnatural amino acid U2;
   the unnatural amino acid U1 meets the requirements (A) and (C); the oxygen atom O1 is contained in a side chain of the unnatural amino acid U1; the oxygen atom O1 constitutes a hydroxyl group; the nitrogen atom N1 is substituted with a methyl group or an ethyl group; and
   a codon encoding the first unnatural amino acid is translated after a codon encoding the second unnatural amino acid.
[60] The method according to any one of [1], [2], [7], [10], [17] to [20], [49], and [51], wherein the first unnatural amino acid is the unnatural amino acid U1, and the second unnatural amino acid is the unnatural amino acid U2;
   the unnatural amino acid U1 meets the requirements (A) and (B); in the unnatural amino acid U1, the nitrogen atom N1, a carbon atom constituting a main chain of the unnatural amino acid U1, and an atom constituting a side chain of the unnatural amino acid U1 together form a ring; the oxygen atom O1 constitutes a methoxy group or a phenoxy group; and
   a codon encoding the first unnatural amino acid is translated after a codon encoding the second unnatural amino acid.
[61] A method for producing a peptide, comprising a step of translating a nucleic acid that comprises a codon encoding a first unnatural amino acid and a codon encoding a second unnatural amino acid which are adjacent to each other, wherein
   the first unnatural amino acid is the unnatural amino acid U1, and the second unnatural amino acid is the unnatural amino acid U2;
   the unnatural amino acid U1 is an α-amino acid; in the unnatural amino acid U1, a nitrogen atom constituting a main-chain amino group (nitrogen atom N1) and an oxygen atom other than an oxygen atom constituting a main-chain carboxy group (oxygen atom O1) are bound via two or three atoms to each other; the oxygen atom O1 is contained in a side chain of the unnatural amino acid U1; the oxygen atom O1 constitutes a hydroxyl group; the nitrogen atom N1 is substituted with a methyl group or an ethyl group; and
   a codon encoding the first unnatural amino acid is translated after a codon encoding the second unnatural amino acid.
[62] The method according to any one of [21], [23], [29], [32], [37], [44] to [47], [49], and [50], wherein the first unnatural amino acid is the unnatural amino acid U1, and the second unnatural amino acid is the unnatural amino acid U2;
   the unnatural amino acid U1 is an unnatural amino acid represented by the formula (III); in the formula (III), R⁵ is a methyl group or a phenyl group; and
   a codon encoding the first unnatural amino acid is translated before a codon encoding the second unnatural amino acid.
[63] The method according to any one of [21], [24], [29], [33], [37], [46], [47], [49], and [50], wherein the first unnatural amino acid is the unnatural amino acid U1, and the second unnatural amino acid is the unnatural amino acid U2;
   the unnatural amino acid U1 is an unnatural amino acid represented by the formula (IV); in the formula (IV), R⁵ is a methyl group or a phenyl group; R^{1b} is a hydrogen atom; and
   a codon encoding the first unnatural amino acid is translated before a codon encoding the second unnatural amino acid.
[64] The method according to any one of [21], [24], [30], [33], [43], [44], [47], [49], and [50], wherein the first unnatural amino acid is the unnatural amino acid U1, and the second unnatural amino acid is the unnatural amino acid U2;
   the unnatural amino acid U1 is an unnatural amino acid represented by the formula (IV); in the formula (IV), R⁵ is a hydrogen atom; R^{1b} is a methyl group or an ethyl group; and
   a codon encoding the first unnatural amino acid is translated before a codon encoding the second unnatural amino acid.
[65] The method according to any one of [21], [25], [29], [34], [37], [44] to [46], [47], [49], and [50], wherein the first unnatural amino acid is the unnatural amino acid U1, and the second unnatural amino acid is the unnatural amino acid U2;
   the unnatural amino acid U1 is an unnatural amino acid represented by the formula (V); in the formula (V), R⁵ is a phenyl group; and
   a codon encoding the first unnatural amino acid is translated before a codon encoding the second unnatural amino acid.
[66] A method for producing a peptide, comprising a step of translating a nucleic acid that comprises a codon encoding a first unnatural amino acid and a codon encoding a second unnatural amino acid which are adjacent, wherein
   a codon encoding the first unnatural amino acid is translated before a codon encoding the second unnatural amino acid; and
   the first unnatural amino acid is the unnatural amino acid U1, and the second unnatural amino acid is the unnatural amino acid U2; and the unnatural amino acid U1 is an unnatural amino acid represented by the following formula (I): 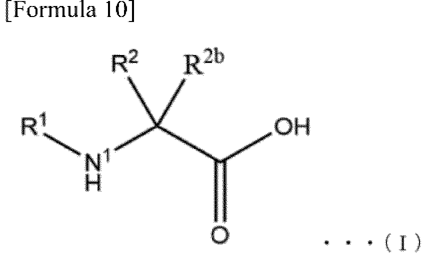 wherein
      R¹ is a methyl group or an ethyl group; 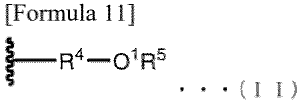
      R² is a group represented by formula (II);
      R^{2b} is a hydrogen atom or a linear or branched C₁-C₄ alkyl group;
      N¹ (nitrogen atom N1) in the formula (I) and O¹ (oxygen atom O1) in the formula (II) are bound via two or three atoms to each other;
      in the formula (II), 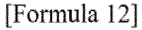 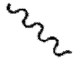 represents a binding position with N¹;
         R⁴ is a C₁-C₂ alkylene group; and
         R⁵ is a hydrogen atom.
[67] The method according to any one of [21], [23], [29], [32], [37], [44] to [47], [49], and [51], wherein the first unnatural amino acid is the unnatural amino acid U1, and the second unnatural amino acid is the unnatural amino acid U2;
   the unnatural amino acid U1 is an unnatural amino acid represented by the formula (III); in the formula (III), R⁵ is a methyl group or a phenyl group; and
   a codon encoding the first unnatural amino acid is translated after a codon encoding the second unnatural amino acid.
[68] The method according to any one of [21], [24], [29], [33], [37], [46], [47], [49], and [51], wherein the first unnatural amino acid is the unnatural amino acid U1, and the second unnatural amino acid is the unnatural amino acid U2;
   the unnatural amino acid U1 is an unnatural amino acid represented by the formula (IV); in the formula (IV), R⁵ is a methyl group or a phenyl group; R^{1b} is a hydrogen atom; and
   a codon encoding the first unnatural amino acid is translated after a codon encoding the second unnatural amino acid.
[69] The method according to any one of [21], [24], [30], [33], [43], [44], [47], [49], and [51], wherein the first unnatural amino acid is the unnatural amino acid U1, and the second unnatural amino acid is the unnatural amino acid U2;
   the unnatural amino acid U1 is an unnatural amino acid represented by the formula (IV); in the formula (IV), R⁵ is a hydrogen atom; R^{1b} is a methyl group or an ethyl group; and
   a codon encoding the first unnatural amino acid is translated after a codon encoding the second unnatural amino acid.
[70] The method according to any one of [21], [25], [29], [34], [37], [44] to [47], [49], and [51], wherein the first unnatural amino acid is the unnatural amino acid U1, and the second unnatural amino acid is the unnatural amino acid U2;
   the unnatural amino acid U1 is an unnatural amino acid represented by the formula (V); in the formula (V), R⁵ is a phenyl group; and
   a codon encoding the first unnatural amino acid is translated after a codon encoding the second unnatural amino acid.
[71] A method for producing a peptide, comprising a step of translating a nucleic acid that comprises a codon encoding a first unnatural amino acid and a codon encoding a second unnatural amino acid which are adjacent to each other, wherein
   a codon encoding the first unnatural amino acid is translated after a codon encoding the second unnatural amino acid;
   the first unnatural amino acid is the unnatural amino acid U1, and the second unnatural amino acid is the unnatural amino acid U2; and the unnatural amino acid U1 is an unnatural amino acid represented by the following formula (I): 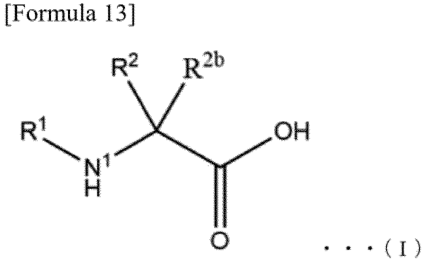 wherein
      R¹ is a methyl group or an ethyl group; 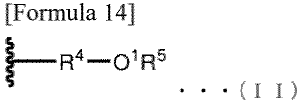
      R² is a group represented by formula (II);
      R^{2b} is a hydrogen atom or a linear or branched C₁-C₄ alkyl group;
      N¹ (nitrogen atom N1) in the formula (I) and O¹ (oxygen atom O1) in the formula (II) are bound via two or three atoms to each other;
      in the formula (II), 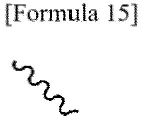 represents a binding position with N¹;
         R⁴ is a C₁-C₂ alkylene group; and
         R⁵ is a hydrogen atom.
[72] The method according to any one of [1] to [71], wherein the unnatural amino acid U1 is a D-form.
[73] The method according to any one of [1] to [71], wherein the unnatural amino acid U1 is a L-form.
[74] The method according to any one of [1] to [73], wherein the nitrogen atom N1 and the oxygen atom O1 are bound via two carbon atoms to each other.
[75] The method according to any one of [1] to [74], wherein the unnatural amino acid U1 does not comprise a heteroatom other than a nitrogen atom and an oxygen atom.
[76] The method according to any one of [1] to [75], wherein the unnatural amino acid U1 has only one oxygen atom O1.
[77] The method according to any one of [1] to [20], [48] to [60], and [68] to [76], wherein a nitrogen atom constituting a main-chain amino group of the unnatural amino acid U2 is substituted with a linear or branched C₁-C₆ alkyl group.
[78] The method according to any one of [1] to [20], [48] to [60], and [72] to [77], wherein a nitrogen atom constituting a main-chain amino group of the unnatural amino acid U2 is substituted with a methyl group or an ethyl group.
[79] The method according to any one of [1] to [20], [48] to [60], and [72] to [78], wherein a nitrogen atom constituting a main-chain amino group of the unnatural amino acid U2 is substituted with a methyl group.
[80] The method according to any one of [1] to [20], [48] to [60], and [72] to [79], wherein the unnatural amino acid U2 has, as a side chain, a linear or branched C₁-C₆ alkyl group that may be substituted with a 5- to 10-membered aryl group or a 5- to 10-membered aryl group that may be substituted with a linear or branched C₁-C₆ alkyl group.
[81] The method according to any one of [1] to [20], [48] to [60], and [72] to [80], wherein the unnatural amino acid U2 has, as a side chain, a linear or branched C₁-C₄ alkyl group or a 5- to 10-membered aryl group substituted with a linear C₁-C₃ alkyl group.
[82] The method according to any one of [1] to [20], [48] to [60], and [72] to [81], wherein the unnatural amino acid U2 has, as a side chain, a methyl group or a benzyl group.
[83] The method according to any one of [1] to [20], [48] to [60], and [72] to [82], wherein the unnatural amino acid U2 has a methyl group as a side chain.
[84] The method according to any one of [1] to [20], [48] to [60], and [72] to [82], wherein the unnatural amino acid U2 has a benzyl group as a side chain.
[85] The method according to any one of [1] to [20], [48] to [60], and [72] to [84], wherein the unnatural amino acid U2 is an α-amino acid.
[86] The method according to any one of [1] to [20], [48] to [60], [72] to [83], and [85],
   wherein a nitrogen atom constituting a main-chain amino group of the unnatural amino acid U2 is substituted with a methyl group, and the unnatural amino acid U2 is an α-amino acid having a methyl group as a side chain.
[87] The method according to any one of [1] to [20], [48] to [60], [72] to [82], and [84] to [85], wherein a nitrogen atom constituting a main-chain amino group of the unnatural amino acid U2 is substituted with a methyl group, and the unnatural amino acid U2 is an α-amino acid having a benzyl group as a side chain.
[88] The method according to any one of [21] to [51] and [61] to [87], wherein the unnatural amino acid U2 is an unnatural amino acid represented by the following formula (VI): 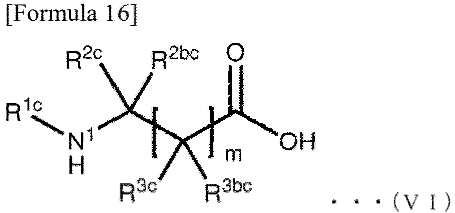 in the formula (VI),
   m is an integer of 0 or more and 2 or less;
   R^{1c} is a hydrogen atom or a linear or branched C₁-C₆ alkyl group;
   R^{2c} is a hydrogen atom, a linear or branched C₁-C₆ alkyl group that may be substituted with a 5- to 10-membered aryl group, or a 5- to 10-membered aryl group that may be substituted with a linear or branched C₁-C₆ alkyl group;
   R^{2bc} is a hydrogen atom or a linear or branched C₁-C₄ alkyl group;
   R^{2c} and R^{2bc} may form a ring together with a carbon atom to which they are bound; and
   a nitrogen atom to which R^{1c} is bound, R^{1c}, a carbon atom to which R^{2c} is bound, and R^{2c} may together form a ring.
[89] The method according to any one of [21] to [51] and [61] to [88], wherein R^{1c} is a linear or branched C₁-C₆ alkyl group.
[90] The method according to any one of [21] to [51] and [62] to [89], wherein R^{1c} is a methyl group or an ethyl group.
[91] The method according to any one of [21] to [51] and [62] to [90], wherein R^{1c} is a methyl group.
[92] The method according to any one of [21] to [51] and [62] to [91], wherein R^{2c} is a linear or branched C₁-C₆ alkyl group that may be substituted with a 5- to 10-membered aryl group, or a 5- to 10-membered aryl group that may be substituted with a linear or branched C₁-C₆ alkyl group.
[93] The method according to any one of [21] to [51] and [62] to [92], wherein R^{2c} is a linear or branched C₁-C₄ alkyl group or a 5- to 10-membered aryl group substituted with a linear C₁-C₃ alkyl group.
[94] The method according to any one of [21] to [51] and [62] to [93], wherein R^{2c} is a methyl group or a benzyl group.
[95] The method according to any one of [21] to [51] and [62] to [94], wherein R^{2c} is a methyl group.
[96] The method according to any one of [21] to [51] and [62] to [94], wherein R^{2c} is a benzyl group.
[97] The method according to any one of [21] to [51] and [62] to [94], wherein m is 0.
[98] The method according to any one of [21] to [51], [62] to [95], and [97], wherein R^{1c} and R^{2c} each are a methyl group and m is 0.
[99] The method according to any one of [21] to [51], [62] to [94], and [96] to [98], wherein R^{1c} is a methyl group, R^{2c} is a benzyl group, and m is 0.
[100] The method according to any one of [1] to [99], wherein the peptide comprises the unnatural amino acid U1 and the unnatural amino acid U2.
[101] The method according to any one of [1] to [100], wherein a molecular weight of the peptide is 500 g/mol or more.
[102] The method according to any one of [1] to [101], wherein the number of amino acid residues constituting the peptide is 2 or more and 100 or less.
[103] The method according to any one of [1] to [102], wherein the peptide is a cyclic peptide.
[104] The method according to [103], wherein the number of amino acid residues constituting a cyclic portion of the cyclic peptide is 5 or more and 30 or less.
[105] The method according to any one of [1] to [104], wherein the peptide comprises one or more N-substituted amino acid residues.
[106] The method according to any one of [1] to [105], wherein a ClogP of the peptide is 4 or more and 25 or less.
[107] The method according to any one of [1] to [106], wherein a ClogP of the peptide is 28% or more and 174% or less with respect to a ClogP value of cyclosporine A.
[108] The method according to any one of [1] to [107], wherein a ClogP/number of amino acid residues of the peptide is 1.0 or more.
[109] The method according to any one of [1] to [108], wherein the translation is performed with a cell-free translation system.
[110] The method according to [109], wherein the cell-free translation system is a reconstituted cell-free translation system.
[111] The method according to [109] or [110], wherein the cell-free translation system is a translation system reconstituted with a factor derived from E. coli.
[112] The method according to any one of [109] to [111], wherein the cell-free translation system comprises a ribosome derived from E. coli.
[113] The method according to any one of [109] to [112], wherein the cell-free translation system comprises a first tRNA to which a first unnatural amino acid is bound and a second tRNA to which a second unnatural amino acid is bound.
[114] The method according to [113], wherein the first tRNA comprises an anticodon that is complementary to a codon encoding the first unnatural amino acid, and the second tRNA comprises an anticodon that is complementary to a codon encoding the second unnatural amino acid.
[115] The method according to [113] or [114], wherein the first tRNA and the second tRNA each are an artificial tRNA.
[116] The method according to [113] or [114], wherein the first tRNA and the second tRNA each are an transcribed tRNA.
[117] The method according to any one of [113] to [115], wherein the first tRNA and the second tRNA each comprise a body derived from a naturally occurring tRNA.
[118] The method according to any one of [113] to [117], further comprising a step of preparing the tRNA by a pCpA method.
[119] The method according to any one of [1] to [118], wherein the nucleic acid is mRNA.
[120] A method for producing a peptide complex, comprising the method according to any one of [1] to [119].
[121] The method according to [120], wherein the peptide complex is a peptide-nucleic acid complex.
[122] A method for producing a peptide complex, comprising:
   obtaining a peptide by the method according to any one of [1] to [119]; and
   binding one or more selected from the group consisting of a nucleic acid, a ribosome, a spacer, and a linker to the peptide.
[123] A method for producing a peptide-nucleic acid complex, comprising:
   obtaining a peptide by the method according to any one of [1] to [119]; and
   binding a nucleic acid to the peptide.

### [Advantageous Effects of Invention]

According to the present invention, when subjecting a peptide having consecutively-disposed unnatural amino acids to translation introduction, excellent translation efficiency is exhibited by using a tRNA bound to an amino acid having an oxygen atom at a specific position. Since aminoacylated tRNA is incorporated into a ribosome at the time of translation, it is believed that the amino acid having an oxygen atom (especially an oxy group) at an appropriate position tends to be stabilized in the ribosome and improves translation efficiency.

### [Brief Description of Drawing]

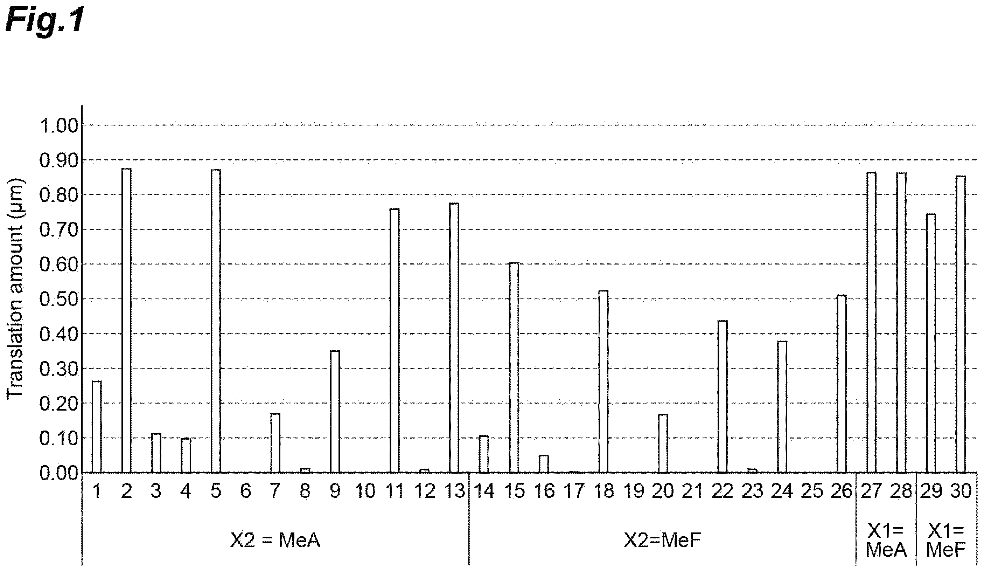

[Figure 1] Figure 1 is a graph showing the translation amount when a peptide is translated using aminoacylated tRNA.

### [Description of Embodiments]

Hereinafter, the embodiments of the present invention are described in detail.

### Amino Acids

The "amino acids" constituting a peptide in the present disclosure include "natural amino acids" such as α-amino acids and "unnatural amino acids" such as β-amino acids and γ-amino acids. The three-dimensional structure of the amino acid may be either an L-form amino acid or a D-form amino acid. The "amino acid", "natural amino acid", and "unnatural amino acid" are sometimes referred to as "amino acid residue", "natural amino acid residue", and "unnatural amino acid residue", respectively.

The "natural amino acid" as used herein is an α-aminocarboxylic acid (α-amino acid) and means glycine (Gly), alanine (Ala), serine (Ser), threonine (Thr), valine (Val), leucine (Leu), isoleucine (Ile), phenylalanine (Phe), tyrosine (Tyr), tryptophan (Trp), histidine (His), glutamic acid (Glu), aspartic acid (Asp), glutamine (Gln), asparagine (Asn), cysteine (Cys), methionine (Met), lysine (Lys), arginine (Arg), and proline (Pro).

The "unnatural amino acid" as used herein means an aminocarboxylic acid that is not included in the "natural amino acid" described above. Examples of the unnatural amino acid can include β-amino acids (including D-form and L-form), γ-amino acids (including D-form and L-form), D-α-amino acids, L-α-amino acids having a side chain different from natural amino acids, α,α-disubstituted amino acids, and amino acids in which an amino group in the main chain is substituted (N-substituted amino acids). The side chain of the unnatural amino acid is not particularly limited, but may be alkyl, alkenyl, alkynyl, aryl, heteroaryl, aralkyl, cycloalkyl, or the like, in addition to a hydrogen atom. In the case of α,α-disubstituted amino acids, the two side chains may form a ring. Furthermore, these side chains may have one or more substituents. In certain aspects, the substituent can be selected from any functional groups containing a halogen atom, an O atom, an S atom, an N atom, a B atom, a Si atom, or a P atom. For example, "C₁-C₆ alkyl having halogen as a substituent" in the present disclosure means "C₁-C₆ alkyl" in which at least one hydrogen atom in the alkyl is replaced by a halogen atom, and specific examples thereof include trifluoromethyl, difluoromethyl, fluoromethyl, pentafluoroethyl, tetrafluoroethyl, trifluoroethyl, difluoroethyl, fluoroethyl, fluoroethyl, trichloromethyl, dichloromethyl, chloromethyl, pentachloroethyl, tetrachloroethyl, trichloroethyl, dichloroethyl, and chloroethyl. Also, for example, "C₅-C₁₀ aryl C₁-C₆ alkyl having a substituent" means "C₅-C₁₀ aryl C₁-C₆ alkyl" in which at least one hydrogen atom in the aryl and/or the alkyl is replaced by a substituent. Furthermore, "having two or more substituents" includes having a functional group (e.g., a functional group containing an S atom) as a substituent, and further having, in the functional group, another substituent (e.g., a substituent such as an amino or halogen group).

The amino group of the main chain of the unnatural amino acid may be an unsubstituted amino group (-NH₂ group) or a substituted amino group (-NHR group). Here, R represents optionally substituted alkyl, alkenyl, alkynyl, aryl, heteroaryl, aralkyl, or cycloalkyl. In addition, as like proline, the carbon chain bound to the N atom of the amino group of the main chain and the carbon atom at the α-position may form a ring. Examples of the alkyl substitution of the amino group include N-methylation, N-ethylation, N-propylation, and N-butylation, and examples of the aralkyl substitution of the amino group include N-benzylation. Specific examples of the N-methylamino acid can include N-methylalanine, N-methylglycine, N-methylphenylalanine, N-methyltyrosine, N-methyl-3-chlorophenylalanine, N-methyl-4-chlorophenylalanine, N-methyl-4-methoxyphenylalanine, N-methyl-4-thiazolealanine, N-methylhistidine, N-methylserine, and N-methylaspartic acid.

Examples of the substituent containing halogen include an alkyl group, a cycloalkyl group, an alkenyl group, an alkynyl group, an aryl group, a heteroaryl group, and an aralkyl group which have halogen as a substituent and more specific examples include fluoroalkyl, difluoroalkyl, and trifluoroalkyl.

Examples of the substituent containing an O atom include hydroxyl (-OH), oxy (-OR), carbonyl (-C(=O)-R), carboxy (-CO₂H), oxycarbonyl (-C(=O)-OR), carbonyloxy (-O-C(=O)-R), thiocarbonyl (-C(=O)-SR), a carbonylthio group (-S-C(=O)-R), aminocarbonyl (-C(=O)-NHR), carbonylamino (-NH-C(=O)-R), oxycarbonylamino (-NH-C(=O)-OR), sulfonylamino (-NH-SO₂-R), aminosulfonyl (-SO₂-NHR), sulfamoylamino (-NH-SO₂-NHR), thiocarboxy (-C(=O)-SH), and carboxylcarbonyl (-C(=O)-CO₂H).

Examples of oxy (-OR) include alkoxy, cycloalkoxy, alkenyloxy, alkynyloxy, aryloxy, heteroaryloxy, and aralkyloxy.

Examples of carbonyl (-C(=O)-R) include formyl (-C(=O)-H), alkylcarbonyl, cycloalkylcarbonyl, alkenylcarbonyl, alkynylcarbonyl, arylcarbonyl, heteroarylcarbonyl, and aralkylcarbonyl.

Examples of oxycarbonyl (-C(=O)-OR) include alkyloxycarbonyl, cycloalkyloxycarbonyl, alkenyloxycarbonyl, alkynyloxycarbonyl, aryloxycarbonyl, heteroaryloxycarbonyl, and aralkyloxycarbonyl.

Examples of carbonyloxy (-O-C(=O)-R) include alkylcarbonyloxy, cycloalkylcarbonyloxy, alkenylcarbonyloxy, alkynylcarbonyloxy, arylcarbonyloxy, heteroarylcarbonyloxy, and aralkylcarbonyloxy.

Examples of thiocarbonyl (-C(=O)-SR) include alkylthiocarbonyl, cycloalkylthiocarbonyl, alkenylthiocarbonyl, alkynylthiocarbonyl, arylthiocarbonyl, heteroarylthiocarbonyl, and aralkylthiocarbonyl.

Examples of carbonylthio (-S-C(=O)-R) include alkylcarbonylthio, cycloalkylcarbonylthio, alkenylcarbonylthio, alkynylcarbonylthio, arylcarbonylthio, heteroarylcarbonylthio, and aralkylcarbonylthio.

Examples of aminocarbonyl (-C(=O)-NHR) include alkylaminocarbonyl, cycloalkylaminocarbonyl, alkenylaminocarbonyl, alkynylaminocarbonyl, arylaminocarbonyl, heteroarylaminocarbonyl, and aralkylaminocarbonyl. Additional examples thereof include these groups each having the H atom bound to the N atom in -C(=O)-NHR further replaced by alkyl, cycloalkyl, alkenyl, alkynyl, aryl, heteroaryl, or aralkyl.

Examples of carbonylamino (-NH-C(=O)-R) include alkylcarbonylamino, cycloalkylcarbonylamino, alkenylcarbonylamino, alkynylcarbonylamino, arylcarbonylamino, heteroarylcarbonylamino, and aralkylcarbonylamino. Additional examples thereof include these groups each having the H atom bound to the N atom in -NH-C(=O)-R further replaced by alkyl, cycloalkyl, alkenyl, alkynyl, aryl, heteroaryl, or aralkyl.

Examples of oxycarbonylamino (-NH-C(=O)-OR) include alkoxycarbonylamino, cycloalkoxycarbonylamino, alkenyloxycarbonylamino, alkynyloxycarbonylamino, aryloxycarbonylamino, heteroaryloxycarbonylamino, and aralkyloxycarbonylamino. Additional examples thereof include these groups each having the H atom bound to the N atom in -NH-C(=O)-OR further replaced by alkyl, cycloalkyl, alkenyl, alkynyl, aryl, heteroaryl, or aralkyl.

Examples of sulfonylamino (-NH-SO₂-R) include alkylsulfonylamino, cycloalkylsulfonylamino, alkenylsulfonylamino, alkynylsulfonylamino, arylsulfonylamino, heteroarylsulfonylamino, and aralkylsulfonylamino. Additional examples thereof include these groups each having the H atom bound to the N atom in -NH-SO₂-R further replaced by alkyl, cycloalkyl, alkenyl, alkynyl, aryl, heteroaryl, or aralkyl.

Examples of aminosulfonyl (-SO₂-NHR) include alkylaminosulfonyl, cycloalkylaminosulfonyl, alkenylaminosulfonyl, alkynylaminosulfonyl, arylaminosulfonyl, heteroarylaminosulfonyl, and aralkylaminosulfonyl. Additional examples thereof include these groups each having the H atom bound to the N atom in -SO₂-NHR further replaced by alkyl, cycloalkyl, alkenyl, alkynyl, aryl, heteroaryl, or aralkyl.

Examples of sulfamoylamino (-NH-SO₂-NHR) include alkylsulfamoylamino, cycloalkylsulfamoylamino, alkenylsulfamoylamino, alkynylsulfamoylamino, arylsulfamoylamino, heteroarylsulfamoylamino, and aralkylsulfamoylamino. The two H atoms bound to the N atoms in -NH-SO₂-NHR may be replaced by substituents each independently selected from the group consisting of alkyl, cycloalkyl, alkenyl, alkynyl, aryl, heteroaryl, and aralkyl, and these two substituents may form a ring.

Examples of the substituent containing an S atom include groups such as thiol (-SH), thio (-S-R), sulfinyl (-S(=O)-R), sulfonyl (-S(O)₂-R), and sulfo (-SO₃H).

Examples of thio (-S-R) include alkylthio, cycloalkylthio, alkenylthio, alkynylthio, arylthio, heteroarylthio, and aralkylthio.

Examples of sulfinyl (-S(=O)-R) include alkylsulfinyl, cycloalkylsulfinyl, alkenylsulfinyl, alkynylsulfinyl, arylsulfinyl, heteroarylsulfinyl, and aralkylsulfinyl.

Examples of sulfonyl (-S(O)₂-R) include alkylsulfonyl, cycloalkylsulfonyl, alkenylsulfonyl, alkynylsulfonyl, arylsulfonyl, heteroarylsulfonyl, and aralkylsulfonyl.

Examples of the substituent containing an N atom include azido (-N₃, also referred to as "azide group"), cyano (-CN), primary amino (-NH₂), secondary amino (-NH-R), tertiary amino (-NR(R')), amidino (-C(=NH)-NH₂), substituted amidino (-C(=NR)-NR'R"), guanidino (-NH-C(=NH)-NH₂), substituted guanidino (-NR-C(=NR‴)-NR'R"), and aminocarbonylamino (-NR-CO-NR'R").

Examples of the secondary amino (-NH-R) include alkylamino, cycloalkylamino, alkenylamino, alkynylamino, arylamino, heteroarylamino, and aralkylamino.

Examples of the tertiary amino (-NR(R')) include an amino group having arbitrary two substituents each independently selected from alkyl, cycloalkyl, alkenyl, alkynyl, aryl, heteroaryl, aralkyl, and the like, such as alkyl(aralkyl)amino, and arbitrary two substituents of these may form a ring.

Examples of the substituted amidino (-C(=NR)-NR'R") include groups in which three substituents R, R', and R" on the N atoms are each independently selected from alkyl, cycloalkyl, alkenyl, alkynyl, aryl, heteroaryl, and aralkyl, for example, alkyl(aralkyl)(aryl)amidino.

Examples of the substituted guanidino (-NR-C(=NR‴)-NR'R") include groups in which R, R', R", and R‴ are each independently selected from alkyl, cycloalkyl, alkenyl, alkynyl, aryl, heteroaryl, and aralkyl, and groups in which these substituents form a ring.

Examples of aminocarbonylamino (-NR-C(=O)-NR'R") include groups in which R, R', and R" are each independently selected from a hydrogen atom, alkyl, cycloalkyl, alkenyl, alkynyl, aryl, heteroaryl, and aralkyl, and groups in which these substituents form a ring.

Examples of the substituent containing a B atom include boryl (-BR(R')) and dioxyboryl (-B(OR)(OR')). The two substituents R and R' may be each independently selected from alkyl, cycloalkyl, alkenyl, alkynyl, aryl, heteroaryl, aralkyl, or the like, or may form a ring.

At least one atom constituting an "amino acid" constituting a peptide may be an atom (isotope) having the same atomic number (the number of protons) and a different mass number (the sum of numbers of protons and neutrons). Examples of the isotope that is contained in the "amino acid" constituting the peptide include a hydrogen atom, a carbon atom, a nitrogen atom, an oxygen atom, a phosphorus atom, a sulfur atom, a fluorine atom, a chlorine atom, and the like, and they include ²H, ³H; ¹³C, ¹⁴C; ¹⁵N; ¹⁷O, ¹⁸O; ³¹P, ³²P; ³⁵S; ¹⁸F; ³⁶Cl; and the like, respectively.

Examples of the "halogen atom" as used herein include F, Cl, Br and I.

The term "alkyl" as used herein is a monovalent group derived from an aliphatic hydrocarbon by removing any one hydrogen atom, and has a subset of hydrocarbyl or hydrocarbon group structures that do not contain a heteroatom (which is an atom other than carbon and hydrogen atoms) or an unsaturated carbon-carbon bond, and contain hydrogen and carbon atoms in the backbone. The alkyl includes not only a linear form but also a branched form. The alkyl is specifically alkyl having 1 to 20 carbon atoms (C₁ to C₂₀; hereinafter, "Cₚ to C_{q}" means that the number of carbon atoms is p to q), preferably C₁ to C₁₀ alkyl, more preferably C₁ to C₆ alkyl. Specific examples of alkyl include methyl, ethyl, n-propyl, i-propyl, n-butyl, s-butyl, t-butyl, isobutyl (2-methylpropyl), n-pentyl, s-pentyl (1-methylbutyl), t-pentyl (1,1-dimethylpropyl), neopentyl (2,2-dimethylpropyl), isopentyl (3-methylbutyl), 3-pentyl (1-ethylpropyl), 1,2-dimethylpropyl, 2-methylbutyl, n-hexyl, 1,1,2-trimethylpropyl, 1,2,2-trimethylpropyl, 1,1,2,2-tetramethylpropyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, and 2-ethylbutyl.

The "alkylene" as used herein means a divalent group induced by the further removal of one arbitrary hydrogen atom from the "alkynyl" described above. Specific examples of alkylene include -CH₂-, -(CH₂)₂-, -(CH₂)₃-, -CH(CH₃)CH₂-, -C(CH₃)₂-, -(CH₂)₄-, - CH(CH₃)CH₂CH₂-, -C(CH₃)₂CH₂-, -CH₂CH(CH₃)CH₂-, -CH₂C(CH₃)₂-, -CH₂CH₂CH(CH₃)-, - CH₂CH(CH₂CH₃)-, -(CH₂)₅-, -CH(CH₃)CH(CH₂CH₃)-, -(CH₂)₆-, -(CH₂)₇-, and -(CH₂)₈-. Examples of alkylene include C₁-C₈ alkylene, preferably C₁-C₆, more preferably C₁-C₄ alkylene, and most preferably C₁-C₂ alkylene.

The term "alkenyl" as used herein is a monovalent group having at least one double bond (two adjacent SP² carbon atoms). Depending on the conformation of the double bond and a substituent (if present), the geometric morphology of the double bond can assume entgegen (E) or zusammen (Z) and cis or trans conformations. The alkenyl includes not only a linear form but also a branched form. Preferred examples of alkenyl include C₂ to C₁₀ alkenyl, more preferably C₂ to C₆ alkenyl, and specific examples thereof include vinyl, allyl, 1-propenyl, 2-propenyl, 1-butenyl, 2-butenyl (which includes cis and trans), 3-butenyl, pentenyl, 3-methyl-2-butenyl, and hexenyl.

The term "alkynyl" as used herein is a monovalent group having at least one triple bond (two adjacent SP carbon atoms). The alkynyl includes not only a linear form but a branched form. Preferred examples of alkynyl include C₂ to C₁₀ alkynyl, more preferably C₂ to C₆ alkynyl, and specific examples thereof include ethynyl, 1-propynyl, propargyl, 3-butynyl, pentynyl, hexynyl, 3-phenyl-2-propynyl, 3-(2'-fluorophenyl)-2-propynyl, 2-hydroxy-2-propynyl, 3-(3-fluorophenyl)-2-propynyl, and 3-methyl-(5-phenyl)-4-pentynyl.

The term "cycloalkyl" as used herein means a saturated or partially saturated cyclic monovalent aliphatic hydrocarbon group and includes a monocyclic ring, a bicyclo ring, and a spiro ring. Preferred examples of cycloalkyl include C₃ to C₈ cycloalkyl, and specific examples thereof include cyclopropyl, cyclobutyl, cyclopentyl, cyclohexyl, cycloheptyl, cyclooctyl, bicyclo[2.2.1]heptyl, and spiro[3.3]heptyl.

The "aryl" as used herein means a monovalent aromatic hydrocarbon ring and is preferably C₆ to C₁₀ aryl. Specific examples of aryl include phenyl and naphthyl (e.g., 1-naphthyl and 2-naphthyl).

The term "heteroaryl" as used herein means an aromatic cyclic monovalent group containing a carbon atom as well as 1 to 5 heteroatoms. The ring may be a monocyclic ring or a condensed ring with another ring and may be partially saturated. The number of atoms forming the ring is preferably 5 to 10 (5- to 10-membered heteroaryl), more preferably 5 to 7 (5-to 7-membered heteroaryl). Specific examples of heteroaryl include furyl, thienyl, pyrrolyl, imidazolyl, pyrazolyl, thiazolyl, isothiazolyl, oxazolyl, isoxazolyl, oxadiazolyl, thiadiazolyl, triazolyl, tetrazolyl, pyridyl, pyrimidyl, pyridazinyl, pyrazinyl, triazinyl, benzofuranyl, benzothienyl, benzothiadiazolyl, benzothiazolyl, benzoxazolyl, benzoxadiazolyl, benzimidazolyl, indolyl, isoindolyl, indazolyl, quinolyl, isoquinolyl, cinnolinyl, quinazolinyl, quinoxalinyl, benzodioxolyl, indolizinyl, and imidazopyridyl.

The "alkoxy" as used herein means an oxy group to which the "alkyl" defined above is bound and is preferably C₁ to C₆ alkoxy. Specific examples of alkoxy include methoxy, ethoxy, 1-propoxy, 2-propoxy, n-butoxy, i-butoxy, s-butoxy, t-butoxy, pentyloxy, and 3-methylbutoxy.

The "alkenyloxy" as used herein means an oxy group to which the "alkenyl" defined above is bound and is preferably C₂ to C₆ alkenyloxy. Specific examples of alkenyloxy include vinyloxy, allyloxy, 1-propenyloxy, 2-propenyloxy, 1-butenyloxy, 2-butenyloxy (which includes cis and trans), 3-butenyloxy, pentenyloxy, and hexenyloxy.

The "cycloalkoxy" as used herein means an oxy group to which the "cycloalkyl" defined above is bound and is preferably C₃ to C₈ cycloalkoxy. Specific examples of cycloalkoxy include cyclopropoxy, cyclobutoxy, and cyclopentyloxy.

The "aryloxy" as used herein means an oxy group to which the "aryl" defined above is bound and is preferably C₆ to C₁₀ aryloxy. Specific examples of aryloxy include phenoxy, 1-naphthyloxy, and 2-naphthyloxy.

The "arylalkoxy" as used herein means an oxy group to which the "arylalkyl (aralkyl)" defined above is bound and is preferably C₇ to C₁₂ arylalkoxy. Specific examples of arylalkoxy include benzyloxy.

The term "amino" as used herein means -NH₂ in the narrow sense and means -NRR' in the broad sense. In this context, R and R' are each independently selected from a hydrogen atom, alkyl, alkenyl, alkynyl, cycloalkyl, heterocyclyl, aryl, or heteroaryl, or R and R' form a ring together with the nitrogen atom to which they are bound. Preferred examples of amino include -NH₂, mono-C₁ to C₆ alkylamino, di-Ci to C₆ alkylamino, and 4- to 8-membered cyclic amino.

The "monoalkylamino" as used herein means a group of the "amino" defined above in which R is a hydrogen atom, and R' is the "alkyl" defined above, and is preferably mono-C₁ to C₆ alkylamino. Specific examples of monoalkylamino include methylamino, ethylamino, n-propylamino, i-propylamino, n-butylamino, s-butylamino, and t-butylamino.

The term "dialkylamino" as used herein means a group of the "amino" defined above in which R and R' are each independently the "alkyl" defined above, and is preferably di-C₁-C₆ alkylamino. Specific examples of dialkylamino include dimethylamino and diethylamino.

The "aminoalkyl" as used herein means the "alkyl" defined above whose one or more hydrogen atoms are replaced by the "amino" defined above, and is preferably C₁-C₆ aminoalkyl. Specific examples of aminoalkyl include 1-pyridylmethyl, 2-(1-piperidyl)ethyl, 3-(1-piperidyl)propyl, and 4-aminobutyl.

The "aralkyl (arylalkyl)" as used herein means a group formed by replacing at least one hydrogen atom of the "alkyl" defined above by the "aryl" defined above, and is preferably C₇ to C₁₄ aralkyl, more preferably C₇ to C₁₀ aralkyl. Specific examples of aralkyl include benzyl, phenethyl, and 3-phenylpropyl.

The term "alkylaryl" as used herein means a group formed by replacing at least one hydrogen atom of the "aryl" as defined above by the "alkyl" as defined above. Specific examples of alkylaryl include ortho-tolyl, meta-tolyl, and para-tolyl.

The alkylaryl is, for example, C₇-C₁₂ alkylaryl, preferably C₇-C₁₀ alkylaryl, more preferably C₇-C₉ alkylaryl, and most preferably C₇-C₈ alkylaryl.

Specific examples of the unnatural amino acids can also be found in WO 2013/100132, WO 2018/143145, and the like. Specific examples of the unnatural amino acid can also include Ser(tBuOH), Ser(iPen), MeSer(iPen), Ser(nPr), Ser(NtBu-Aca), MeSer(tBuOH), MeSer(nPr), MeCys(StBu), MeGly, nBuGly, MeOEtGly, MeOPrGly, PhPrGly, PhOEtGly, Nle, Ser(3F5MePyr), Ser(Ph2Cl), MePhe, MeHph, MeAla, MeAla(3Pyr), Pic(2), D-Nva, D-Ser(Me), Hph, D-Hph, D-Ser(Ph), D-MeAbu, D-MeSer, Pro4RBn, Hyp(Ph), MePhe(3-Cl), MeHph(4-Cl), Hyp(Et), and EtOEtGly. These unnatural amino acids may be contained, for example, in the second or third position counting from the N-terminus of the peptide.

At least one atom constituting an "amino acid" constituting a peptide may be an atom (isotope) having the same atomic number (the number of protons) and a different mass number (the sum of numbers of protons and neutrons). Examples of the isotope that is contained in an "amino acid" constituting a peptide herein include a hydrogen atom, a carbon atom, a nitrogen atom, an oxygen atom, a phosphorus atom, a sulfur atom, a fluorine atom, a chlorine atom, and the like, and they include ²H, ³H; ¹³C, ¹⁴C; ¹⁵N; ¹⁷O, ¹⁸O; ³¹P, ³²P; ³⁵S; ¹⁸F; ³⁶Cl; and the like, respectively.

### Method for producing peptide

One embodiment of the present invention comprises a method for producing a peptide, comprising a step of translating a nucleic acid that comprises a codon encoding a first unnatural amino acid and a codon encoding a second unnatural amino acid which are adjacent to each other, wherein at least one of the first unnatural amino acid and the second unnatural amino acid is an unnatural amino acid (unnatural amino acid U1) that meets the following requirement (A) and meets at least one selected from the group consisting of the following requirements (B) and (C).
(A) A nitrogen atom constituting a main-chain amino group (nitrogen atom N1) and an oxygen atom other than an oxygen atom constituting a main-chain carboxy group (oxygen atom O1) are bound via two atoms to each other.
(B) The oxygen atom O1 constitutes an alkoxy group or an aryloxy group.
(C) The nitrogen atom N1 is bound to a linear or branched C₁-C₆ alkyl group.

### Peptide

The term "peptide" as used herein refers to one in which two or more amino acids are linked by an amide bond. Peptides having an ester bond in a portion of the main chain, as like a depsipeptide, are also included in the "peptide" herein. Without limitation, the peptide in the present disclosure include a linear peptide and a cyclic peptide. The peptide may be in the form of a pharmaceutically acceptable salt thereof.

The peptide as used herein, in one aspect, has 2 to 100, 3 to 50, 4 to 30, or 5 to 30 amino acids linked by an amide bond and/or an ester bond. In one aspect, examples of the number of amino acids constituting a peptide include 30 or less, 20 or less, and 16 or less. Examples of the number of amino acids constituting the peptide include 5 or more, 8 or more, and 9 or more. Examples of the number of amino acids constituting the peptide include 5 to 30, 8 to 20, 8 to 16, and 9 to 16.

In one aspect, the number of amino acids constituting the peptide herein is, for example, 5 to 30, preferably 8 to 20, more preferably 8 to 16, and most preferably 9 to 16.

The term "cyclic peptide" as used herein is a peptide having a cyclic structure composed of 4 or more amino acid residues. Such a cyclic structure is also referred to as a "cyclic portion". As an aspect of cyclization of the cyclic peptide, it may take any form, such as cyclization with a carbon-nitrogen bond such as an amide bond, cyclization with a carbon-oxygen bond such as an ester bond or ether bond, cyclization with a carbon-sulfur bond such as a thioether bond, cyclization with a carbon-carbon bond, or cyclization by heterocyclic construction. Among these, cyclization via a covalent bond such as an amide bond, a carbon-sulfur bond, or a carbon-carbon bond is preferred. Cyclization with an amide bond is more particularly preferred, and the position of the carboxy group or amino group used for cyclization may be on the main chain or on a side chain. Cyclization via an amide bond between a carboxyl group on a side chain and an amino group at the N-terminus of the main chain is more preferred. The cyclic structure is different from the cyclic structure contained in phenylalanine, tyrosine, proline, and the like (e.g., benzene ring, pyrrolidine ring). The number of amino acids constituting the cyclic portion of a cyclic peptide is not limited, but examples thereof include 5 or more, 6 or more, 8 or more, and 9 or more. Examples thereof also include 30 or less, 20 or less, 16 or less, and 11 or less. The number of amino acid residues constituting the cyclic portion is preferably 5 to 20, more preferably 8 to 16, and most preferably 9 to 16. In one aspect, the total number of amino acid residues constituting the cyclic portion of the cyclic peptide is 5 or more and 30 or less, of which 5 or more and 15 or less amino acid residues constitute the cyclic structure.

In one aspect, the total number of amino acid residues constituting the cyclic peptide is, for example, 5 to 30, preferably 8 to 20, more preferably 8 to 16, and most preferably 9 to 16.

In one aspect, the cyclic peptide may have a linear portion. The "linear portion" used herein in referring to a partial structure of the cyclic peptide refers to a portion that is not included in the main chain structure of the cyclic portion and has at least one amide bond and/or ester bond on the chain of the portion. As the linear portion, it is preferred that two or more amino acid residues are linked by an amide bond. In this case, the peptide may have an ester bond in part of the main chain, as like a depsipeptide. The number of amino acid residues (number of units) of the linear portion is, for example, 0 to 8, preferably 0 to 8, more preferably 0 to 5, and most preferably 0 to 3. In one non-limiting aspect, the linear portion as used herein may contain natural amino acids and unnatural amino acids (including chemically modified or skeleton-transformed amino acids).

In one aspect, the peptides include a peptide having one or more N-substituted amino acid residues. In one aspect, examples of the number of N-substituted amino acid residues of the peptide is 1 to 13, preferably 3 to 12, more preferably 4 to 11, and most preferably 5 to 10.

In one aspect, the peptide has a ClogP of, for example, 4 to 25, preferably 6 to 23, more preferably 8 to 21, and most preferably 9 to 20. ClogP is a computer calculated partition coefficient and can be determined according to the principles described in "CLOGP Reference Manual Daylight Version 4.9 (release date: August 1, 2011, https://www.daylight.com/dayhtml/doc/clogp/)". Examples of the method for calculating ClogP include calculating ClogP using Daylight Version 4.95 (release date: August 1, 2011, ClogP algorithm version 5.4, database version 28, https://www.daylight.com/dayhtml/doc/release_notes/index.html) of Daylight Chemical Information Systems, Inc. In one aspect, the ClogP of the peptide is, for example, 28% to 174%, preferably 42% to 160%, more preferably 56% to 146%, and most preferably 63% to 139% with respect to the ClogP value of cyclosporine A (ClogP: 14.36).

In one aspect, the ClogP (ClogP/number of amino acid residues) of the peptide per constituent amino acid residue is 1.0 or more, preferably 1.1 or more. The upper limit of ClogP/number of amino acid residues of the peptide according to the present embodiments is preferably 1.8 or less, more preferably 1.7 or less, further preferably 1.6 or less, and still more preferably 1.5 or less. The range of the number of ClogP/amino acid residues of the peptide according to the present embodiments is, for example, 1.0 to 1.8, preferably 1.0 to 1.7, more preferably 1.1 to 1.6, and most preferably 1.1 to 1.5.

In one aspect, the peptide has a molecular weight of, for example, 500 g/mol to 2000 g/mol, preferably 1000 g/mol to 1800 g/mol, more preferably 1300 g/mol to 1600 g/mol, and most preferably 1400 g/mol to 1500 g/mol.

The molecular weight as used herein means the sum of the atomic weights of the atoms constituting the compound molecule (unit: "g/mol"), and is obtained by calculating the sum of the atomic weights of the atoms included in the molecular structure formula (unit: "g/mol"). The unit of molecular weight is sometimes omitted herein. The molecular weight of the peptide according to the present embodiments can be measured by any method known in the art, but preferably can be measured by liquid chromatography, and more preferably by liquid chromatography-mass spectrometry (LC/MS) as described in Examples.

In one aspect, examples of the number of unnatural amino acids contained in the peptide include 2 or more, 4 or more, 5 or more, 6 or more, 7 or more, or 8 or more. Examples thereof include 20 or less, 15 or less, 14 or less, 13 or less, 12 or less, 10 or less, or 9 or less. Examples of the number of unnatural amino acids contained in the peptide in the present disclosure include 30% or more, 40% or more, 50% or more, 60% or more, 70% or more, or 80% or more of the number of amino acids constituting the cyclic portion of the cyclic peptide. Examples of the number of types of the unnatural amino acid contained in the peptide in the present disclosure include 1 or more, 2 or more, 3 or more, 4 or more, 7 or more, or 8 or more, and 20 or less, 15 or less, 14 or less, 13 or less, 12 or less, 10 or less, or 9 or less.

In one aspect, the number of the unnatural amino acids contained in the peptide is, for example, 1 to 13, preferably 3 to 12, more preferably 4 to 11, and most preferably 5 to 10.

The moiety containing an unnatural amino acid in a peptide containing one or more unnatural amino acids in the present disclosure is not limited, but in one aspect, an unnatural amino acid may be contained at the position of the starting amino acid and may be contained at the position of the second and/or third amino acids.

The peptide produced by the method according to the present embodiments has an amino acid sequence comprising a first unnatural amino acid and a second unnatural amino acid at adjacent positions. At least one of the first unnatural amino acid and the second unnatural amino acid is an unnatural amino acid that meets the above requirement (A) and meets at least one selected from the group consisting of the above requirements (B) and (C) (hereinafter the unnatural amino acid is also referred to as "unnatural amino acid U1"). The peptide may comprise any unnatural amino acid in addition to the first unnatural amino acid and the second unnatural amino acid described above.

As used herein, "a nitrogen atom constituting a main-chain amino group (hereinafter also referred to as "nitrogen atom N1") and an oxygen atom other than an oxygen atom constituting a main-chain carboxy group (hereinafter also referred to as "oxygen atom O1") are bound via two atoms to each other" means that, in the chemical structure of amino acid, the oxygen atom O1 is bound via two atoms counting from the nitrogen atom constituting the main-chain amino group. At this time, the nitrogen atom constituting the main-chain amino group is not included in the number. The "oxygen atom constituting a main-chain carboxy group" is an oxygen atom constituting a carboxy group corresponding to the C-terminus of an amino acid and is not a carboxy group present in a side chain such as one in glutamic acid, aspartic acid, and the like.

Specific examples are described below with numbers shown in the following formulas (i) to (iii). The formulas (i) to (iii) are schematic diagrams for describing the requirement (A), wherein X represents the oxygen atom O1; R^{A}, R^{B}, R^{C}, and R^{D} each represent any substituent; and p represents an integer of 1 or more. The above unnatural amino acid U1 is not limited to these formulas and is understood with reference to these specific examples.

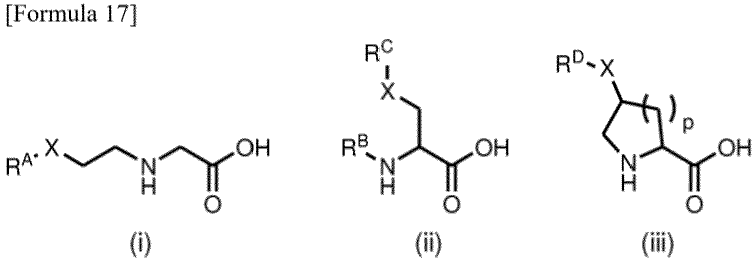

The formula (i) represents N-substituted glycine, and the third atom X counting from the nitrogen atom constituting the main-chain amino group (denoted as NH in the formula) corresponds to the oxygen atom O1. The formula (ii) represents N,O-disubstituted-D-serine, and the third atom X counting from the nitrogen atom constituting the main-chain amino group (denoted as NH in the formula) corresponds to the oxygen atom O1. The formula (iii) represents 4-substituted-proline (when p = 1), and the third atom X counting the carbon atoms on the pyrrolidine ring in the shortest distance corresponds to the oxygen atom O1. In this way, in the case of cyclic amino acids, the number of atoms present between the nitrogen atom N1 and the oxygen atom O1 can vary depending on the counting method, but in this case, the smallest number is adopted. That is, the number of atoms is counted so that the distance between the nitrogen atom N1 and the oxygen atom O1 becomes the shortest.

The "oxygen atom O1 constitutes an alkoxy group or an aryloxy group" as used herein means that the oxygen atom O1 is an oxy group. In other words, the oxygen atom O1 is not an oxygen atom that constitutes a hydroxyl group, a carbonyl group, a carboxy group, a nitro group, or the like.

The "nitrogen atom N1 is bound to a linear or branched C₁-C₆ alkyl group" as used herein means that the N-terminal amino group is not an unsubstituted amino group (-NH₂) but an amino group substituted with a linear or branched C₁-C₆ alkyl group. Referring to the above formulas (i) to (iii) as an example, R^{A} and R^{B} each are a linear or branched C₁-C₆ alkyl group.

In one aspect, the nitrogen atom N1 constitutes an unsubstituted amino group or is substituted with a methyl or ethyl group. In a preferred embodiment, the nitrogen atom N1 is substituted with a methyl group.

In one aspect, one of the first unnatural amino acid and the second unnatural amino acid is the unnatural amino acid U1, and the other is an unnatural amino acid that is different from the unnatural amino acid U1 (hereinafter also referred to as "unnatural amino acid U2"). The unnatural amino acid U2 may be any unnatural amino acid that is not identical to the unnatural amino acid U1. Both the first unnatural amino acid and the second unnatural amino acid may be the unnatural amino acid U1.

In one aspect, the unnatural amino acid U1 may meet both the above requirements (A) and (B), may meet both the above requirements (A) and (C), and may meet all of the above requirements (A), (B), and (C). In a preferred aspect, the unnatural amino acid U1 is a D-α-amino acid, a D-amino acid, or an L-amino acid (but not a natural amino acid).

In one aspect, in the unnatural amino acid U1, the oxygen atom O1 may be present in a substituent bound to the nitrogen atom N1. Examples in this case include amino acids represented by the above formula (i).

In one aspect, in the unnatural amino acid U1, the oxygen atom O1 may be present in a side chain of the unnatural amino acid U1. Examples in this case include amino acids represented by the above formula (ii).

In one aspect, in the unnatural amino acid U1, the nitrogen atom N1, a carbon atom constituting the main chain of the unnatural amino acid U1, and an atom constituting a side chain of the unnatural amino acid U1 together form a ring. Examples in this case include amino acids represented by the above formula (iii).

In one aspect, in the unnatural amino acid U1, there is only one oxygen atom corresponding to the oxygen atom O1.

In one aspect, the oxygen atom O1, together with a linear or branched C₁-C₆ alkyl group, a C₃-C₁₀ cycloalkyl group, a C₆-C₁₀ aryl group, a C₇-C₁₂ arylalkyl group, or a C₇-C₁₂ alkylaryl group, may constitute a corresponding C₁-C₆ alkoxy group, a corresponding C₃-C₁₀ cycloalkoxy group, a corresponding C₆-C₁₀ aryloxy group, a corresponding C₇-C₁₂ arylalkyloxy group, or a corresponding C₇-C₁₂ alkylaryloxy group. In one aspect, the oxygen atom O1 may constitute a methoxy group, an ethoxy group, a phenoxy group, or a benzyloxy group. In a more preferred embodiment, the oxygen atom O1 may constitute a methoxy group or a phenoxy group.

In one aspect, the oxygen atom O1, together with a linear or branched C₁-C₆ alkyl group, may constitute a corresponding C₁-C₆ alkoxy group. In a preferred aspect, the oxygen atom O1 may constitute a methoxy group or an ethoxy group. In a more preferred aspect, the oxygen atom O1 may constitute a methoxy group.

In one aspect, the oxygen atom O1, together with a C₆-C₁₀ aryl group, constitutes a corresponding C₆-C₁₀ aryloxy group. In a preferred aspect, the oxygen atom O1 may constitute a phenoxy group. In one aspect, the oxygen atom O1, together with a hydrogen atom, may constitute a corresponding hydroxy group.

In certain embodiments, the unnatural amino acid U1 does not comprise a heteroatom other than a nitrogen atom and an oxygen atom. Examples of such a heteroatom include a sulfur atom.

In certain embodiments, one of the first unnatural amino acid and the second unnatural amino acid is the unnatural amino acid U1, and the other is the unnatural amino acid U2; and a nitrogen atom constituting a main-chain amino group of the unnatural amino acid U2 is substituted with a linear or branched C₁-C₆ alkyl group, preferably with a methyl group or an ethyl group, and more preferably with a methyl group.

In certain embodiments, one of the first unnatural amino acid and the second unnatural amino acid is the unnatural amino acid U1, and the other is the unnatural amino acid U2; the unnatural amino acid U2 has, as a side chain, a linear or branched C₁-C₆ alkyl group that may be substituted with a 5- to 10-membered aryl group, or a 5- to 10-membered aryl group that may be substituted with a linear or branched C₁-C₆ alkyl group; preferably, the unnatural amino acid U2 has, as a side chain, a linear or branched C₁-C₄ alkyl group, or a 5- to 10-membered aryl group that may be substituted with a linear or branched C₁-C₃ alkyl group; and more preferably, the unnatural amino acid U2 has, as a side chain, a methyl group or a benzyl group.

In certain embodiments, one of the first unnatural amino acid and the second unnatural amino acid is the unnatural amino acid U1, and the other is the unnatural amino acid U2; and the unnatural amino acid U2 is an α-amino acid. In certain embodiments, a nitrogen atom constituting a main-chain amino group of the unnatural amino acid U2 is substituted with a methyl group, and the unnatural amino acid U2 is an α-amino acid having a methyl group as a side chain. In certain embodiments, a nitrogen atom constituting a main-chain amino group of the unnatural amino acid U2 is substituted with a methyl group, and the unnatural amino acid U2 is an α-amino acid having a benzyl group as a side chain.

In the amino acid sequence of the peptide produced in the present embodiments, the first unnatural amino acid and the second unnatural amino acid are adjacent to each other; the first unnatural amino acid may be on the N-terminal side, and the second unnatural amino acid may be on the C-terminal side; and the second unnatural amino acid may be on the N-terminal side, and the first unnatural amino acid may be on the C-terminal side. In the amino acid sequence of the peptide, the unnatural amino acid U1 may be on the N-terminal side, and the unnatural amino acid U2 may be on the C-terminal side; and the unnatural amino acid U2 may be on the N-terminal side, and the unnatural amino acid U1 may be on the C-terminal side.

In one aspect of the method for producing a peptide according to the present embodiments, the first unnatural amino acid is the unnatural amino acid U1, and the second unnatural amino acid is the unnatural amino acid U2; the unnatural amino acid U1 meets the requirements (A) and (B); the oxygen atom O1 is present in a substituent substituted with a nitrogen atom N1; the oxygen atom O1 constitutes a methoxy group or a phenoxy group; and the codon encoding the first unnatural amino acid is translated before the codon encoding the second unnatural amino acid.

In one aspect of the method for producing a peptide according to the present embodiments, the first unnatural amino acid is the unnatural amino acid U1, and the second unnatural amino acid is the unnatural amino acid U2; the unnatural amino acid U1 meets the requirements (A) and (B); the oxygen atom O1 is contained in a side chain of the unnatural amino acid U1; the oxygen atom O1 constitutes a methoxy group or a phenoxy group; the nitrogen atom N1 constitutes an unsubstituted amino group; and the codon encoding the first unnatural amino acid is translated before the codon encoding the second unnatural amino acid.

In one aspect of the method for producing a peptide according to the present embodiments, the first unnatural amino acid is the unnatural amino acid U1, and the second unnatural amino acid is the unnatural amino acid U2; the unnatural amino acid U1 meets the requirements (A) and (C); the oxygen atom O1 is contained in a side chain of the unnatural amino acid U1; the oxygen atom O1 constitutes a hydroxyl group; the nitrogen atom N1 is substituted with a methyl group or an ethyl group; and the codon encoding the first unnatural amino acid is translated before the codon encoding the second unnatural amino acid.

In one aspect of the method for producing a peptide according to the present embodiments, the first unnatural amino acid is the unnatural amino acid U1, and the second unnatural amino acid is the unnatural amino acid U2; the unnatural amino acid U1 meets the requirements (A) and (B); in the unnatural amino acid U 1, the nitrogen atom N1, a carbon atom constituting a main chain of the unnatural amino acid U1, and an atom constituting a side chain of the unnatural amino acid U1 together form a ring; the oxygen atom O1 constitutes a methoxy group or a phenoxy group; and the codon encoding the first unnatural amino acid is translated before the codon encoding the second unnatural amino acid.

In one aspect of the method for producing a peptide according to the present embodiments, the method comprises a step of translating a nucleic acid that comprises a codon encoding a first unnatural amino acid and a codon encoding a second unnatural amino acid which are adjacent to each other; the first unnatural amino acid is the unnatural amino acid U1, and the second unnatural amino acid is the unnatural amino acid U2; the unnatural amino acid U1 is an α-amino acid; in the unnatural amino acid U1, a nitrogen atom constituting a main-chain amino group (nitrogen atom N1) and an oxygen atom other than an oxygen atom constituting a main-chain carboxy group (oxygen atom O1) are bound via two or three atoms to each other; the oxygen atom O1 is contained in a side chain of the unnatural amino acid U1; the oxygen atom O1 constitutes a hydroxyl group; the nitrogen atom N1 is substituted with a methyl group or an ethyl group; and the codon encoding the first unnatural amino acid is translated before the codon encoding the second unnatural amino acid.

In one aspect of the method for producing a peptide according to the present embodiments, the first unnatural amino acid is the unnatural amino acid U1, and the second unnatural amino acid is the unnatural amino acid U2; the unnatural amino acid U1 meets the requirements (A) and (B); the oxygen atom O1 is present in a substituent substituted with a nitrogen atom N1; the oxygen atom O1 constitutes a methoxy group or a phenoxy group; and the codon encoding the first unnatural amino acid is translated after the codon encoding the second unnatural amino acid.

In one aspect of the method for producing a peptide according to the present embodiments, the first unnatural amino acid is the unnatural amino acid U1, and the second unnatural amino acid is the unnatural amino acid U2; the unnatural amino acid U1 meets the requirements (A) and (B); the oxygen atom O1 is contained in a side chain of the unnatural amino acid U1; the oxygen atom O1 constitutes a methoxy group or a phenoxy group; the nitrogen atom N1 constitutes an unsubstituted amino group; and the codon encoding the first unnatural amino acid is translated after the codon encoding the second unnatural amino acid.

In one aspect of the method for producing a peptide according to the present embodiments, the first unnatural amino acid is the unnatural amino acid U1, and the second unnatural amino acid is the unnatural amino acid U2; the unnatural amino acid U1 meets the requirements (A) and (C); the oxygen atom O1 is contained in a side chain of the unnatural amino acid U1; the oxygen atom O1 constitutes a hydroxyl group; the nitrogen atom N1 is substituted with a methyl group or an ethyl group; and the codon encoding the first unnatural amino acid is translated after the codon encoding the second unnatural amino acid.

In one aspect of the method for producing a peptide according to the present embodiments, the first unnatural amino acid is the unnatural amino acid U1, and the second unnatural amino acid is the unnatural amino acid U2; the unnatural amino acid U1 meets the requirements (A) and (B); in the unnatural amino acid U1, the nitrogen atom N1, a carbon atom constituting a main chain of the unnatural amino acid U1, and an atom constituting a side chain of the unnatural amino acid U1 together form a ring; the oxygen atom O1 constitutes a methoxy group or a phenoxy group; and the codon encoding the first unnatural amino acid is translated after the codon encoding the second unnatural amino acid.

In certain embodiments, at least one of the first unnatural amino acid and the second unnatural amino acid is the unnatural amino acid U1, and the unnatural amino acid U1 is an unnatural amino acid represented by the following formula (I).

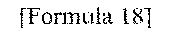

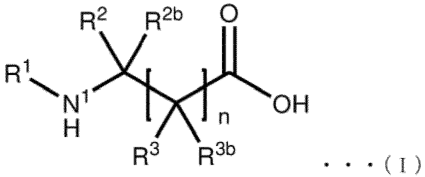

In the formula (I), n is an integer of 0 or more and 2 or less, preferably an integer of 0 or more and 2 or less, more preferably an integer of 0 or more and 1 or less, and most preferably 0.

In the formula (I), R¹ is (a) a group represented by formula (II), (b) a hydrogen atom, or (c) a linear or branched C₁-C₆ alkyl group. In certain embodiments, R¹ is not a hydrogen atom.

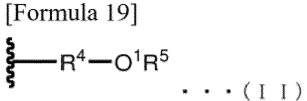

In the formula (II), 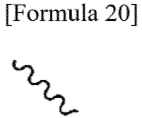 represents a binding position with N¹.

In the formula (II), R⁴ is a C₁-C₂ alkylene group.

In the formula (II), R⁵ is a hydrogen atom, a linear or branched C₁-C₆ alkyl group, a C₃-C₁₀ cycloalkyl group, a C₆-C₁₀ aryl group, a C₇-C₁₂ arylalkyl group, or a C₇-C₁₂ alkylaryl group, preferably a methyl group, an ethyl group, a phenyl group, or a benzyl group, and more preferably a methyl group or a phenyl group.

In one aspect, R⁵ is a linear or branched C₁-C₆ alkyl group, preferably a methyl group or an ethyl group, and more preferably a methyl group. In certain embodiments, R⁵ is a C₆-C₁₀ aryl group, and preferably a phenyl group. In certain embodiments, R⁵ is a hydrogen atom.

In the formula (I), R² and R³ are each independently a group represented by the formula (II), a hydrogen atom, or a linear or branched C₁-C₄ alkyl group, and preferably a group represented by the formula (II) or a hydrogen atom. In one aspect, at least one of R² and R³ may be a group represented by the formula (II).

In the formula (1), R^{2b} and R^{3b} are each independently a hydrogen atom or a linear or branched C₁-C₄ alkyl group, and preferably a hydrogen atom.

In the formula (I), R² and R^{2b} or R³ and R^{3b} may form a ring together with a carbon atom to which they are bound.

One or more of R¹, R², and R³ is a group represented by the formula (II), and N¹ (nitrogen atom N1) in the formula (I) and O¹ (oxygen atom O1) in the formula (II) are bound via two atoms to each other. N¹ represents a nitrogen atom and O¹ represents an oxygen atom.

When R¹ is a group represented by the formula (II), R⁴ and R² may form a ring together with a carbon atom to which R² is bound and N¹. Examples in this case include amino acids represented by the above formula (iii).

When R² is a substituent represented by the formula (II), at least one of R¹ in the formula (I) and R⁵ in the formula (II) is not a hydrogen atom. In one aspect, R¹ in the formula (I) is not a hydrogen atom. In certain embodiments, R⁵ in the formula (II) is not a hydrogen atom. In another aspect, neither R¹ in the formula (I) nor R⁵ in the formula (II) is a hydrogen atom.

When n is 2 or more, a plurality of R³ and R^{3b} may be present. When a plurality of R³ is present, they are each independently the same as or different from each other, and when a plurality of R^{3b} is present, they are each independently the same as or different from each other.

In one aspect, the unnatural amino acid U1 is an amino acid represented by the following formula (III), (IV), or (V). In one aspect, the unnatural amino acid U1 is an amino acid represented by the following formula (III). In one aspect, the unnatural amino acid U1 is an amino acid represented by the following formula (IV). In one aspect, the unnatural amino acid U1 is an amino acid represented by the following formula (V). In the formulas (III), (IV) and (V), R^{1b} is a hydrogen atom or a linear or branched C₁-C₆ alkyl, and R⁵ has the same meaning as R⁵ in the formula (II).

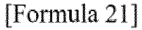

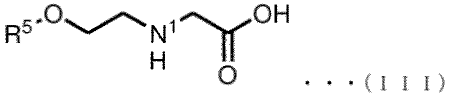

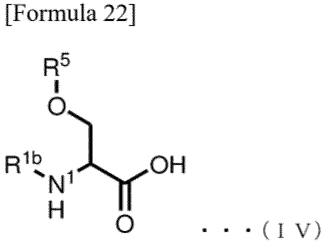

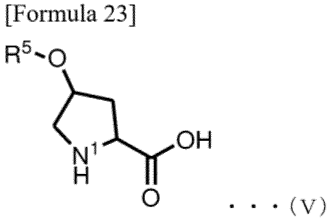

In one aspect, the unnatural amino acid U1 is an amino acid represented by the formula (III), (IVb) or (Vb). In one aspect, the unnatural amino acid U1 is an amino acid represented by the formula (IVb). In one aspect, the unnatural amino acid U1 is an amino acid represented by the formula (Vb). In the formulas (III), (IVb) and (Vb), R^{1b} is a hydrogen atom or a linear or branched C₁-C₆ alkyl, and R⁵ has the same meaning as R⁵ in the formula (II).

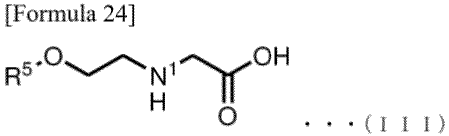

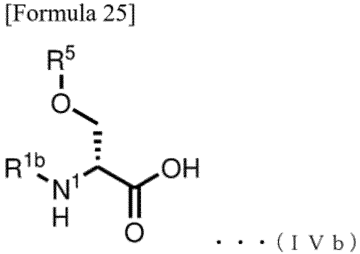

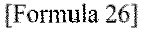

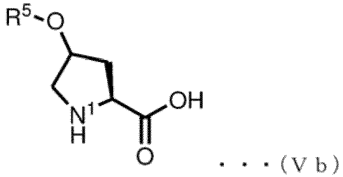

In one aspect, R¹ and R^{1b} each are a methyl group or an ethyl group, preferably a methyl group. In one aspect, R¹ and R^{1b} each are a hydrogen atom.

In one aspect, R⁵ is a linear or branched C₁-C₆ alkyl group, a C₃-C₁₀ cycloalkyl group, a C₆-C₁₀ aryl group, a C₇-C₁₂ arylalkyl group, or a C₇-C₁₂ alkylaryl group; and R¹ and R^{1b} each are a hydrogen atom. In a preferred aspect, R⁵ is a linear or branched C₁-C₆ alkyl group, a C₆-C₁₀ aryl group, or a C₇-C₁₂ arylalkyl group; and R¹ and R^{1b} each are a hydrogen atom. In a more preferred aspect, R⁵ is a methyl group, an ethyl group, a phenyl group or a benzyl group; and R¹ and R^{1b} each are a hydrogen atom. In a further preferred aspect, R⁵ is a methyl group or a phenyl group; and R' and R^{1b} each are a hydrogen atom.

In one aspect, one of the first unnatural amino acid and the second unnatural amino acid is the unnatural amino acid U1, and the other is the unnatural amino acid U2"; and the unnatural amino acid U2 is represented by the following formula (VI).

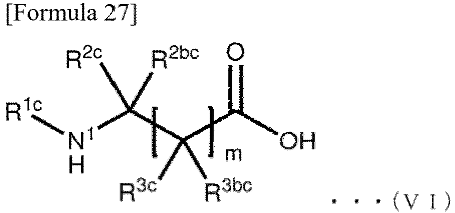

In the formula (VI), m is an integer of 0 or more and 2 or less, preferably 0 or 1, and most preferably 0.

In the formula (VI), R^{1c} is a hydrogen atom or a linear or branched C₁-C₆ alkyl group, preferably a linear or branched C₁-C₆ alkyl group, more preferably a methyl group or an ethyl group, and particularly preferably a methyl group.

In the formula (VI), R^{2c} and R^{3c} are each independently a hydrogen atom, a linear or branched C₁-C₆ alkyl group that may be substituted with a 5- to 10-membered aryl group, or a 5-to 10-membered aryl group that may be substituted with a linear or branched C₁-C₆ alkyl group; preferably, a linear or branched C₁-C₆ alkyl group that may be substituted with a 5- to 10-membered aryl group, or a 5- to 10-membered aryl group that may be substituted with a linear or branched C₁-C₆ alkyl group; and more preferably, a linear or branched C₁-C₄ alkyl group, or a 5-to 10-membered aryl group that may be substituted with a linear or branched C₁-C₃ alkyl group. In a preferred aspect, R^{2c} and R^{3c} are each independently a methyl group or a benzyl group. In one aspect, R^{2c} and R^{3c} each are a methyl group. In one aspect, R^{2c} and R^{3c} each are a benzyl group.

In the formula (VI), R^{2bc} and R^{3bc} are each independently a hydrogen atom or a linear or branched C₁-C₄ alkyl group, and preferably a hydrogen atom.

R^{2c} and R^{2bc} or R^{3c} and R^{3bc} may form a ring together with a carbon atom to which they are bound.

A nitrogen atom to which R^{1c} is bound, R^{1c}, a carbon atom to which R^{2c} is bound, and R^{2c} may together form a ring.

When m is 2, R³ and R^{3b} may be present two by two. When a plurality of R^{3c} is present, they are each independently the same as or different from each other, and when a plurality of R^{3bc} is present, they are each independently the same as or different from each other.

In one aspect, R^{1c} and R^{2c} each are a methyl group, and m is 0. In one aspect, R^{1c} is a methyl group, R^{2c} is a benzyl group, and m is 0.

### [Method for Producing Peptide]

The method for producing a peptide according to the present embodiments comprises a step of translating a nucleic acid that comprises a codon encoding a first unnatural amino acid and a codon encoding a second unnatural amino acid which are adjacent to each other. The codon encoding the first unnatural amino acid may be translated before the codon encoding the second unnatural amino acid, and the codon encoding the first unnatural amino acid may be translated after the codon encoding the second unnatural amino acid. That is, either of each codon encoding the first unnatural amino acid and the second unnatural amino acid may be at the 5' end of mRNA.

When one of the first unnatural amino acid and the second unnatural amino acid is the unnatural amino acid U1, and the other is the unnatural amino acid U2, the codon encoding the unnatural amino acid U1 may be translated before the codon encoding the unnatural amino acid U2, and the codon encoding the unnatural amino acid U1 may be translated after the codon encoding the unnatural amino acid U2. That is, either of each codon encoding the unnatural amino acid U1 and the unnatural amino acid U2 may be at the 5' end of mRNA. Preferably, a codon encoding the unnatural amino acid U1 is translated before a codon encoding the unnatural amino acid U2.

### mRNA Encoding Peptide

In the present specification, a peptide is produced by translating an mRNA encoding a peptide containing a particular unnatural amino acid. The mRNA is an RNA having genetic information that can be translated into a protein. The genetic information is encoded on the mRNA as codons (the order of three nucleotides), which typically correspond to all 20 amino acids, respectively. The translation of protein is initiated with a start codon and is terminated with a stop codon. The start codon in eukaryotes is generally AUG, but in prokaryotes (eubacteria and archaea), in addition to AUG, GUG and UUG are sometimes used as start codons. AUG is a codon encoding methionine (Met), and translation is initiated from methionine as-is in eukaryotes and archaea. On the other hand, in eubacteria, only the start codon AUG corresponds to N-formylmethionine (fMet), thus translation is initiated from formylmethionine. In the stop codon, there are three types: UAA (ochre), UAG (amber), and UGA (opal). Once the stop codon is recognized by a protein called a release factor (RF), the previously synthesized peptide chain dissociates from the tRNA and the translation process terminates. One example of an mRNA encoding a peptide containing a particular unnatural amino acid in one aspect can be an mRNA comprising a codon encoding a peptide containing an unnatural amino acid at least at a position of the starting amino acid.

In the translation of natural amino acids, 20 proteinaceous amino acids and termination of translation are assigned to each of the 64 codons. When aminoacylated tRNA corresponding to a specific amino acid is removed from the translation system, the codon corresponding to the specific amino acid becomes a vacant codon. When binding an unnatural amino acid of interest to a tRNA having an anticodon complementary to the vacant codon and translating it after adding to a translation system, the amino acid is encoded by the codon, and the peptide into which the unnatural amino acid of interest is introduced instead of the removed amino acid is translated.

In one aspect of the present disclosure, the initiation suppression (iSP) method is preferably used for the translation of the peptide. In usual translations, methionine is generally translated as an N-terminal amino acid as a translation initiation amino acid. There is a dedicated "starting tRNA" for initiating the translation, and translation is initiated when the starting tRNA is bound to methionine (formylmethionine in prokaryotes) and transported to a ribosome, so that the N-terminal amino acid becomes methionine (formylmethionine in prokaryotes). In contrast, in the initiation suppression method, a peptide having a desired amino acid at the N-terminus is translated by removing (or not generating) the methionine-aminoacylated starting tRNA from the translation system and adding instead a desired amino acid-aminoacylated starting tRNA, which is prepared in advance, to a translation system. Regarding the allowance of the unnatural amino acids, it is known that an unnatural amino acid that is higher in introduction to the N-terminus than in extension of amino acids and has a structure largely different from that of the natural amino acid can be used as the N-terminal amino acid (see Non Patent Literature: J Am Chem Soc. 2009 Apr 15;131(14):5040-1. Translation initiation with initiator tRNA charged with exotic peptides. Goto Y, Suga H.). In one aspect of the present embodiments, the starting tRNA contained in the translation system may be acylated with an unnatural amino acid.

### Translation System

The "translation system" herein is defined as a composition for translating a peptide. The "translation system" herein preferably comprises groups of protein factors involved in translation, tRNAs, amino acids, energy sources such as ATPs, and regeneration systems thereof that can be combined to translate an mRNA into a protein. The system in which the translation is in progress may be included in the "translation system" herein. The translation system herein may comprise a nucleic acid that serves as a template for translating a peptide, and may additionally comprise an initiation factor, an elongation factor, a dissociation factor, an aminoacyl tRNA synthetase, and the like. These factors can be obtained by purification from extracts of various cells. Examples of the cells for purifying factors can include prokaryotic cells and eukaryotic cells. Examples of the prokaryotic cells can include Escherichia coli cells, extreme thermophile cells, or Bacillus subtilis cells. As the eukaryotic cells, those made from yeast cells, wheat germ, rabbit reticulocytes, plant cells, insect cells, or animal cells are known. As the tRNA and aminoacyl tRNA synthetase (ARS), not only naturally occurring ones, but also artificial tRNAs and artificial aminoacyl tRNA synthetases that recognize an unnatural amino acid can be used. With the artificial tRNA and artificial aminoacyl tRNA synthetase, it is possible to synthesize a peptide in which an unnatural amino acid is site-specifically introduced. If necessary, RNA polymerase such as T7 RNA polymerase can be added to the translation system to perform transcription from template DNA.

The translation process in the present embodiments may be performed in a cell-free translation system. The cell-free translation system may be a reconstituted cell-free translation system, and specifically, a translation system composed of factors derived from E. coli may be used. The cell-free translation system may also be one that contains a ribosome derived from E. coli.

The cell-free translation system preferably comprises a tRNA having an anticodon that is complementary to a codon encoding the first unnatural amino acid and comprises a tRNA having an anticodon that is complementary to a codon encoding the second unnatural amino acid.

The main types of translation systems include translation systems using live cells and translation systems using cell extracts (cell-free translation systems). Examples of the translation systems using live cells include the systems in which peptide translation is performed by introducing a desired aminoacyl tRNA and an mRNA into live cells such as oocytes of African horseshoe frogs or mammalian cells by microinjection or lipofection methods (Nowak et al., Science (1995) 268: 439-442). Examples of the cell-free translation systems include those using extracts from E. coli (Chen et al., Methods Enzymol (1983) 101: 674-690), yeast (Gasior et al., J Biol Chem (1979) 254: 3965-3969), wheat germ (Erickson et al., Methods Enzymol (1983) 96: 38-50), rabbit reticulocytes (Jackson et al., Methods Enzymol (1983) 96: 50-74), HeLa cells (Barton et al., Methods Enzymol (1996) 275: 35-57), or insect cells (Swerdel et al., Comp Biochem Physiol B (1989) 93: 803-806). Such translation systems can be suitably prepared by the method known to those skilled in the art or by the method equivalent thereto. The cell-free translation systems also include translation systems in which the factors necessary for peptide translation are isolated and purified separately, and reconstituted and constructed (reconstituted cell-free translation systems) (Shimizu et al., Nat Biotech (2001) 19: 751-755). The reconstituted cell-free translation systems can typically contain ribosomes, amino acids, tRNAs, aminoacyl tRNA synthetases (aaRS), translation initiation factors (e.g., IF1, IF2, IF3), translation extension factors (e.g., EF-Tu, EF-Ts, EF-G), translation termination factors (e.g., RF1, RF2, RF3), ribosome recycling factors (RRF), NTPs as an energy source, energy regeneration systems, and other factors necessary for translation. When transcriptional reactions from DNA are performed in conjunction, RNA polymerase and the like may be further contained. The various factors contained in the cell-free translation system can be isolated and purified by methods well known to those skilled in the art, and can be used to appropriately construct a reconstituted cell-free translation system. Alternatively, commercially available reconstituted cell-free translation systems such as PUREfrex(R) manufactured by GeneFrontier Corporation and PURExpress(R) manufactured by New England Biolabs Inc. can be used. In the case of a reconstituted cell-free translation system, only the necessary components of the translation system can be reconstituted to construct a desired translation system.

PURESYSTEM(R) (manufactured by BioComber Co., Ltd.) is a reconstituted cell-free translation system in which protein factors, energy regeneration system enzymes, and ribosomes necessary for translation in E. coli are extracted and purified, and mixed with tRNA, amino acids, ATP, GTP, and the like. PURESYSTEM(R) not only has a low impurity content, but also allows easy preparation of systems that do not contain protein factors or amino acids that are desired to be eliminated, because it is a reconstitution system ((i) Nat Biotechnol. 2001; 19: 751-5. Cell-free translation reconstituted with purified components. Shimizu Y, Inoue A, Tomari Y, Suzuki T, Yokogawa T, Nishikawa K, Ueda T. (ii) Methods Mol Biol. 2010; 607: 11-21. PURE technology. Shimizu Y, Ueda T.).

For example, many methods in which a stop codon is used as a codon that introduces an unnatural amino acid have been reported. However, by using PURESYSTEM described above, a synthetic system excluding natural amino acids and ARS can be constructed. This allows an unnatural amino acid to be associated with a codon encoding the natural amino acid to be removed (J Am Chem Soc. 2005; 127: 11727-35. Ribosomal synthesis of unnatural peptides. Josephson K, Hartman MC, Szostak JW.). Furthermore, by decoding the degeneracy of the codon, an unnatural amino acids can be added without excluding natural amino acids (Kwon I, et al. Breaking the degeneracy of the genetic code. J Am Chem Soc. 2003, 125, 7512-3.). Peptides containing N-methylamino acids can be synthesized in ribosomes by using cell-free translation systems such as PURESYSTEM.

More specifically, translation synthesis can be performed by adding mRNA to a known cell-free translation system such as PURESYSTEM in which, for example, protein factors (selected from methionyl tRNA transformylase, EF-G, RF1, RF2, RF3, RRF, IF1, IF2, IF3, EF-Tu, EF-Ts, ARS (AlaRS, ArgRS, AsnRS, AspRS, CysRS, GlnRS, GluRS, GlyRS, HisRS, IleRS, LeuRS, LysRS, MetRS, PheRS, ProRS, SerRS, ThrRS, TrpRS, TyrRS, ValRS), ribosomes, amino acids, cleatin kinases, myokinase, inorganic pyrophosphatase, nucleoside diphosphate kinase, E. coli-derived tRNA, creatine phosphate, potassium glutamate, HEPES-KOH pH 7.6, magnesium acetate, spermidine, dithiothreitol, GTP, ATP, CTP, UTP, and the like, necessary for translation in E. coli, are appropriately selected and mixed. When T7 RNA polymerase is added, transcription and translation from a template DNA containing a T7 promoter can also be performed in conjugation. In addition, a peptide containing an unnatural amino acids can be translationally synthesized by adding a group of desired aminoacyl tRNAs or a group of unnatural amino acids (e.g., F-Tyr) that are allowed by aminoacyl tRNA synthetases (ARS) to the system (Kawakami T, et al. Ribosomal synthesis of polypeptoids and peptoid-peptide hybrids. J Am Chem Soc. 2008, 130, 16861-3., Kawakami T, et al. Diverse backbone-cyclized peptides via codon reprogramming. Nat Chem Biol. 2009, 5, 888-90.). Furthermore, mRNA encoding a peptide containing an unnatural amino acid can also be translated by adding a modified ARS to a system instead of or in addition to natural ARS, and adding a group of unnatural amino acids to the system. Alternatively, ribosomes and mutants of EF-Tu or the like can be used to increase the efficiency of translation of mRNA encoding a peptide containing an unnatural amino acid and the accompanying introduction of the unnatural amino acid (Dedkova LM, et al. Construction of modified ribosomes for incorporation of D-amino acids into proteins. Biochemistry 2006, 45, 15541-51., Doi Y, et al. Elongation factor Tu mutants expand amino acid tolerance of protein biosynthesis system. J Am Chem Soc. 2007, 129, 14458-62., Park HS, et al. Expanding the genetic code of Escherichia coli with phosphoserine. Science 2011, 333, 1151-4.).

The translation system may also contain a ribosome containing a modified L31 protein described in WO 2021/117848. In this case, the ribosome containing the modified L31 protein is preferably contained at a ratio of at least 50% or more, preferably 60% or more, more preferably 70% or more, further preferably 80% or more, and particularly preferably 90% or more (e.g., 91%, 92%, 93%, 94%, 95%, 96%, 97%, 98%, or 99% or more) based on the total ribosomes contained in the translation system in terms of the number of molecules. The "total ribosomes" are not particularly limited as long as they are ribosomes contained in the translation system, but examples thereof include the sum of the ribosomes containing the modified L31 protein and other ribosomes (e.g., ribosomes containing Escherichia coli wild-type L31 and functionally equivalent ribosomes thereto).

It is also preferred that the translation system contains a magnesium ion. It is known that a magnesium ion is required to maintain the association state of the small subunit and the large subunit of a ribosome. The L31 protein is one of the proteins that form the interaction between the large subunit and the small subunit of the ribosome. It has been revealed that ribosomes lacking L31 proteins and ribosomes containing short L31 require higher concentrations of magnesium ion to maintain the association state than ribosomes containing intact L31. Thus, in the production method of the present disclosure in which a ribosome containing a modified L31 protein is used, the magnesium ion concentration is preferably higher than the magnesium ion concentration in the translation system in which a ribosome containing intact L31 is used. To prepare such a translation system, the method of the present invention can further comprise the step of adding a magnesium ion to the translation system of the present invention. Alternatively, in the present invention, a translation system in which a magnesium ion is added in advance can also be used. The amount of magnesium added is not particularly limited, but examples thereof can include ranges that can be specified, for example, by any combination of a lower limit selected from a value of 1 mM or more, 2 mM or more, 3 mM or more, 4 mM or more, 5 mM or more, 6 mM or more, or 7 mM or more, and an upper limit selected from 9 mM or less, 8 mM or less, 7 mM or less, 6 mM or less, 5 mM or less, 4 mM or less, or 3 mM or less.

For example, the translation system may be a reconstituted cell-free protein synthesis system derived from a prokaryote and may contain tRNA (e.g., any aminoacylated tRNAs, artificial aminoacylated tRNAs corresponding to unnatural amino acids) and protein factors (e.g., GTP, ATP, creatine phosphate, pH buffer, potassium acetate, spermidine, dithiothreitol, enzymes (e.g., creatine kinase, myokinase, inorganic pyrophosphatase, nucleoside diphosphate kinase), and various amino acids), depending on the purpose. Specific components of the translation system may be selected based on techniques well known to those skilled in the art and commercially available translation systems may be used.

In one aspect, the cell-free translation system comprises a first tRNA to which a first unnatural amino acid is bound and a second tRNA to which a second unnatural amino acid is bound. In one aspect, the first tRNA comprises an anticodon that is complementary to a codon encoding the first unnatural amino acid, and the second tRNA comprises an anticodon that is complementary to a codon encoding the second unnatural amino acid. On the other hand, the nucleic acid sequence of the mRNA comprises a codon encoding a first unnatural amino acid and a codon encoding a second unnatural amino acid at adjacent positions. Thus, the corresponding first and second unnatural amino acids are introduced into a translated peptide when translating the mRNA. Either of the codon encoding the first unnatural amino acid and the codon encoding the second unnatural amino acid may be at the 5' end. A desired combination of unnatural amino acids can be introduced into a translated peptide in a desired order by appropriately modifying the combination of anticodons and unnatural amino acids in the first and second tRNAs depending on the sequence in the mRNA.

### tRNA

In introducing an unnatural amino acid into a peptide by translating mRNA encoding a peptide containing an unnatural amino acid, aminoacylation of tRNA which attains orthogonality and efficient incorporation into ribosomes ((i) Biochemistry 2003; 42: 9598-608. Adaptation of an orthogonal archaeal leucyl-tRNA and synthetase pair for four-base, amber, and opal suppression. Anderson JC, Schultz PG., (ii) Chem Biol. 2003; 10: 1077-84. Using a solid-phase ribozyme aminoacylation system to reprogram the genetic code. Murakami H, Kourouklis D, Suga H.) is required. The following five methods can be used as a method for aminoacylating tRNA.

Inside cells, aminoacyl tRNA synthetase (ARS) is provided for each amino acid individually as an enzyme for aminoacylation of tRNA. Thus, as the first method, a method of using certain ARS that allows unnatural amino acids such as N-MeHis, and a method of preparing and utilizing a modified aminoacyl tRNA synthetase that allows unnatural amino acids can be used ((i) Proc Natl Acad Sci U S A. 2002; 99: 9715-20. An engineered Escherichia coli tyrosyl-tRNA synthetase for site-specific incorporation of an unnatural amino acid into proteins in eukaryotic translation and its application in a wheat germ cell-free system. Kiga D, Sakamoto K, Kodama K, Kigawa T, Matsuda T, Yabuki T, Shirouzu M, Harada Y, Nakayama H, Takio K, Hasegawa Y, Endo Y, Hirao I, Yokoyama S. (ii) Science 2003; 301: 964-7. An expanded eukaryotic genetic code. Chin JW, Cropp TA, Anderson JC, Mukherji M, Zhang Z, Schultz PG. Chin, JW. (iii) Proc Natl Acad Sci U S A. 2006; 103: 4356-61. Enzymatic aminoacylation of tRNA with unnatural amino acids. Hartman MC, Josephson K, Szostak JW.). Second, a method of aminoacylating tRNA and then chemically modifying an amino acid in a test tube can be used (J Am Chem Soc. 2008; 130: 6131-6. Ribosomal synthesis of N-methyl peptides. Subtelny AO, Hartman MC, Szostak JW.). Third, binding tRNA in which CA is removed from the CCA sequence at the 3' end to an aminoacylated pdCpA prepared separately with an RNA ligase can be performed to obtain an aminoacyl tRNA (Biochemistry 1984; 23: 1468-73. T4 RNA ligase mediated preparation of novel "chemically misacylated" tRNAPheS. Heckler TG, Chang LH, Zama Y, Naka T, Chorghade MS, Hecht SM.). Aminoacylation by flexizyme, which is a ribozyme that makes active esters of various unnatural amino acids carried on tRNA, can also be used (J Am Chem Soc. 2002; 124: 6834-5. Aminoacyl-tRNA synthesis by a resin-immobilized ribozyme. Murakami H, Bonzagni NJ, Suga H.). Fourth, a method of ultrasonically mixing tRNA and amino acid active esters in a cationic micelle can also be used (Chem Commun (Camb). 2005; (34): 4321-3. Simple and quick chemical aminoacylation of tRNA in cationic micellar solution under ultrasonic agitation. Hashimoto N, Ninomiya K, Endo T, Sisido M.). Fifth, amino acid acylation is also possible by adding an amino acid active ester bound to PNA complementary to near the 3' end of tRNA to tRNA (J Am Chem Soc. 2004; 126: 15984-9. In situ chemical aminoacylation with amino acid thioesters linked to a peptide nucleic acid. Ninomiya K, Minohata T, Nishimura M, Sisido M.).

More specifically, aminoacyl tRNA can be produced by using the following method. RNA can be synthesized by preparing a template DNA that encodes a desired tRNA sequence and disposes a T7, T3 or SP6 promoter upstream and transcribing with RNA polymerases adapted for promoters, such as T7 RNA polymerase and T3, and SP6 RNA polymerase. A generated tRNA of interest can also be extracted by extracting and purifying tRNA from the cells and using probes of complementary sequences of tRNA sequence. At this time, the cells transformed with an expression vector of tRNA of interest can be used as a source. RNA of the sequence of interest can also be synthesized by chemical synthesis. For example, an aminoacyl tRNA can be obtained by binding tRNA in which CA is removed from the CCA sequence at the 3' end obtained in this way to an aminoacylated pdCpA or pCpA prepared separately with an RNA ligase (pdCpA method, pCpA method). The tRNA is useful in the production of peptides. Alternatively, full-length tRNA is prepared and aminoacylation by flexizyme, which is a ribozyme that makes active esters of various unnatural amino acids carried on tRNA, can be performed. Aminoacyl tRNA can also be produced using natural ARS or a modified one thereof, although this is not intended to be limiting. When natural ARS or a modified one thereof is used, it is not necessary to have a large amount of previously-prepared aminoacyl tRNA in the translation system, because the aminoacyl tRNA once consumed in the translation system can be regenerated by natural ARS or a modified one thereof. Such modified ARS are described in WO 2016/148044. These methods for producing aminoacyl tRNAs can also be used in appropriate combination.

The first tRNA and the second tRNA may be artificial tRNAs or may be transcribed tRNAs. The first tRNA and the second tRNA each may also have a body derived from a naturally occurring tRNA. The "body" of tRNA as used herein refers to a body portion of tRNA excluding anticodons. The "body derived from a naturally occurring tRNA" as used herein means that the body of tRNA has the same polynucleotide sequence as the body of the naturally occurring tRNA. That is, in one aspect, the first tRNA and the second tRNA may be bodies of naturally occurring tRNA bound by the unnatural amino acid.

### Cyclization of Peptide

In one aspect, the peptide is a cyclic peptide in which some or all of the amino acid residues constitute a cyclic structure. When producing a cyclic peptide, the translation step alone may not be necessarily sufficient, and the step of cyclizing the translated peptide can be further included. Examples of the aspects of cyclization include cyclization using an amide bond, a carbon-carbon bond, a thioether bond, a disulfide bond, an ester bond, a thioester bond, a lactam bond, a bond via a triazole structure, or a bond via a fluorophore structure. Among them, cyclization using an amide bond is preferred because of high metabolic stability. The translation step and cyclization step of the peptide may proceed separately or continuously. Cyclization can be performed by the methods known to those skilled in the art, such as those described in WO 2013/100132, WO 2008/117833, WO 2012/074129, and the like.

The aspects of the bonding in cyclization may be, but are not limited to, the bonding between the N-terminus and the C-terminus of the peptide, the bonding between the N-terminus and a side chain of another amino acid residue of the peptide, the bonding between the C-terminus and a side chain of another amino acid residue of the peptide, or the bonding between the side chains of amino acid residues, and two or more of these may be used in combination.

### [Peptide Complex]

The peptide according to the present embodiments may form a complex with another molecule such as a nucleic acid. This complex is referred to herein as a peptide complex. In one aspect, the peptide complex is obtained by binding one or more selected from the group consisting of a nucleic acid, a ribosome, a spacer, and a linker to the peptide according to the present embodiments. As the peptide complex, a peptide-nucleic acid complex in which a peptide and a nucleic acid are linked is preferred. In the peptide-nucleic acid complex, one or more selected from the group consisting of a spacer and a linker can be appropriately present between the peptide and the nucleic acid. Examples of the linker include puromycin and a polymer of hexaethylene glycol (spc18) (e.g., five polymers), and examples of the spacer include amino acids.

The nucleic acid herein can be a nucleic acid encoding a peptide that is a phenotype, i.e., an RNA or DNA that is a genotype of the peptide, more preferably an RNA, and most preferably an mRNA. Examples of the method for producing a peptide-nucleic acid complex include a method that utilizes non-specific linking of an antibiotic puromycin that is an analogue of aminoacyl tRNA to a peptide during mRNA translation extension by ribosome. For example, when puromycin is bound to the 3' end of the mRNA via an appropriate linker and the mRNA is translated in a cell-free translation system, the puromycin is incorporated into a peptide in place of an amino acid by ribosome, and the mRNA and the peptide encoded therein are linked to form a peptide-nucleic acid complex in which the mRNA and its product peptide correspond. In one aspect, a nucleic acid comprising a base sequence encoding a spacer downstream of a base sequence encoding a peptide (peptide of interest) according to the present embodiments can be used in the production of a peptide-nucleic acid complex. Examples of the spacer sequence include, but are not limited to, a sequence containing glycine or serine. It is also preferable to include a linker formed of RNA, DNA, a polymer of hexaethylene glycol (spc18) (e.g., five polymers), or the like between puromycin and the nucleic acid. In one aspect, a peptide-nucleic acid complex can be produced using a puromycin analogue instead of puromycin. Peptide-nucleic acid complexes are reported as mRNA display (Proc Natl Acad Sci USA. 1997;94:12297-302. RNA-peptide fusions for the in vitro selection of peptides and proteins. Roberts RW, Szostak JW.) or in vitro virus(FEBS Lett. 1997;414:405-8. In vitro virus: bonding of mRNA bearing puromycin at the 3'-terminal end to the C-terminal end of its encoded protein on the ribosome in vitro. Nemoto N, Miyamoto-Sato E, Husimi Y, Yanagawa H.).

In one aspect, the present invention relates to a method of producing a peptide or a library containing a peptide, comprising, for example, the steps of:
(1) producing an acyclic peptide containing one or more unnatural amino acids by the method described herein, wherein the acyclic peptide contains an amino acid residue having a reaction point at one of the side chains on the C-terminal side and an amino acid residue having another reaction point on the N-terminal side; and
(2) binding the reaction point of the amino acid residue on the N-terminal side and the reaction point of the amino acid residue having the reaction point at the side chain on the C-terminal side to form an amide bond, a carbon-carbon bond, or a thioether bond.

Note that these steps (1) and (2) may be performed independently or continuously. The library herein includes a display library that is a library of peptides tagged (i.e., encoded) with nucleic acids (e.g., DNA-encoded library/DEL, mRNA display library, DNA display library; see, e.g., Molecules. 2019 Apr; 24(8): 1629.) or a library of peptides not tagged, preferably a display library, more preferably an mRNA display library, a DNA display library, and a ribosomal display library, and most preferably an mRNA display library.

Specifically, one aspect of the method of cyclizing a peptide by an amide bond includes a method of cyclization by cross-linking an amino group of methionine at the N-terminus and an amino group of lysine disposed downstream (C-terminal side) with disuccinimidyl glutarate (DSG), a method of cyclization by introducing an amino acid derivative having a chloroacetyl group as an N-terminal translation initiation amino acid and forming a thioether by an intramolecular cyclization reaction by disposing Cys downstream, or a method of cyclization using native chemical ligation to translate a peptide having a cysteine or cysteine analogue at the N-terminus and an active ester at the side chain of the amino acid on the C-terminal side.

In one aspect, the C-terminal moiety of the peptide in the present disclosure may be chemically modified, not being carboxylic acid as is. For example, the carboxylic acid moiety may be reacted with piperidine or the like to be converted to piperidinamide or the like.

### [Examples]

The present invention will be further illustrated with reference to Examples given below, but is not limited by Examples below. In Examples, the following abbreviations were used.
AA: ammonium acetate
CH₂CN: cyanomethyl group
DBU: 1,8-diazabicyclo[5.4.0]-7-undecene
DCM: dichloromethane
DIPEA: N,N-diisopropylethylamine
DMF: N,N-dimethylformamide
DMSO: dimethyl sulfoxide
FA: formic acid
Fmoc: 9-fluorenylmethyloxycarbonyl group
F-Pnaz: 4-(2-(4-fluorophenyl)acetamido)benzyloxycarbonyl group 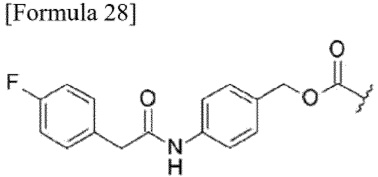
   MeCN: acetonitrile
   TEA: triethylamine
   TFA: trifluoroacetic acid
   THF: tetrahydrofuran

The following abbreviations were used in Examples. Gly or G (glycine), Ile or I (isoleucine), Leu or L (leucine), Phe or F (phenylalanine), Pro or P (proline), Thr or T (threonine).

The analytical conditions of LCMS are shown in Table 1.

**[Table 1]**

| | |
|---|---|
| Fractionation condition | SQDFA05 |
| Device | Acquity UPLC/SQD2 |
| Column (I.D.× Length (mm)) | Aldrich Ascentis Express C18 2.7 µm (2.1×50) |
| Mobile phase | A) 0.1% aqueous formic acid solution |
| | B) 0.1% formic acid acetonitrile solution |
| Gradient (A/B) | 95/5 → 0/100 (1.0 min) → 0/100 (0.4 min) |
| Flow rate (mL/min) | 0.9 |
| Column temperature (°C) | 35 |
| Detection wavelength | 210-440 nm (PDA total) |

### Example 1. Synthesis of aminoacyl pCpA used for cell-free translation system

Aminoacylated pCpA (Compounds ST01, ST04, ST07, ST10, ST13, ST16, ST19, ST22, ST25, ST28, ST31, ST34, ST37, ST40, ST41, ST42) was synthesized according to the scheme below.

### Synthesis of [(2R,3S,4R,5R)-2-[[[(2R,3S,4R,5R)-5-(4-amino-2-oxopyrimidin-1-yl)-4-hydroxy-2-(phosphonooxymethyl)oxolan-3-yl]oxy-hydroxyphosphoryl]oxymethyl]-5-(6-aminopurin-9-yl)-4-hydroxyoxolan-3-yl] 2-[butyl-[[[4-[[2-(4-fluorophenyl)acetyl]amino]phenyl]methoxycarbonyl]amino]acetate (Compound ST01)

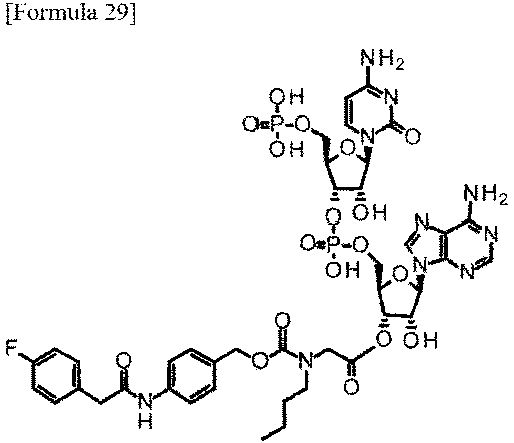

Compound ST01 was synthesized according to the method described in Patent Literature (WO 2018/143145).

### Synthesis of 2-[[4-[[2-(4-fluorophenyl)acetyl]amino]phenyl]methoxycarbonyl-(2-methoxyethyl)amino]acetic acid (Compound ST02)

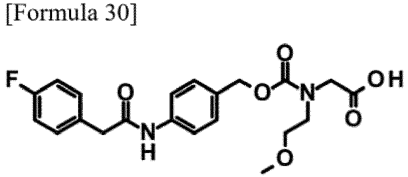

Under nitrogen atmosphere, 2-iodoacetic acid (37.2 mg, 0.2 mmol) was dissolved in water (0.5 mL) and stirred at 0°C for 5 minutes. To this, an acetonitrile solution (0.5 mL) of 2-methoxyethan-1-amine (30.0 mg, 0.4 mmol) and DIPEA (70.0 µL, 0.4 mmol) was added at 0°C. After stirring the mixture at room temperature for 60 hours, DMSO (2.0 mL) was added, and then the reaction solution was concentrated, and acetonitrile and water were distilled off. To the obtained DMSO solution, (4-nitrophenyl)-4-(2-(4-fluorophenyl)acetamide)benzyl carbonate (170.0 mg, 0.40 mmol) synthesized by the method described in Patent Literature (WO 2018/143145) and DIPEA (70.0 µL, 0.4 mmol) were sequentially added, and the mixture was stirred at 40°C for 4 hours, and then purified by reverse phase silica gel column chromatography (0.1% aqueous formic acid solution/0.1% formic acid acetonitrile solution) to obtain the title compound (Compound ST02) (30 mg, 36% yield).
LCMS (ESI) m/z = 417.2 (M-H)⁻
Retention time: 0.66 minutes (analytical condition SQDFA05)

### Synthesis of cyanomethyl 2-[[4-[[2-(4-fluorophenyl)acetyl]amino]phenyl]methoxycarbonyl-(2-methoxyethyl)amino]acetate (Compound ST03)

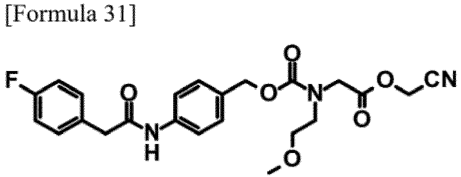

Under nitrogen atmosphere, 2-bromoacetonitrile (30.0 µL, 0.45 mmol) and DIPEA (11.0 µL, 0.07 mmol) were sequentially added to an acetonitrile solution (0.25 mL) of 2-[[4-[[2-(4-fluorophenyl)acetyl]amino]phenyl]methoxycarbonyl-(2-methoxyethyl)amino]acetic acid (Compound ST02) (21.0 mg, 0.05 mmol) at room temperature. After stirring the reaction mixture at room temperature for 1 hour, the reaction solution was concentrated to obtain the title compound (Compound ST03) as a crude product. The obtained crude product was dissolved in acetonitrile (3.0 mL) and used as is in the next step.
LCMS (ESI) m/z = 456.3 (M-H)⁻
Retention time: 0.77 minutes (analytical condition SQDFA05)

### Synthesis of [(2R,3S,4R,5R)-2-[[[(2R,3S,4R,5R)-5-(4-amino-2-oxopyrimidin-1-yl)-4-hydroxy-2-(phosphonooxymethyl)oxolan-3-yl]oxy-hydroxyphosphoryl]oxymethyl]-5-(6-aminopurin-9-yl)-4-hydroxyoxolan-3-yl] 2-[[4-[[2-(4-fluorophenyl)acetyl]amino]phenyl]methoxycarbonyl-(2-methoxyethyl)amino]acetate (Compound ST04)

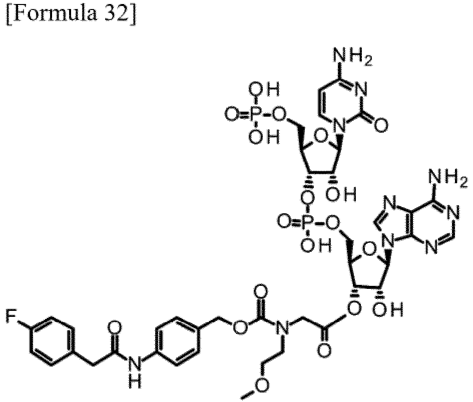

((2R,3R,4R,5R)-5-(4-Amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogen phosphate (36 mg, 0.05 mmol) synthesized by the method described in the Literature (Helv. Chim. Acta, 90, 297-310) was dissolved in a buffer solution A (60.0 mL), then an acetonitrile solution (3.0 mL, 0.05 mmol) of cyanomethyl 2-[[4-[[2-(4-fluorophenyl)acetyl]amino]phenyl]methoxycarbonyl-(2-methoxyethyl)amino]acetate (Compound ST03) was added, and the mixture was stirred at room temperature for 90 minutes. The reaction solution was cooled to 0°C, and then trifluoroacetic acid (3.0 mL) was added. After stirring the reaction solution at room temperature for 30 minutes, the reaction solution was purified by reverse phase silica gel column chromatography (0.05% aqueous trifluoroacetic acid solution/0.05% trifluoroacetic acid acetonitrile solution) to obtain the title compound (Compound ST04) (11.0 mg, 21%).
LCMS (ESI) m/z = 1051.5 (M-H)⁻
Retention time: 0.49 minutes (analytical condition SQDFA05)

The buffer solution A was prepared as follows.

Acetic acid was added to an aqueous solution of N,N,N-trimethylhexadecan-1-aminium chloride (6.40 g, 20 mmol) and imidazole (6.81 g, 100 mmol) to obtain a buffer solution A (1 L) of pH 8, 20 mM N,N,N-trimethylhexadecan-1-aminium, and 100 mM imidazole.

### Synthesis of 2-[[4-[[2-(4-fluorophenyl)acetyl]amino]phenyl]methoxycarbonyl-(3-methoxypropyl)amino]acetic acid (Compound ST05)

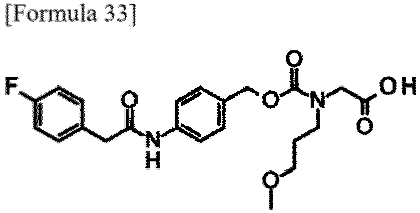

Under nitrogen atmosphere, 2-iodoacetic acid (37.2 mg, 0.2 mmol) was dissolved in water (0.5 mL) and stirred at 0°C for 5 minutes. To this, an acetonitrile solution (0.5 mL) of 3-methoxypropan-1-amine (35.7 mg, 0.4 mmol) and DIPEA (70.0 µL, 0.4 mmol) was added at 0°C. After stirring the mixture at room temperature for 60 hours, DMSO (2.0 mL) was added, and then the reaction solution was concentrated, and acetonitrile and water were distilled off. To the obtained DMSO solution, (4-nitrophenyl)-4-(2-(4-fluorophenyl)acetamide)benzyl carbonate (170.0 mg, 0.40 mmol) synthesized by the method described in Patent Literature (WO 2018/143145) and DIPEA (70.0 µL, 0.4 mmol) were sequentially added, and the mixture was stirred at 40°C for 4 hours, and then purified by reverse phase silica gel column chromatography (0.1% aqueous formic acid solution/0. 1% formic acid acetonitrile solution) to obtain the title compound (Compound ST05) (30 mg, 35% yield).
LCMS (ESI) m/z = 431.7 (M-H)⁻
Retention time: 0.67 minutes (analytical condition SQDFA05)

### Synthesis of cyanomethyl 2-[[4-[[2-(4-fluorophenyl)acetyl]amino]phenyl]methoxycarbonyl-(3-methoxypropyl)amino]acetate (Compound ST06)

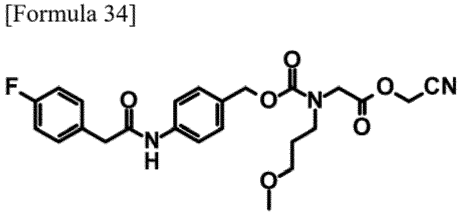

Under nitrogen atmosphere, 2-bromoacetonitrile (30.0 µL, 0.45 mmol) and DIPEA (11.0 µL, 0.07 mmol) were sequentially added to an acetonitrile solution (0.25 mL) of 2-[[4-[[2-(4-fluorophenyl)acetyl]amino]phenyl]methoxycarbonyl-(2-methoxyethyl)amino]acetic acid (Compound ST05) (22.0 mg, 0.05 mmol) at room temperature. After stirring the reaction mixture at room temperature for 1 hour, the reaction solution was concentrated to obtain the title compound (Compound ST06) as a crude product. The obtained crude product was dissolved in acetonitrile (3.0 mL) and used as is in the next step.
LCMS (ESI) m/z = 470.3 (M-H)⁻
Retention time: 0.76 minutes (analytical condition SQDFA05)

### Synthesis of [(2R,3S,4R,5R)-2-[[[(2R,3S,4R,5R)-5-(4-amino-2-oxopyrimidin-1-yl)-4-hydroxy-2-(phosphonooxymethyl)oxolan-3-yl]oxy-hydroxyphosphoryl]oxymethyl]-5-(6-aminopurin-9-yl)-4-hydroxyoxolan-3-yl] 2-[[4-[[2-(4-fluorophenyl)acetyl]amino]phenyl]methoxycarbonyl-(3-methoxypropyl)amino]acetate (Compound ST07)

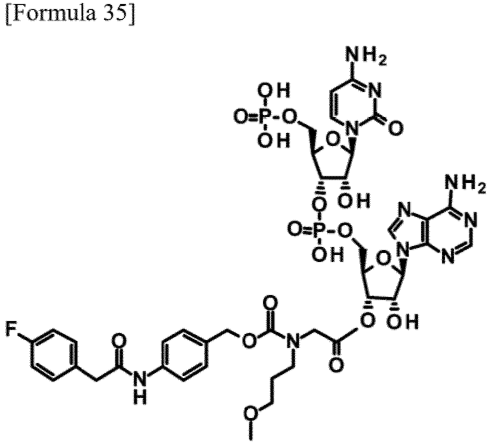

((2R,3R,4R,5R)-5-(4-Amino-2-oxopyrimidin-l(2H)-yl)-3-(((((2R.,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogen phosphate (36 mg, 0.05 mmol) synthesized by the method described in the Literature (Helv. Chim. Acta, 90, 297-310) was dissolved in a buffer solution A (60.0 mL), then an acetonitrile solution (3.0 mL, 0.05 mmol) of cyanomethyl 2-[[4-[[2-(4-fluorophenyl)acetyl]amino]phenyl]methoxycarbonyl-(2-methoxyethyl)amino]acetate (Compound ST06) was added, and the mixture was stirred at room temperature for 120 minutes. The reaction solution was cooled to 0°C, and then trifluoroacetic acid (3.0 mL) was added. After stirring the reaction solution at room temperature for 30 minutes, the reaction solution was purified by reverse phase silica gel column chromatography (0.05% aqueous trifluoroacetic acid solution/0.05% trifluoroacetic acid acetonitrile solution) to obtain the title compound (Compound ST07) (11.0 mg, 21% yield).
LCMS (ESI) m/z = 1065.7 (M-H)⁻
Retention time: 0.50 minutes (analytical condition SQDFA05)

### Synthesis of 2-[[4-[[2-(4-fluorophenyl)acetyl]amino]phenyl]methoxycarbonyl-(3-phenylpropyl)amino]acetic acid (Compound ST08)

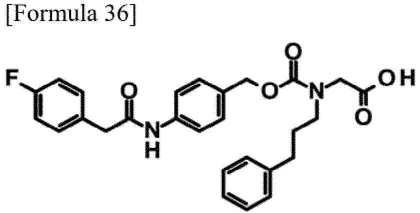

Under nitrogen atmosphere, 2-iodoacetic acid (37.2 mg, 0.2 mmol) was dissolved in water (0.5 mL) and stirred at 0°C for 5 minutes. To this, an acetonitrile solution (0.5 mL) of 3-phenylpropan-1-amine (54.1 mg, 0.4 mmol) and DIPEA (70.0 µL, 0.4 mmol) was added at 0°C. After stirring the mixture at room temperature for 16 hours, DMSO (2.0 mL) was added, and then the reaction solution was concentrated, and acetonitrile and water were distilled off. To the obtained DMSO solution, (4-nitrophenyl)-4-(2-(4-fluorophenyl)acetamide)benzyl carbonate (170.0 mg, 0.40 mmol) synthesized by the method described in Patent Literature (WO 2018/143145) and DIPEA (70.0 µL, 0.4 mmol) were sequentially added, and the mixture was stirred at 40°C for 4 hours, and then purified by reverse phase silica gel column chromatography (0.1% aqueous formic acid solution/0.1% formic acid acetonitrile solution) to obtain the title compound (Compound ST08) (40 mg, 42% yield).
LCMS (ESI) m/z = 477.2 (M-H)⁻
Retention time: 0.83 minutes (analytical condition SQDFA05)

### Synthesis of cyanomethyl 2-[[4-[[2-(4-fluorophenyl)acetyl]amino]phenyl]methoxycarbonyl-(3-phenylpropyl)amino]acetate (Compound ST9)

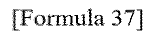

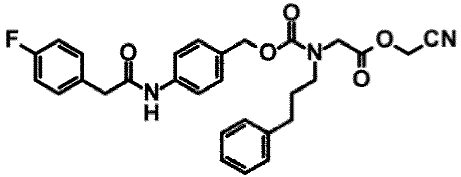

Under nitrogen atmosphere, 2-bromoacetonitrile (23.0 µL, 0.35 mmol) and DIPEA (11.0 µL, 0.06 mmol) were sequentially added to an acetonitrile solution (0.25 mL) of 2-[[4-[[2-(4-fluorophenyl)acetyl]amino]phenyl]methoxycarbonyl-(3-phenylpropyl)amino]acetic acid (Compound ST08) (24.0 mg, 0.05 mmol) at room temperature. After stirring the reaction mixture at room temperature for 5 hours, the reaction solution was concentrated to obtain the title compound (Compound ST9) as a crude product. The obtained crude product was dissolved in acetonitrile (1.5 mL) and used as is in the next step.
LCMS (ESI) m/z = 516.3 (M-H)⁻
Retention time: 0.90 minutes (analytical condition SQDFA05)

### Synthesis of [(2R,3S,4R,5R)-2-[[[(2R,3S,4R,5R)-5-(4-amino-2-oxopyrimidin-1-yl)-4-hydroxy-2-(phosphonooxymethyl)oxolan-3-yl]oxy-hydroxyphosphoryl]oxymethyl]-5-(6-aminopurin-9-yl)-4-hydroxyoxolan-3-yl] 2-[[4-[[2-(4-fluorophenyl)acetyl]amino]phenyl]methoxycarbonyl-(3-phenylpropyl)amino]acetate (Compound ST10)

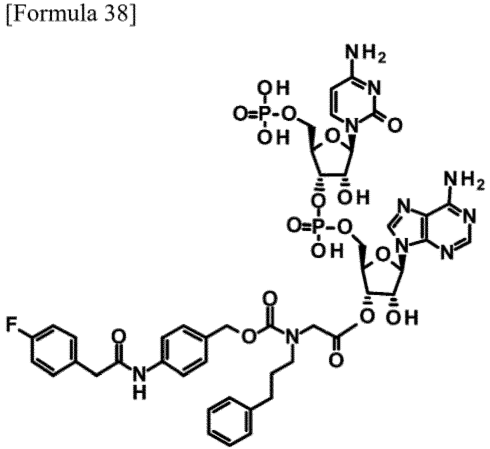

((2R,3R,4R,5R)-5-(4-Amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogen phosphate (36 mg, 0.05 mmol) synthesized by the method described in the Literature (Helv. Chim. Acta, 90, 297-310) was dissolved in a buffer solution A (30.0 mL), then an acetonitrile solution (1.5 mL, 0.05 mmol) of cyanomethyl 2-[[4-[[2-(4-fluorophenyl)acetyl]amino]phenyl]methoxycarbonyl-(2-methoxyethyl)amino]acetate (Compound ST9) was added, and the mixture was stirred at room temperature for 90 minutes. The reaction solution was cooled to 0°C, and then trifluoroacetic acid (1.5 mL) was added. After stirring the reaction solution at room temperature for 30 minutes, the reaction solution was purified by reverse phase silica gel column chromatography (0.05% aqueous trifluoroacetic acid solution/0.05% trifluoroacetic acid acetonitrile solution) to obtain the title compound (Compound ST10) (11.0 mg, 20% yield).
LCMS (ESI) m/z = 1111.5 (M-H)⁻
Retention time: 0.59 minutes (analytical condition SQDFA05)

### Synthesis of 2-[[4-[[2-(4-fluorophenyl)acetyl]amino]phenyl]methoxycarbonyl-(2-phenoxyethyl)amino]acetic acid (Compound ST11)

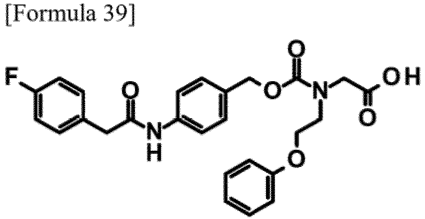

Under nitrogen atmosphere, 2-iodoacetic acid (37.2 mg, 0.2 mmol) was dissolved in water (0.5 mL) and stirred at 0°C for 5 minutes. To this, an acetonitrile solution (0.5 mL) of 2-phenoxyethan-1-amine (54.9 mg, 0.4 mmol) and DIPEA (70.0 µL, 0.4 mmol) was added at 0°C. After stirring the mixture at room temperature for 16 hours, DMSO (2.0 mL) was added, and then the reaction solution was concentrated, and acetonitrile and water were distilled off. To the obtained DMSO solution, (4-nitrophenyl)-4-(2-(4-fluorophenyl)acetamide)benzyl carbonate (170.0 mg, 0.40 mmol) synthesized by the method described in Patent Literature (WO 2018/143145) and DIPEA (70.0 µL, 0.4 mmol) were sequentially added, and the mixture was stirred at 40°C for 4 hours, and then purified by reverse phase silica gel column chromatography (0.1% aqueous formic acid solution/0.1% formic acid acetonitrile solution) to obtain the title compound (Compound ST11) (41 mg, 43% yield).
LCMS (ESI) m/z = 479.3 (M-H)⁻
Retention time: 0.79 minutes (analytical condition SQDFA05)

### Synthesis of cyanomethyl 2-[[4-[[2-(4-fluorophenyl)acetyl]amino]phenyl]methoxycarbonyl-(2-phenoxyethyl)amino]acetate (Compound ST12)

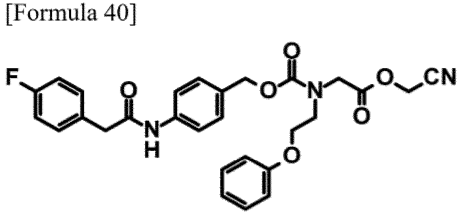

Under nitrogen atmosphere, 2-bromoacetonitrile (23.0 µL, 0.35 mmol) and DIPEA (11.0 µL, 0.06 mmol) were sequentially added to an acetonitrile solution (0.25 mL) of 2-[[4-[[2-(4-fluorophenyl)acetyl]amino]phenyl]methoxycarbonyl-(2-phenoxyethyl)amino]acetic acid (Compound ST11) (24.0 mg, 0.05 mmol) at room temperature. After stirring the reaction mixture at room temperature for 5 hours, the reaction solution was concentrated to obtain the title compound (Compound ST12) as a crude product. The obtained crude product was dissolved in acetonitrile (1.5 mL) and used as is in the next step.
LCMS (ESI) m/z = 518.2 (M-H)⁻
Retention time: 0.87 minutes (analytical condition SQDFA05)

### Synthesis of [(2R,3S,4R,5R)-2-[[[(2R,3S,4R,5R)-5-(4-amino-2-oxopyrimidin-1-yl)-4-hydroxy-2-(phosphonooxymethyl)oxolan-3-yl]oxy-hydroxyphosphoryl]oxymethyl]-5-(6-aminopurin-9-yl)-4-hydroxyoxolan-3-yl] 2-[[4-[[2-(4-fluorophenyl)acetyl]amino]phenyl]methoxycarbonyl-(2-phenoxyethyl)amino]acetate (Compound ST13)

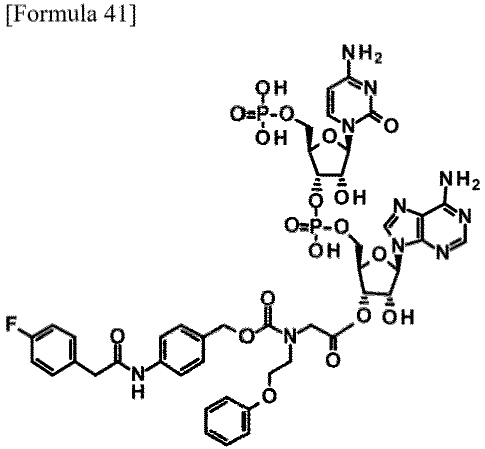

((2R,3R,4R,5R)-5-(4-Amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogen phosphate (36 mg, 0.05 mmol) synthesized by the method described in the Literature (Helv. Chim. Acta, 90, 297-310) was dissolved in a buffer solution A (30.0 mL), then an acetonitrile solution (1.5 mL, 0.05 mmol) of cyanomethyl 2-[[4-[[2-(4-fluorophenyl)acetyl]amino]phenyl]methoxycarbonyl-(2-phenoxyethyl)amino]acetate (Compound ST12) was added, and the mixture was stirred at room temperature for 90 minutes. The reaction solution was cooled to 0°C, and then trifluoroacetic acid (1.5 mL) was added. After stirring the reaction solution at room temperature for 30 minutes, the reaction solution was purified by reverse phase silica gel column chromatography (0.05% aqueous trifluoroacetic acid solution/0.05% trifluoroacetic acid acetonitrile solution) to obtain the title compound (Compound ST13) (11.0 mg, 20% yield).
LCMS (ESI) m/z = 1113.4 (M-H)⁻
Retention time: 0.57 minutes (analytical condition SQDFA05)

### Synthesis of (2R)-2-[[4-[[2-(4-fluorophenyl]acetyl]amino]phenyl]methoxycarbonylamino]pentanoic acid (Compound ST14)

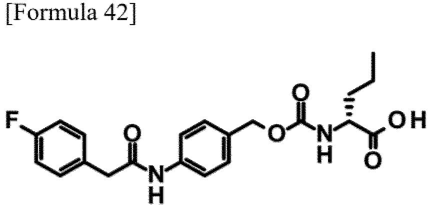

Under nitrogen atmosphere, (R)-2-aminopentanecarboxylic acid (11.7 mg, 0.1 mmol) and (4-nitrophenyl)-4-(2-(4-fluorophenyl)acetamide)benzyl carbonate (42.4 mg, 0.1 mmol) synthesized by the method described in Patent Literature (WO 2018/143145) were added to DMSO (0.5 mL), and the mixture was stirred at room temperature for 5 minutes. Triethylamine (20.9 µL, 0.15 mmol) was added at 0°C, and then the reaction mixture was stirred at room temperature for 16 hours and then purified by reverse phase silica gel column chromatography (0.1% aqueous formic acid solution/0.1% formic acid acetonitrile solution) to obtain the title compound (Compound ST14) (33 mg, 82% yield).
LCMS (ESI) m/z = 401.3 (M-H)⁻
Retention time: 0.71 minutes (analytical condition SQDFA05)

### Synthesis of cyanomethyl (2R)-2-[[4-[[2-(4-fluorophenyl)acetyl]amino]phenyl]methoxycarbonylamino]pentanoate (Compound ST15)

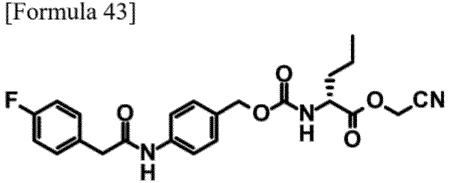

Under nitrogen atmosphere, 2-bromoacetonitrile (10.1 µL, 0.15 mmol) and DIPEA (10.5 µL, 0.06 mmol) were sequentially added to an acetonitrile solution (0.25 mL) of (2R)-2-[[4-[[2-(4-fluorophenyl)acetyl]amino]phenyl]methoxycarbonylamino]pentanoic acid (Compound ST14) (20.0 mg, 0.05 mmol) at room temperature. After stirring the reaction mixture at room temperature for 16 hours, the reaction solution was concentrated to obtain the title compound (Compound ST15) as a crude product. The obtained crude product was dissolved in acetonitrile (1.5 mL) and used as is in the next step.
LCMS (ESI) m/z = 440.3 (M-H)⁻
Retention time: 0.80 minutes (analytical condition SQDFA05)

### Synthesis of [(2R,3S,4R,5R)-2-[[[(2R,3S,4R,5R)-5-(4-amino-2-oxopyrimidin-1-yl)-4-hydroxy-2-(phosphonooxymethyl)oxolan-3-yl]oxy-hydroxyphosphoryl]oxymethyl]-5-(6-aminopurin-9-yl)-4-hydroxyoxolan-3-yl] (2R)-2-[[4-[[2-(4-fluorophenyl)acetyl]amino]phenyl]methoxycarbonylamino]pentanoate (Compound ST16)

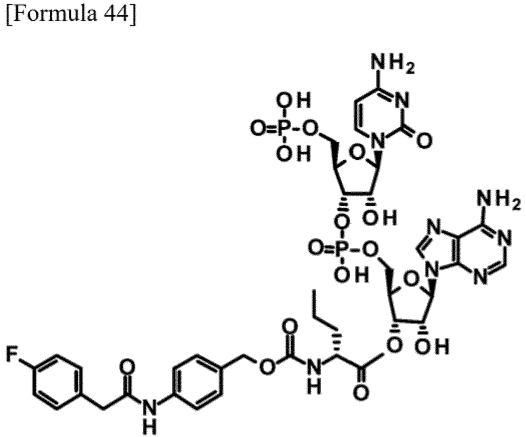

((2R,3R,4R,5R)-5-(4-Amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogen phosphate (36 mg, 0.05 mmol) synthesized by the method described in the Literature (Helv. Chim. Acta, 90, 297-310) was dissolved in a buffer solution A (30.0 mL), then an acetonitrile solution (1.5 mL, 0.05 mmol) of cyanomethyl (2R)-2-[[4-[[2-(4-fluorophenyl)acetyl]amino]phenyl]methoxycarbonyl]amino]pentanoate (Compound ST15) was added, and the mixture was stirred at room temperature for 90 minutes. The reaction solution was cooled to 0°C, and then trifluoroacetic acid (1.5 mL) was added. After stirring the reaction solution at room temperature for 30 minutes, the reaction solution was purified by reverse phase silica gel column chromatography (0.05% aqueous trifluoroacetic acid solution/0.05% trifluoroacetic acid acetonitrile solution) to obtain the title compound (Compound ST16) (9.0 mg, 17% yield).
LCMS (ESI) m/z = 1035.4 (M-H)⁻
Retention time: 0.52 minutes (analytical condition SQDFA05)

### Synthesis of (2R)-2-[[4-[[2-(4-fluorophenyl)acetyl]amino]phenyl]methoxycarbonylamino]-3-methoxypropanoic acid (Compound ST17)

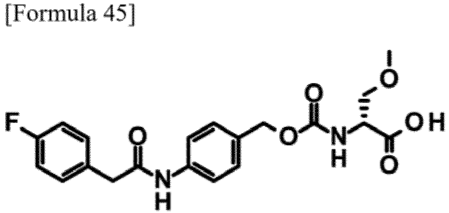

Under nitrogen atmosphere, O-methyl-D-serine (11.9 mg, 0.1 mmol) and (4-nitrophenyl)-4-(2-(4-fluorophenyl)acetamide) benzyl carbonate (42.4 mg, 0.1 mmol) synthesized by the method described in Patent Literature (WO 2018/143145) were added to DMSO (0.5 mL), and the mixture was stirred at room temperature for 5 minutes. Triethylamine (20.9 µL, 0.15 mmol) was added at 0°C, and then the reaction mixture was stirred at room temperature for 16 hours and then purified by reverse phase silica gel column chromatography (0.1% aqueous formic acid solution/0.1% formic acid acetonitrile solution) to obtain the title compound (Compound ST17) (36 mg, 89% yield).
LCMS (ESI) m/z = 403.3 (M-H)⁻
Retention time: 0.63 minutes (analytical condition SQDFA05)

### Synthesis of cyanomethyl (2R)-2-[[4-[[2-(4-fluorophenyl)acetyl]amino]phenyl]methoxycarbonylamino]-3-methoxypropanoate (Compound ST18)

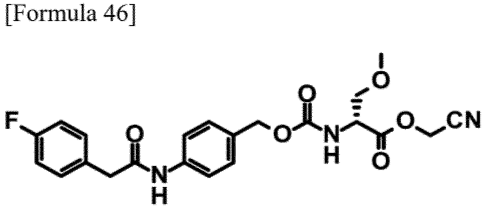

Under nitrogen atmosphere, 2-bromoacetonitrile (10.1 µL, 0.15 mmol) and DIPEA (10.5 µL, 0.06 mmol) were sequentially added to an acetonitrile solution (0.25 mL) of (2R)-2-[[4-[[2-(4-fluorophenyl)acetyl]amino]phenyl]methoxycarbonylamino]-3-methoxypropanoic acid (Compound ST17) (20.0 mg, 0.05 mmol) at room temperature. After stirring the reaction mixture at room temperature for 16 hours, the reaction solution was concentrated to obtain the title compound (Compound ST18) as a crude product. The obtained crude product was dissolved in acetonitrile (1.5 mL) and used as is in the next step.
LCMS (ESI) m/z = 442.2 (M-H)⁻
Retention time: 0.72 minutes (analytical condition SQDFA05)

### Synthesis of [(2R,3S,4R,5R)-2-[[[(2R,3S,4R,5R)-5-(4-amino-2-oxopyrimidin-1-yl)-4-hydroxy-2-(phosphonooxymethyl)oxolan-3-yl]oxy-hydroxyphosphoryl]oxymethyl]-5-(6-aminopurin-9-yl)-4-hydroxyoxolan-3-yl] (2R)-2-[[4-[[2-(4-fluorophenyl)acetyl]amino]phenyl]methoxycarbonylamino]-3-methoxypropanoate (Compound ST19)

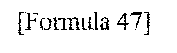

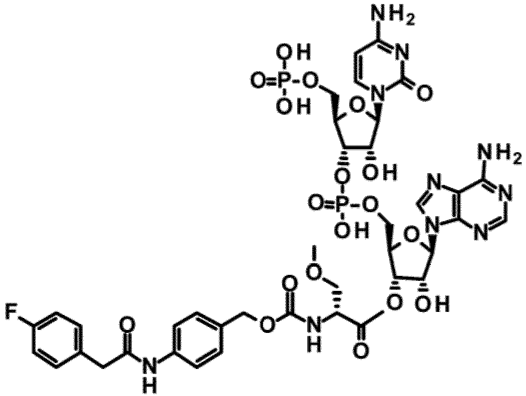

((2R,3R,4R,5R)-5-(4-Amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogen phosphate (36 mg, 0.05 mmol) synthesized by the method described in the Literature (Helv. Chim. Acta, 90, 297-310) was dissolved in a buffer solution A (30.0 mL), then an acetonitrile solution (1.5 mL, 0.05 mmol) of cyanomethyl (2R)-2-[[4-[[2-(4-fluorophenyl)acetyl]amino]phenyl]methoxycarbonylamino]-3-methoxypropanoate (Compound ST18) was added, and the mixture was stirred at room temperature for 90 minutes. The reaction solution was cooled to 0°C, and then trifluoroacetic acid (1.5 mL) was added. After stirring the reaction solution at room temperature for 30 minutes, the reaction solution was purified by reverse phase silica gel column chromatography (0.05% aqueous trifluoroacetic acid solution/0.05% trifluoroacetic acid acetonitrile solution) to obtain the title compound (Compound ST19) (9.0 mg, 17% yield).
LCMS (ESI) m/z = 1037.5 (M-H)⁻
Retention time: 0.48 minutes (analytical condition SQDFA05)

### Synthesis of (2R)-2-[[4-[[2-(4-fluorophenyl)acetyl]amino]phenyl]methoxycarbonylamino]-4-phenylbutanoic acid (Compound ST20)

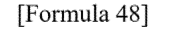

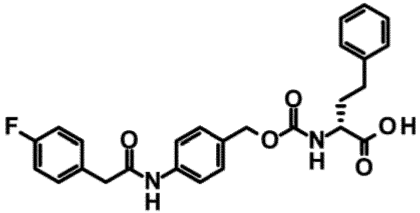

Under nitrogen atmosphere, (R)-2-amino-4-phenylbutanecarboxylic acid (17.9 mg, 0.1 mmol) and (4-nitrophenyl)-4-(2-(4-fluorophenyl)acetamide) benzyl carbonate (42.4 mg, 0.1 mmol) synthesized by the method described in Patent Literature (WO 2018/143145) were added to DMSO (0.5 mL), and the mixture was stirred at room temperature for 5 minutes. Triethylamine (20.9 µL, 0.15 mmol) was added at 0°C, and then the reaction mixture was stirred at room temperature for 16 hours and then purified by reverse phase silica gel column chromatography (0.1% aqueous formic acid solution/0.1% formic acid acetonitrile solution) to obtain the title compound (Compound ST20) (36 mg, 78% yield).
LCMS (ESI) m/z = 463.2 (M-H)⁻
Retention time: 0.78 minutes (analytical condition SQDFA05)

### Synthesis of cyanomethyl (2R)-2-[[4-[[2-(4-fluorophenyl)acetyl]amino]phenyl]methoxycarbonylamino]-4-phenylbutanoate (Compound ST21)

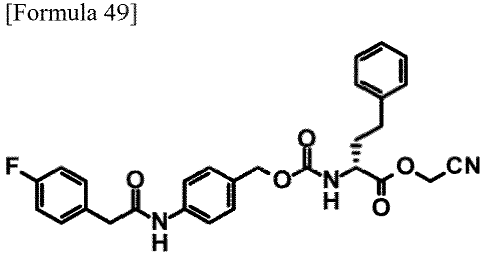

Under nitrogen atmosphere, 2-bromoacetonitrile (10.1 µL, 0.15 mmol) and DIPEA (10.5 µL, 0.06 mmol) were sequentially added to an acetonitrile solution (0.25 mL) of ((2R)-2-[[4-[[2-(4-fluorophenyl)acetyl]amino]phenyl]methoxycarbonylamino]-4-phenylbutanoic acid (Compound ST20) (23.0 mg, 0.05 mmol) at room temperature. After stirring the reaction mixture at room temperature for 16 hours, the reaction solution was concentrated to obtain the title compound (Compound ST21) as a crude product. The obtained crude product was dissolved in acetonitrile (1.5 mL) and used as is in the next step.
LCMS (ESI) m/z = 502.3 (M-H)⁻
Retention time: 0.86 minutes (analytical condition SQDFA05)

### Synthesis of [(2R,3S,4R,5R)-2-[[[(2R,3S,4R,5R)-5-(4-amino-2-oxopyrimidin-1-yl)-4-hydroxy-2-(phosphonooxymethyl)oxolan-3-yl]oxy-hydroxyphosphoryl]oxymethyl]-5-(6-aminopurin-9-yl)-4-hydroxyoxolan-3-yl] (2R)-2-[[4-[[2-(4-fluorophenyl)acetyl]amino]phenyl]methoxycarbonylamino]-4-phenylbutanoate (Compound ST22)

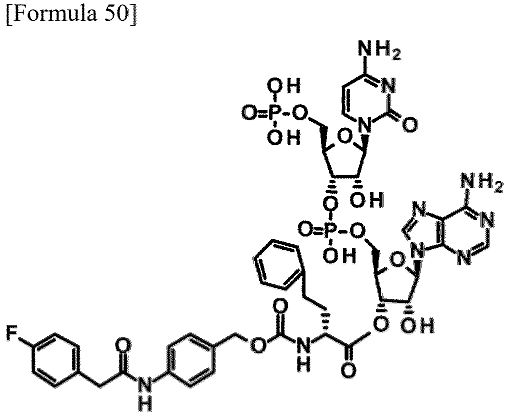

((2R,3R,4R,5R)-5-(4-Amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogen phosphate (36 mg, 0.05 mmol) synthesized by the method described in the Literature (Helv. Chim. Acta, 90, 297-310) was dissolved in a buffer solution A (30.0 mL), then an acetonitrile solution (1.5 mL, 0.05 mmol) of cyanomethyl (2R)-2-[[4-[[2-(4-fluorophenyl)acetyl]amino]phenyl]methoxycarbonyl]amino]-4-phenylbutanoate (Compound ST21) was added, and the mixture was stirred at room temperature for 90 minutes. The reaction solution was cooled to 0°C, and then trifluoroacetic acid (1.5 mL) was added. After stirring the reaction solution at room temperature for 30 minutes, the reaction solution was purified by reverse phase silica gel column chromatography (0.05% aqueous trifluoroacetic acid solution/0.05% trifluoroacetic acid acetonitrile solution) to obtain the title compound (Compound ST22) (11.0 mg, 20% yield).
LCMS (ESI) m/z = 1097.6 (M-H)⁻
Retention time: 0.57 minutes (analytical condition SQDFA05)

### Synthesis of (2R)-2-[[4-[[2-(4-fluorophenyl)acetyl]amino]phenyl]methoxycarbonylamino]-3-phenoxypropanoic acid (Compound ST23)

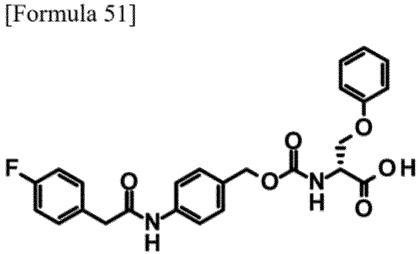

Under nitrogen atmosphere, O-phenyl-D-serine (18.1 mg, 0.1 mmol) and (4-nitrophenyl)-4-(2-(4-fluorophenyl)acetamide)benzyl carbonate (42.4 mg, 0.1 mmol) synthesized by the method described in Patent Literature (WO 2018/143145) were added to DMSO (0.5 mL), and the mixture was stirred at room temperature for 5 minutes. Triethylamine (20.9 µL, 0.15 mmol) was added at 0°C, and then the reaction mixture was stirred at room temperature for 16 hours and then purified by reverse phase silica gel column chromatography (0.1% aqueous formic acid solution/0.1% formic acid acetonitrile solution) to obtain the title compound (Compound ST23) (34 mg, 73% yield).
LCMS (ESI) m/z = 465.3 (M-H)⁻
Retention time: 0.76 minutes (analytical condition SQDFA05)

### Synthesis of cyanomethyl (2R)-2-[[4-[[2-(4-fluorophenyl)acetyl]amino]phenyl]methoxycarbonylamino]-3-phenoxypropanoate (Compound ST24)

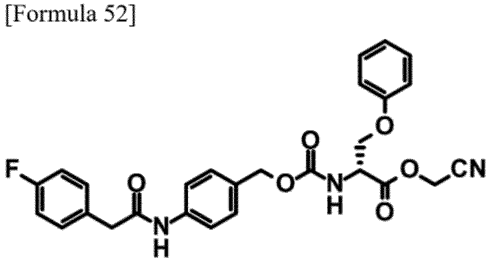

Under nitrogen atmosphere, 2-bromoacetonitrile (6.7 µL, 0.10 mmol) and DIPEA (10.5 µL, 0.06 mmol) were sequentially added to an acetonitrile solution (0.25 mL) of (2R)-2-[[4-[[2-(4-fluorophenyl)acetyl]amino]phenyl]methoxycarbonylamino]-3-phenoxypropanoic acid (Compound ST23) (23.0 mg, 0.05 mmol) at room temperature. After stirring the reaction mixture at room temperature for 16 hours, the reaction solution was concentrated to obtain the title compound (Compound ST24) as a crude product. The obtained crude product was dissolved in acetonitrile (1.5 mL) and used as is in the next step.
LCMS (ESI) m/z = 504.2 (M-H)⁻
Retention time: 0.84 minutes (analytical condition SQDFA05)

### Synthesis of [(2R,3S,4R,5R)-2-[[[(2R,3S,4R,5R)-5-(4-amino-2-oxopyrimidin-1-yl)-4-hydroxy-2-(phosphonooxymethyl)oxolan-3-yl]oxy-hydroxyphosphoryl]oxymethyl]-5-(6-aminopurin-9-yl)-4-hydroxyoxolan-3-yl](2R)-2-[[4-[[2-(4-fluorophenyl)acetyl]amino]phenyl]methoxycarbonylamino]-3-phenoxypropanoate (Compound ST25)

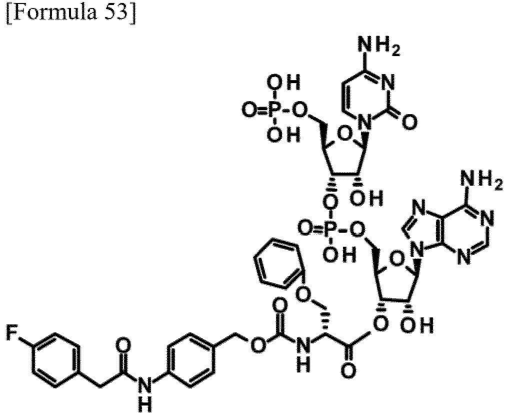

((2R,3R,4R,5R)-5-(4-Amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogen phosphate (36 mg, 0.05 mmol) synthesized by the method described in the Literature (Helv. Chim. Acta, 90, 297-310) was dissolved in a buffer solution A (30.0 mL), then an acetonitrile solution (1.5 mL, 0.05 mmol) of cyanomethyl (2R)-2-[[4-[[2-(4-fluorophenyl)acetyl]amino]phenyl]methoxycarboxamino]-3-phenoxypropanoate (Compound ST24) was added, and the mixture was stirred at room temperature for 60 minutes. The reaction solution was cooled to 0°C, and then trifluoroacetic acid (1.5 mL) was added. After stirring the reaction solution at room temperature for 30 minutes, the reaction solution was purified by reverse phase silica gel column chromatography (0.05% aqueous trifluoroacetic acid solution/0.05% trifluoroacetic acid acetonitrile solution) to obtain the title compound (Compound ST25) (7.0 mg, 13% yield).
LCMS (ESI) m/z = 1099.5 (M-H)⁻
Retention time: 0.56 minutes (analytical condition SQDFA05)

### Synthesis of (2R)-2-[[4-[[2-(4-fluorophenyl)acetyl]amino]phenyl]methoxycarbonyl-methylamino]butanoic acid (Compound ST26)

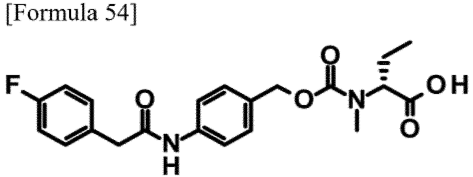

Under nitrogen atmosphere, 1,8-diazabicyclo[5.4.0]-7-undecene (DBU) (45.2 µL, 0.3 mmol) was added to a DMSO solution (1.0 ml) of (2R)-2-[9H-fluoren-9-ylmethoxycarbonyl(methyl)amino]butanoic acid (67.9 mg, 0.2 mmol), and the mixture was stirred for 5 minutes. To this, (4-nitrophenyl)-4-(2-(4-fluorophenyl)acetamide)benzyl carbonate (85.0 mg, 0.2 mmol) synthesized by the method described in Patent Literature (WO 2018/143145) and DIPEA (70.0 µL, 0.4 mmol) were sequentially added, and the mixture was stirred at room temperature for 3 hours, and then purified by reverse phase silica gel column chromatography (0.1% aqueous formic acid solution/0.1% formic acid acetonitrile solution) to obtain the title compound (Compound ST26) (60 mg, 75% yield).
LCMS (ESI) m/z = 401.3 (M-H)⁻
Retention time: 0.71 minutes (analytical condition SQDFA05)

### Synthesis of cyanomethyl (2R)-2-[[4-[[2-(4-fluorophenyl)acety]amino]phenyl]methoxycarbonyl-methylamino]butanoate (Compound ST27)

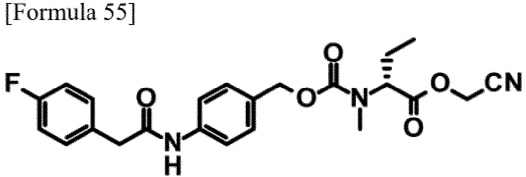

Under nitrogen atmosphere, 2-bromoacetonitrile (13.0 µL, 0.20 mmol) and DIPEA (26.0 µL, 0.15 mmol) were sequentially added to an acetonitrile solution (0.50 mL) of (2R)-2-[[4-[[2-(4-fluorophenyl)acetyl]amino]phenyl]methoxycarbonyl-methylamino]butanoic acid (Compound ST26) (40.0 mg, 0.10 mmol) at room temperature. After stirring the reaction mixture at room temperature for 15 hours, the reaction solution was concentrated to obtain the title compound (Compound ST27) as a crude product. The obtained crude product was dissolved in acetonitrile (1.25 mL) and used as is in the next step.
LCMS (ESI) m/z = 440.3 (M-H)⁻
Retention time: 0.80 minutes (analytical condition SQDFA05)

### Synthesis of [(2R,3S,4R,5R)-2-[[[(2R,3S,4R,5R)-5-(4-amino-2-oxopyrimidin-1-yl)-4-hydroxy-2-(phosphonooxymethyl)oxolan-3-yl]oxy-hydroxyphosphoryl]oxymethyl]-5-(6-aminopurin-9-yl)-4-hydroxyoxolan-3-yl] (2R)-2-[[4-[[2-(4-fluorophenyl)acetyl]amino]phenyl]methoxycarbonyl-methylamino]butanoate (Compound ST28)

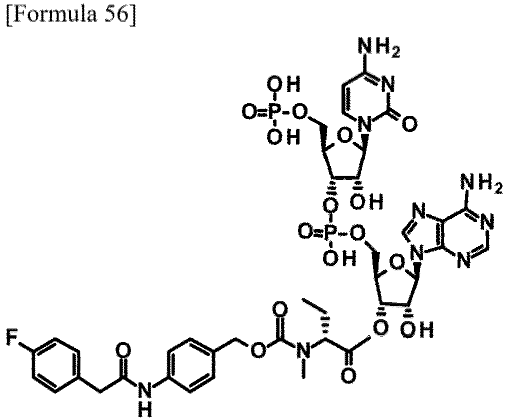

((2R,3R,4R,5R)-5-(4-Amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogen phosphate (36 mg, 0.05 mmol) synthesized by the method described in the Literature (Helv. Chim. Acta, 90, 297-310) was dissolved in a buffer solution A (25.0 mL), then an acetonitrile solution (0.63 mL, 0.05 mmol) of cyanomethyl (2R)-2-[[4-[[2-(4-fluorophenyl)acetyl]amino]phenyl]methoxycarbonyl-methylamino]butanoate (Compound ST27) was added, and the mixture was stirred at 30°C for 1 hour. An acetonitrile solution (0.63 mL, 0.05 mmol) of cyanomethyl (2R)-2-[[4-[[2-(4-fluorophenyl)acetyl]amino]phenyl]methoxycarbonyl-methylamino]butanoate (Compound ST27) was then added again, and the mixture was stirred at 30°C for an additional 2 hours. The reaction solution was cooled to 0°C, and then trifluoroacetic acid (1.25 mL) was added. After stirring the reaction solution at 0°C for 1 hour, the reaction solution was purified by reverse phase silica gel column chromatography (0.05% aqueous trifluoroacetic acid solution/0.05% trifluoroacetic acid acetonitrile solution) to obtain the title compound (Compound ST28) (12.8 mg, 25% yield).
LCMS (ESI) *m*/*z* = 1035.7 (M-H)⁻
Retention time: 0.52 minutes (analytical condition SQDFA05)

### Synthesis of (2R)-2-[[4-[[2-(4-fluorophenyl)acetyl]amino]phenyl]methoxycarbonyl-methylamino]-3-hydroxypropanoic acid (Compound ST29)

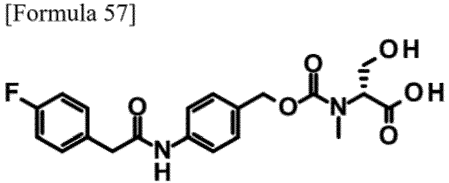

Under nitrogen atmosphere, methyl-D-serine (100.0 mg, 0.64 mmol) and (4-nitrophenyl)-4-(2-(4-fluorophenyl)acetamide) benzyl carbonate (273.0 mg, 0.64 mmol) synthesized by the method described in Patent Literature (WO 2018/143145) were added to DMSO (2.14 mL), and the mixture was stirred at room temperature for 5 minutes. Triethylamine (296.0 µL, 2.12 mmol) was added at 0°C, and then the reaction mixture was stirred at room temperature for 5 hours and then purified by reverse phase silica gel column chromatography (0.1% aqueous formic acid solution/0.1% formic acid acetonitrile solution) to obtain the title compound (Compound ST29) (215.6 mg, 83% yield).
LCMS (ESI) m/z = 403.0 (M-H)⁻
Retention time: 0.58 minutes (analytical condition SQDFA05)

### Synthesis of cyanomethyl (2R)-2-[[4-[[2-(4-fluorophenyl)acetyl]amino]phenyl]methoxycarbonyl-methylamino]-3-hydroxypropanoate (Compound ST30)

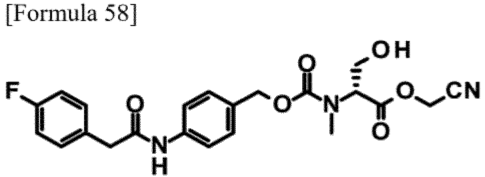

Under nitrogen atmosphere, 2-bromoacetonitrile (361.0 µL, 5.33 mmol) and DIPEA (186.0 µL, 1.07 mmol) were sequentially added to an acetonitrile solution (2.67 mL) of (2R)-2-[[4-[[2-(4-fluorophenyl)acetyl]amino]phenyl]methoxycarbonyl-methylamino]-3-hydroxypropanoic acid (Compound ST29) (215.6 mg, 0.53 mmol) at 0°C. The reaction mixture was stirred at room temperature for 2 hours, and then purified by reverse phase silica gel column chromatography (0.1% aqueous formic acid solution/0.1% formic acid acetonitrile solution) to obtain the title compound (Compound ST30) (220.3 mg, 93% yield).
LCMS (ESI) m/z = 442.0 (M-H)⁻
Retention time: 0.66 minutes (analytical condition SQDFA05)

### Synthesis of [(2R,3S,4R,5R)-2-[[[(2R,3S,4R,5R)-5-(4-amino-2-oxopyrimidin-1-yl)-4-hydroxy-2-(phosphonooxymethyl)oxolan-3-yl]oxy-hydroxyphosphoryl]oxymethyl]-5-(6-aminopurin-9-yl)-4-hydroxyoxolan-3-yl] (2R)-2-[[4-[[2-(4-fluorophenyl)acetyl]amino]phenyl]methoxycarbonyl-methylamino]-3-hydroxypropanoate (Compound ST31)

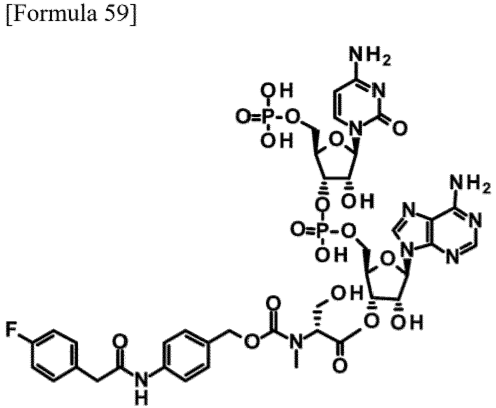

((2R,3R,4R,5R)-5-(4-Amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryloxy)-4-((tetrahydrofuran-2-yl)oxy)tetrahydrofuran-2-yl)methyl dihydrogen phosphate (60 mg, 0.08 mmol) synthesized by the method described in the Literature (Helv. Chim. Acta, 90, 297-310) was dissolved in a buffer solution A (45.0 mL), then an acetonitrile solution (0.5 mL, 0.08 mmol) of cyanomethyl (2R)-2-[[4-[[2-(4-fluorophenyl)acetyl]amino]phenyl]methoxycarbonyl-methylamino]-3-hydroxypropanoate (Compound ST30) was added, and the mixture was stirred at room temperature for 30 minutes. An acetonitrile solution (0.5 mL, 0.08 mmol) of cyanomethyl (2R)-2-[[4-[[2-(4-fluorophenyl)acetyl]amino]phenyl]methoxycarbonyl-methylamino]-3-hydroxypropanoate (Compound ST30) was then added again, and the mixture was stirred at room temperature for an additional 1 hour and 30 minutes. The reaction solution was cooled to 0°C, and then trifluoroacetic acid (2.4 mL) was added. After stirring the reaction solution at room temperature for 30 minutes, the reaction solution was purified by reverse phase silica gel column chromatography (0.05% aqueous trifluoroacetic acid solution/0.05% trifluoroacetic acid acetonitrile solution) to obtain the title compound (Compound ST31) (11.5 mg, 13% yield).
LCMS (ESI) m/z = 1037.5 (M-H)⁻
Retention time: 0.43 minutes (analytical condition SQDFA05)

### Synthesis of (2S,4R)-4-benzyl-1-[[4-[[2-(4-fluorophenyl)acetyl]amino]phenyl]methoxycarbonyl]pyrrolidine-2-carboxylic acid (Compound ST32)

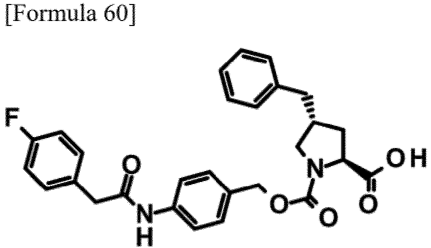

Under nitrogen atmosphere, 1,8-diazabicyclo[5.4.0]-7-undecene (DBU) (45.2 µL, 0.3 mmol) was added to a DMSO solution (1.0 ml) of (2S,4R)-4-benzyl-1-(9H-fluoren-9-ylmethoxycarbonyl)pyrrolidine-2-carboxylic acid (86.0 mg, 0.2 mmol), and the mixture was stirred at room temperature for 5 minutes. To this, (4-nitrophenyl)-4-(2-(4-fluorophenyl)acetamide)benzyl carbonate (85.0 mg, 0.2 mmol) synthesized by the method described in Patent Literature (WO 2018/143145) and DIPEA (70.0 µL, 0.4 mmol) were sequentially added, and the mixture was stirred at room temperature for 1 hour, and then purified by reverse phase silica gel column chromatography (0.1% aqueous formic acid solution/0.1% formic acid acetonitrile solution) to obtain the title compound (Compound ST32) (78 mg, 80% yield).
LCMS (ESI) m/z = 489.2 (M-H)⁻
Retention time: 0.81 minutes (analytical condition SQDFA05)

### Synthesis of cyanomethyl (2R)-2-[[4-[[2-(4-fluorophenyl)acetyl]amino]phenyl]methoxycarbonyl-methylamino]-3-hydroxypropanoate (Compound ST33)

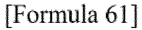

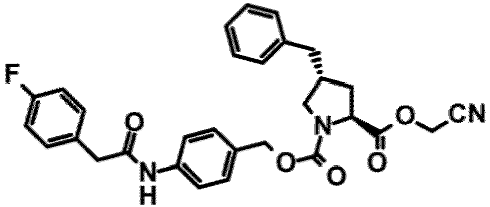

Under nitrogen atmosphere, 2-bromoacetonitrile (67.0 µL, 1.0 mmol) and DIPEA (19.0 µL, 0.11 mmol) were sequentially added to an acetonitrile solution (0.20 mL) of (2S,4R)-4-benzyl-1-[[4-[[2-(4-fluorophenyl)acetyl]amino]phenyl]methoxycarbonyl]pyrrolidine-2-carboxylic acid (Compound ST32) (49.0 mg, 0.10 mmol) at room temperature. After stirring the reaction mixture at room temperature for 1 hour, the reaction solution was concentrated to obtain the title compound (Compound ST33) as a crude product. The obtained crude product was dissolved in a mixed solution of acetonitrile/DMF = 1 : 1 (1.4 mL) and used as is in the next step.
LCMS (ESI) m/z = 528.4 (M-H)⁻
Retention time: 0.89 minutes (analytical condition SQDFA05)

### Synthesis of 2-O-[(2R,S,4R5R)-2-[[[(2R,3S,4R,5R)-5-(4-amino-2-oxopyrimidin-1-yl)-4-hydroxy-2-(phosphonooxymethyl)oxolan-3-yl]oxy-hydroxyphosphoryl]oxymethyl]-5-(6-aminopurin-9-yl)-4- hydroxyoxolan-3-yl] 1-O-[[4-[[2-(4-fluorophenyl)acetyl]amino]phenyl]methyl](2S,4R)-4-benzyl pyrrolidine-1,2-dicarboxylate (Compound ST34)

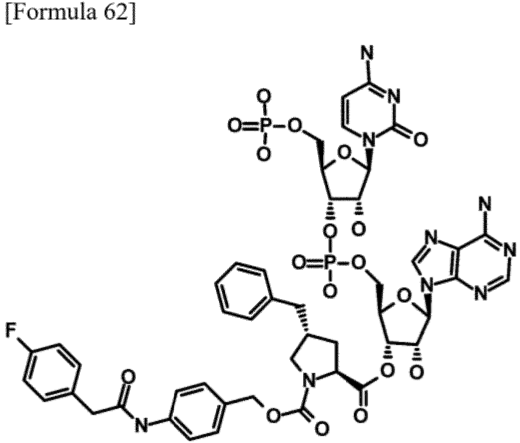

((2R,3R,4R,5R)-5-(4-Amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogen phosphate (36.1 mg, 0.05 mmol) synthesized by the method described in the Literature (Helv. Chim. Acta, 90, 297-310) was dissolved in a buffer solution A (28.0 mL), then a mixed solution of acetonitrile/DMF = 1 : 1 (0.7 mL, 0.05 mmol) of cyanomethyl (2R)-2-[[4-[[2-(4-fluorophenyl)acetyl]amino]phenyl]methoxycarbonyl-methylamino]-3-hydroxypropanoate (Compound ST33) was added, and the mixture was stirred at 30°C for 150 minutes. A mixed solution of acetonitrile/DMF = 1 : 1 (0.7 mL, 0.05 mmol) of cyanomethyl (2R)-2-[[4-[[2-(4-fluorophenyl)acetyl]amino]phenyl]methoxycarbonyl-methylamino]-3-hydroxypropanoate (Compound ST33) was then added again, and the mixture was stirred at 30°C for an additional 1 hour and 30 minutes. The reaction solution was cooled to 0°C, and then trifluoroacetic acid (1.4 mL) was added. After stirring the reaction solution at room temperature for 30 minutes, the reaction solution was purified by reverse phase silica gel column chromatography (0.05% aqueous trifluoroacetic acid solution/0.05% trifluoroacetic acid acetonitrile solution) to obtain the title compound (Compound ST34) (6.4 mg, 11% yield).
LCMS (ESI) m/z = 1123.7 (M-H)⁻
Retention time: 0.61 minutes (analytical condition SQDFA05)

### Synthesis of (2S,4R)-1-[[4-[[2-(4-fluorophenyl)acetyl]amino]phenyl]methoxycarbonyl]-4-phenoxypyrrolidine-2-carboxylic acid (Compound ST35)

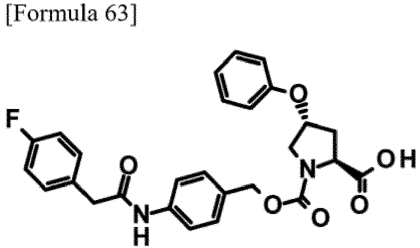

Under nitrogen atmosphere, 1,8-diazabicyclo[5.4.0]-7-undecene (DBU) (45.2 µL, 0.3 mmol) was added to a DMSO solution (1.0 ml) of (2S,4R)-1-(9H-fluoren-9-ylmethoxycarbonyl)-4-phenoxypyrrolidine-2-carboxylic acid (86.0 mg, 0.2 mmol) and the mixture was stirred at room temperature for 5 minutes. To this, (4-nitrophenyl)-4-(2-(4-fluorophenyl)acetamide)benzyl carbonate (85.0 mg, 0.2 mmol) synthesized by the method described in Patent Literature (WO 2018/143145) and DIPEA (70.0 µL, 0.4 mmol) were sequentially added, and the mixture was stirred at room temperature for 1 hour, and then purified by reverse phase silica gel column chromatography (0.1% aqueous formic acid solution/0.1% formic acid acetonitrile solution) to obtain the title compound (Compound ST35) (79 mg, 80% yield).
LCMS (ESI) m/z = 491.4 (M-H)⁻
Retention time: 0.79 minutes (analytical condition SQDFA05)

### Synthesis of 2-O-(cyanomethyl)1-O-[[4-[[2-(4-fluorophenyl)acetyl]amino]phenyl]methyl](2S,4R)-4-phenoxypyrrolidine-1,2-dicarboxylate (Compound ST36)

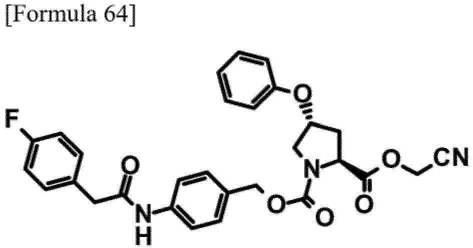

Under nitrogen atmosphere, 2-bromoacetonitrile (20.0 µL, 0.3 mmol) and DIPEA (7.86 µL, 0.05 mmol) were sequentially added to an acetonitrile solution (0.15 mL) of (2S,4R)-1-[[4-[[2-(4-fluorophenyl)acetyl]amino]phenyl]methoxycarbonyl}-4-phenoxypyrrolidine-2-carboxylic acid (Compound ST35) (15.0 mg, 0.03 mmol) at room temperature. After stirring the reaction mixture at room temperature for 1 hour, the reaction solution was concentrated to obtain the title compound (Compound ST36) as a crude product. The obtained crude product was dissolved in acetonitrile (0.75 mL) and used as is in the next step.
LCMS (ESI) m/z = 530.3 (M-H)⁻
Retention time: 0.87 minutes (analytical condition SQDFA05)

### Synthesis of 2-O-[(2R,3 S,4R,5R)-2-[[[(2R,3S,4R,5R)-5-(4-amino-2-oxopyrimidin-1-yl)-4-hydroxy-2-(phosphonooxymethyl)oxolan-3-yl]oxy-hydroxyphosphoryl]oxymethyl]-5-(6-aminopurin-9-yl)-4-hydroxyoxolan-3-yl] 1-O-[[4-[[2-(4-fluorophenyl)acetyl]aminolphenyl]methyl](2S,4R)-4-phenoxypyrrolidine-1,2-dicarboxylate (Compound ST37)

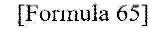

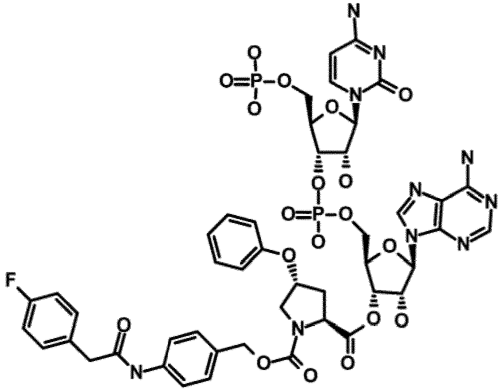

((2R,3R,4R,5R)-5-(4-Amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogen phosphate (32.5 mg, 0.05 mmol) synthesized by the method described in the Literature (Helv. Chim. Acta, 90, 297-310) was dissolved in a buffer solution A (15.0 mL), then an acetonitrile solution (0.75 mL, 0.03 mmol) of 2-O-(cyanomethyl)1-O-[[4-[[2-(4-fluorophenyl)acetyl]amino]phenyl]methyl](2S,4R)-4-phenoxypyrrolidine-1,2-dicarboxylate (Compound ST36) was added, and the mixture was stirred at 30°C for 120 minutes. The reaction solution was cooled to 0°C, and then trifluoroacetic acid (0.75 mL) was added. After stirring the reaction solution at room temperature for 30 minutes, the reaction solution was purified by reverse phase silica gel column chromatography (0.05% aqueous trifluoroacetic acid solution/0.05% trifluoroacetic acid acetonitrile solution) to obtain the title compound (Compound ST37) (4.9 mg, 15% yield).
LCMS (ESI) m/z = 1125.6 (M-H)⁻
Retention time: 0.60 minutes (analytical condition SQDFA05)

### Synthesis of (2S)-2-[[4-[[2-(4-fluorophenyl)acetyl]amino]phenyl]methoxycarbonyl-methylamino]propanoic acid (Compound ST38)

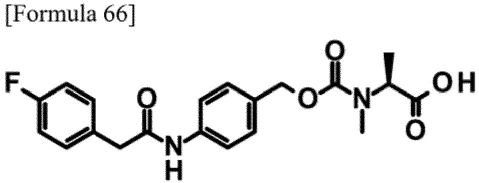

Under nitrogen atmosphere, N-methyl-alanine (15.5 mg, 0.15 mmol) and (4-nitrophenyl)-4-(2-(4-fluorophenyl)acetamide) benzyl carbonate (63.7 mg, 0.15 mmol) synthesized by the method described in Patent Literature (WO 2018/143145) were added to DMSO (0.38 mL), and the mixture was stirred at room temperature for 5 minutes. Triethylamine (46.0 µL, 0.33 mmol) was added at 0°C, and then the reaction mixture was stirred at 30°C for 3 hours and then purified by reverse phase silica gel column chromatography (0.1% aqueous formic acid solution/0.1% formic acid acetonitrile solution) to obtain the title compound (Compound ST38) (53 mg, 91% yield).
LCMS (ESI) m/z = 387.3 (M-H)⁻
Retention time: 0.69 minutes (analytical condition SQDFA05)

### Synthesis of cyanomethyl (2S)-2-[[4-[[2-(4-fluorophenyl)acetyl]amino]phenyl]methoxycarbonyl-methylamino]propanoate (Compound ST39)

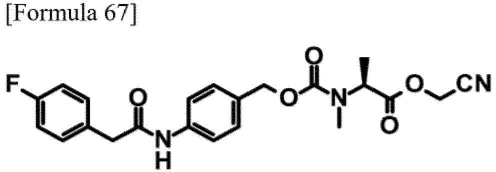

Under nitrogen atmosphere, 2-bromoacetonitrile (87.0 µL, 1.30 mmol) and DIPEA (34.0 µL, 0.20 mmol) were sequentially added to an acetonitrile solution (0.26 mL) of (2S)-2-[[4-[[2-(4-fluorophenyl)acetyl]amino]phenyl]methoxycarbonyl-methylamino]propanoic acid (Compound ST38) (50.0 mg, 0.13 mmol) at 0°C. After stirring the reaction mixture at room temperature for 1 hour, the reaction solution was concentrated to obtain the title compound (Compound ST39) as a crude product. The obtained crude product was dissolved in acetonitrile (2.50 mL) and used as is in the next step.
LCMS (ESI) *m*/*z* = 426.3 (M-H)⁻
Retention time: 0.77 minutes (analytical condition SQDFA05)

### Synthesis of [(2R,3S,4R,5R)-2-[[[(2R,3S,4R,5R)-5-(4-amino-2-oxopyrimidin-1-yl)-4-hydroxy-2-(phosphonooxymethyl)oxolan-3-yl]oxy-hydroxyphosphoryl]oxymethyl]-5-(6-aminopurin-9-yl)-4-hydroxyoxolan-3-yl] (2S)-2-[[4-[[2-(4-fluorophenyl)acetyl]amino]phenyl]methoxycarbonyl-methylamino]propanoate (Compound ST40)

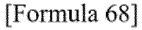

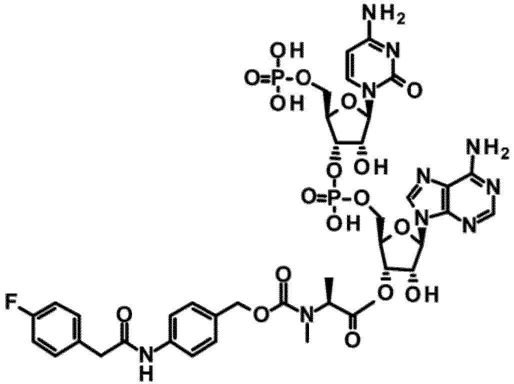

((2R,3R,4R,5R)-5-(4-Amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogen phosphate (94 mg, 0.13 mmol) synthesized by the method described in the Literature (Helv. Chim. Acta, 90, 297-310) was dissolved in a buffer solution A (50.0 mL), then an acetonitrile solution (2.50 mL, 0.13 mmol) of cyanomethyl (2S)-2-[[4-[[2-(4-fluorophenyl)acetyl]amino]phenyl]methoxycarbonyl-methylamino]propanoate (Compound ST39) was added, and the mixture was stirred at 30°C for 1 hour. The reaction solution was cooled to 0°C, and then trifluoroacetic acid (2.50 mL) was added. After stirring the reaction solution at 0°C for 1 hour, the reaction solution was purified by reverse phase silica gel column chromatography (0.05% aqueous trifluoroacetic acid solution/0.05% trifluoroacetic acid acetonitrile solution) to obtain the title compound (Compound ST40) (24.0 mg, 18% yield).
LCMS (ESI) m/z = 1021.6 (M-H)⁻
Retention time: 0.50 minutes (analytical condition SQDFA05)

### Synthesis of [(2R,3S,4R,5R)-2-[[[(2R,3S,4R,5R)-5-(4-amino-2-oxopyrimidin-1-yl)-4-hydroxy-2-(phosphonooxymethyl)oxolan-3-yl]oxy-hydroxyphosphoryl]oxymethyl]-5-(6-aminopurin-9-yl)-4-hydroxyoxolan-3-yl] (2S)-2-[[4-[[2-(4-fluorophenyl)acetyl]amino]phenyl]methoxycarbonyl-methylamino]butanoate (Compound ST41)

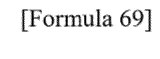

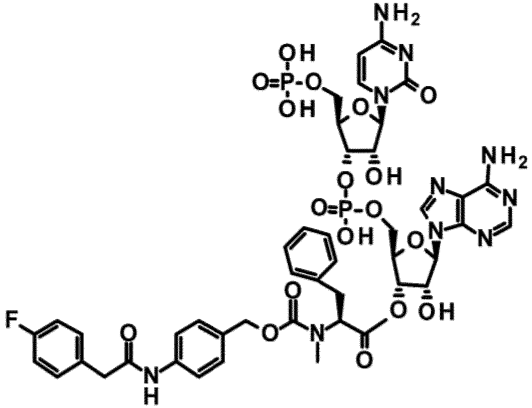

Compound ST41 was synthesized according to the method described in Patent Literature (WO 2018/225864).

### Synthesis of [(2R,3S,4R,5R)-2-[[[(2R,3S,4R,5R)-5-(4-amino-2-oxopyrimidin-1-yl)-4-hydroxy-2-(phosphonooxymethyl)oxolan-3-yl]oxy-hydroxyphosphoryl]oxymethyl]-5-(6-aminopurin-9-yl)-4-hydroxyoxolan-3-yl] (2S)-2-[3-(2,2-difluoro-10,12-dimethyl-3-aza-1-azonia-2-boranuidatricyclo[7.3.0.03,7]dodeca-1(12),4,6,8,10-pentaen-4-yl)propanoylaminol-3-phenylpropanoate (Compound ST42)

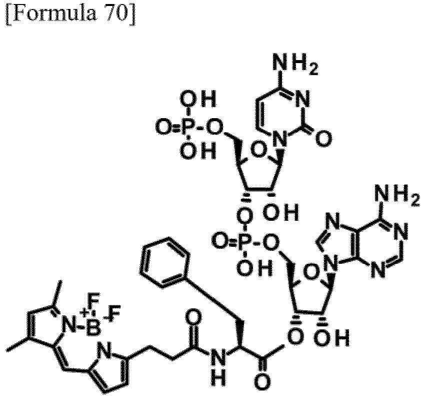

Compound ST42 was synthesized according to the method described in Patent Literature (WO 2020/138336).

### Example 2. Synthesis of peptide (LCT-12) used for LC/MS preparation

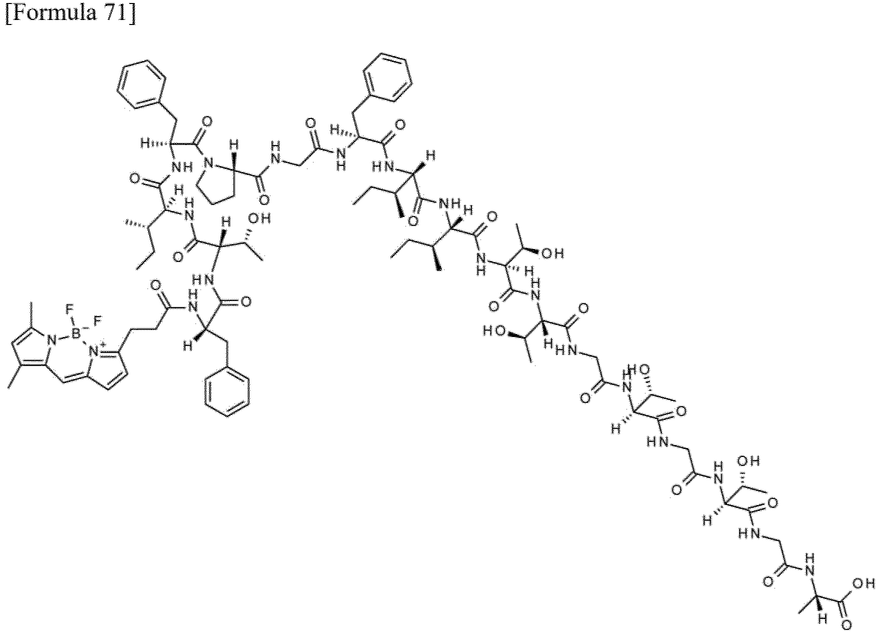

Compound LCT-12 was synthesized according to the method described in Patent Literature (WO 2020/138336).

### Example 3: Synthesis of aminoacyl tRNA

The following tRNAGluUAG (-CA) was prepared by the usual method.
SEQ ID NO: 1 (TR-1)
   tRNA(Glu)uag-CA RNA sequence: 
GUCCCCUUCGUCUAGAGGCCCAGGACACCGCCCUuagACGGCGGUAACAGGGGUUC
GAAUCCCCUAGGGGACGC
SEQ ID NO: 2 (TR-2)
   tRNA(Glu)cug-CA RNA sequence: 
GUCCCCUUCGUCUAGAGGCCCAGGACACCGCCCUcugACGGCGGUAACAGGGGUUC
GAAUCCCCUAGGGGACGC
SEQ ID NO: 3 (TR-3)
   tRNA (fMet)cau-CA RNA sequence:

GGCGGGGUGGAGCAGCCUGGUAGCUCGUCGGGCUcauAACCCGAAGAUCGUCGGUU
CAAAUCCGGCCCCCGCAAC

### Example 4. Preparation of aminoacyl tRNA mixed solution using aminoacyl pCpA

The reaction solution was prepared to be 25 µM transcribed tRNA (Glu)uag-CA (SEQ ID NO: 1), 50 mM HEPES-KOH pH 7.5, 20 mM MgCl₂, 1 mM ATP, 0.6 unit/µL T4 RNA ligase (New England Biolabs Inc.), and 0.25 mM aminoacyl pCpA (DMSO solution) by diluting them in a measuring flask with Nuclease free water, and the ligation reaction was performed at 15°C for 45 minutes. However, the reaction solution before the addition of T4 RNA ligase and aminoacyl pCpA was subjected to refolding of tRNA in advance by heating at 95°C for 2 minutes and then leaving at room temperature for 5 minutes.

Sodium acetate was added to the ligation reaction solution to be 0.3 mM, the mixture was subjected to phenol-chloroform extraction, and aminoacylated tRNA was recovered by ethanol precipitation. The obtained aminoacylated tRNA was dissolved in 1 mM sodium acetate just before the addition to the cell-free translation system.

### Compound AAtR-1 (nBuGly-tRNA(Glu)uag)

In the method described above, Compound ST01 was used as aminoacyl pCpA to prepare Compound AAtR-1 represented by the following formula.

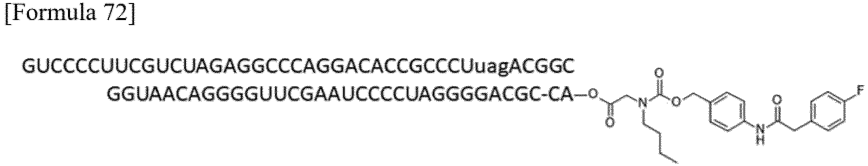

### Compound AAtR-2 (MeOEtGly-tRNA(Glu)uag)

In the method described above, Compound ST04 was used as aminoacyl pCpA to prepare Compound AAtR-2 represented by the following formula.

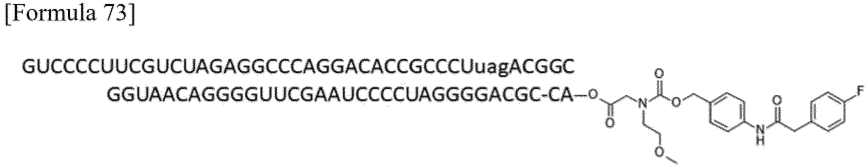

### Compound AAtR-3 (MeOPrGly-tRNA(Glu)uag)

In the method described above, Compound ST07 was used as aminoacyl pCpA to prepare Compound AAtR-3 represented by the following formula.

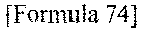

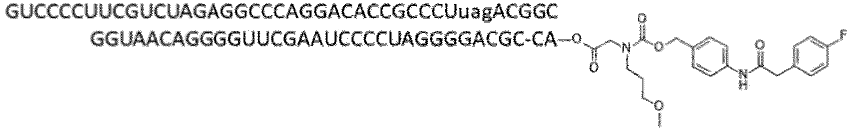

### Compound AAtR-4 (PhPrGly-tRNA(Glu)uag)

In the method described above, Compound ST10 was used as aminoacyl pCpA to prepare Compound AAtR-4 represented by the following formula.

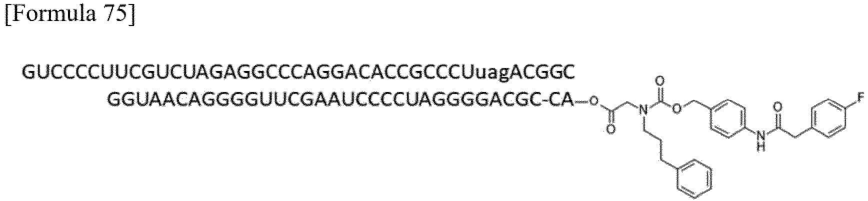

### Compound AAtR-5 (PhOEtGly-tRNA(Glu)uag)

In the method described above, Compound ST13 was used as aminoacyl pCpA to prepare Compound AAtR-5 represented by the following formula.

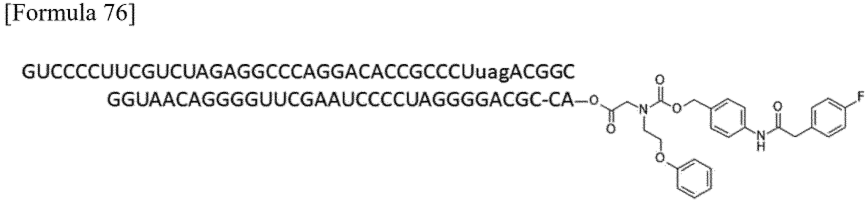

### Compound AAtR-6 (D-Nva-tRNA(Glu)uag)

In the method described above, Compound ST16 was used as aminoacyl pCpA to prepare Compound AAtR-6 represented by the following formula.

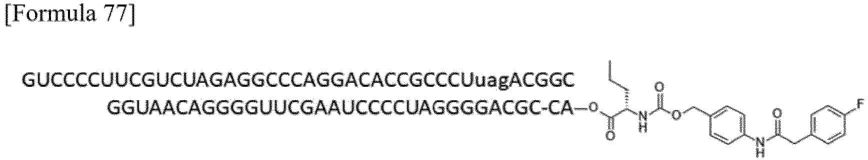

### Compound AAtR-7 (D-Ser(Me)-tRNA(Glu)uag)

In the method described above, Compound ST19 was used as aminoacyl pCpA to prepare Compound AAtR-7 represented by the following formula.

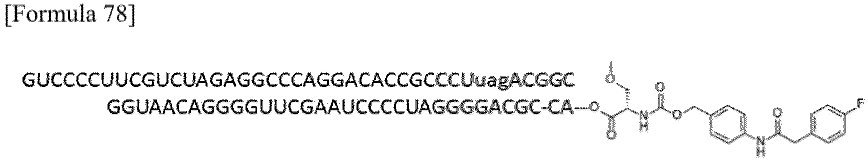

### Compound AAtR-8 (D-Hph-tRNA(Glu)uag)

In the method described above, Compound ST22 was used as aminoacyl pCpA to prepare Compound AAtR-8 represented by the following formula.

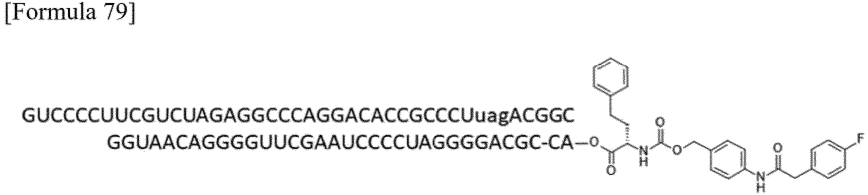

### Compound AAtR-9 (D-Ser(Ph)-tRNA(Glu)uag)

In the method described above, Compound ST25 was used as aminoacyl pCpA to prepare Compound AAtR-9 represented by the following formula.

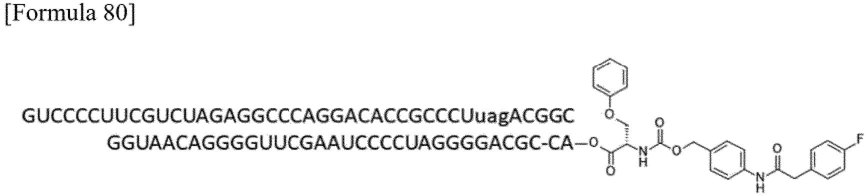

### Compound AAtR-10 (D-MeAb-u-tRNA(Glu)uag)

In the method described above, Compound ST28 was used as aminoacyl pCpA to prepare Compound AAtR-10 represented by the following formula.

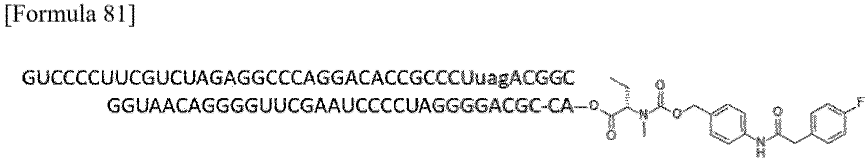

### Compound AAtR-11 (D-MeSer-tRNA(Glu)uag)

In the method described above, Compound ST31 was used as aminoacyl pCpA to prepare Compound AAtR-11 represented by the following formula.

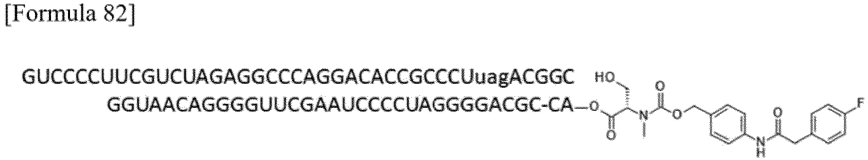

### Compound AAtR-12 (ProdRBn-tRNA(Glu)uag)

In the method described above, Compound ST34 was used as aminoacyl pCpA to prepare Compound AAtR-12 represented by the following formula.

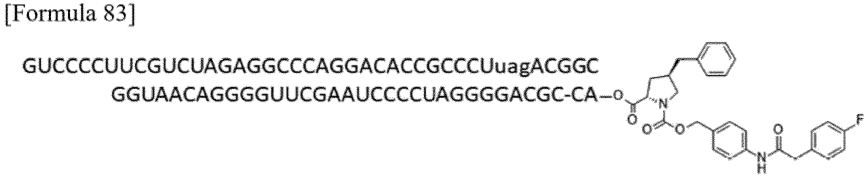

### Compound AAtR-13 (Hyp(Ph)-tRNA(Glu)uag)

In the method described above, Compound ST37 was used as aminoacyl pCpA to prepare Compound AAtR-13 represented by the following formula.

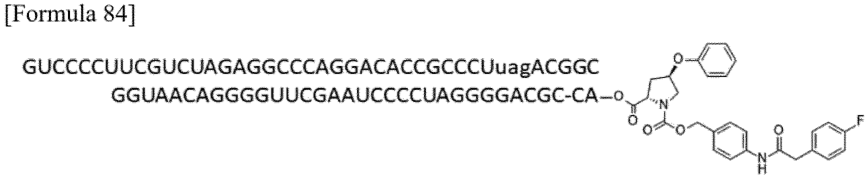

### Example 5. Preparation of aminoacyl tRNA mixed solution using aminoacyl pCpA

The reaction solution was prepared to be 25 µM transcribed tRNA (Glu)cug-CA (SEQ ID NO: 2), 50 mM HEPES-KOH pH 7.5, 20 mM MgCl₂, 1 mM ATP, 0.6 unit/µL T4 RNA ligase (New England Biolabs Inc.), and 0.25 mM aminoacyl pCpA (DMSO solution) by diluting them in a measuring flask with Nuclease free water, and the ligation reaction was performed at 15°C for 45 minutes. However, the reaction solution before the addition of T4 RNA ligase and aminoacyl pCpA was subjected to refolding of tRNA in advance by heating at 95°C for 2 minutes and then leaving at room temperature for 5 minutes.

Sodium acetate was added to the ligation reaction solution to be 0.3 mM, the mixture was subjected to phenol-chloroform extraction, and aminoacyl tRNA was recovered by ethanol precipitation. The obtained aminoacyl tRNA was dissolved in 1 mM sodium acetate just before the addition to the cell-free translation system.

### Compound AAtR-14 (MeAla-tRNA(Glu)cug)

In the method described above, Compound ST40 was used as aminoacyl pCpA to prepare Compound AAtR-14 represented by the following formula.

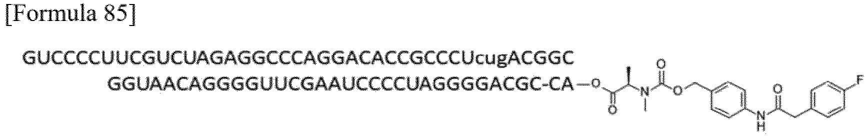

### Compound AAtR-15 (MePhe-tRNA(Glu)cug)

In the method described above, Compound ST41 was used as aminoacyl pCpA to prepare Compound AAtR-15 represented by the following formula.

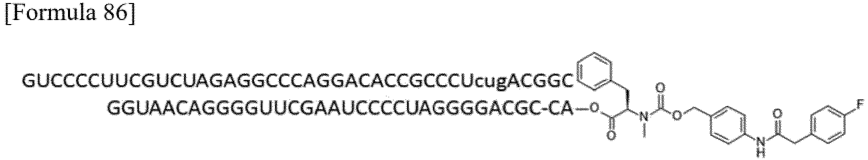

### Example 6. Preparation of initiator aminoacyl tRNA mixed solution using aminoacyl pCpA

The reaction solution was prepared to be 25 µM transcribed tRNA (fMet)cau-CA (SEQ ID NO: 3), 50 mM HEPES-KOH pH 7.5, 20 mM MgCl₂, 1 mM ATP, 0.6 unit/µL T4 RNA ligase (New England Biolabs Inc.), and 0.25 mM aminoacyl pCpA (DMSO solution of ST45) by diluting them in a measuring flask with Nuclease free water, and the ligation reaction was performed at 15°C for 45 minutes. However, the reaction solution before the addition of T4 RNA ligase and aminoacyl pCpA was subjected to refolding of tRNA in advance by heating at 95°C for 2 minutes and then leaving at room temperature for 5 minutes. Sodium acetate was added to the ligation reaction solution to be 0.3 mM, the mixture was subjected to phenol-chloroform extraction, and initiator aminoacyl tRNA (Compound AAtR-19) was recovered by ethanol precipitation. The obtained initiator aminoacyl tRNA was dissolved in 1 mM aqueous sodium acetate solution just before the addition to the cell-free translation system.

### Compound AATR-16 (BdpPhe-tRNA (fMet)cau)

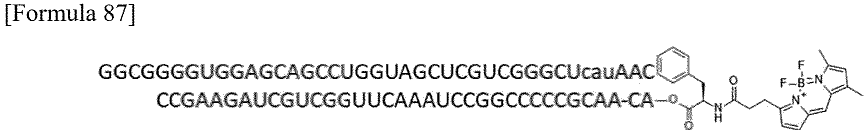

The corresponding relationship between compound number, aminoacylated tRNA, and amino acid is shown in Table 2.

**[Table 2]**

| Compound number | Aminoacylated tRNA | Amino acid notation |
|---|---|---|
| ST01 | AAtR-1 | nBuGly |
| ST04 | AAtR-2 | MeOEtGly |
| ST07 | AAtR-3 | MeOPrGly |
| ST10 | AAtR-4 | PhPrGly |
| ST13 | AAtR-5 | PhOEtGly |
| ST16 | AAtR-6 | D-Nva |
| ST19 | AAtR-7 | D-Ser(Me) |
| ST22 | AAtR-8 | D-Hph |
| ST25 | AAtR-9 | D-Ser(Ph) |
| ST28 | AAtR-10 | D-MeAbu |
| ST31 | AAtR-11 | D-MeSer |
| ST34 | AAtR-12 | Pro4RBn |
| ST37 | AAtR-13 | Hyp(Ph) |
| ST40 | AAtR-14 | MeAla |
| ST41 | AAtR-15 | MePhe |
| ST42 | AAtR-16 | BdpPhe |

The corresponding relationship between amino acid notation in Table 2 and chemical structure is shown in Table 3.

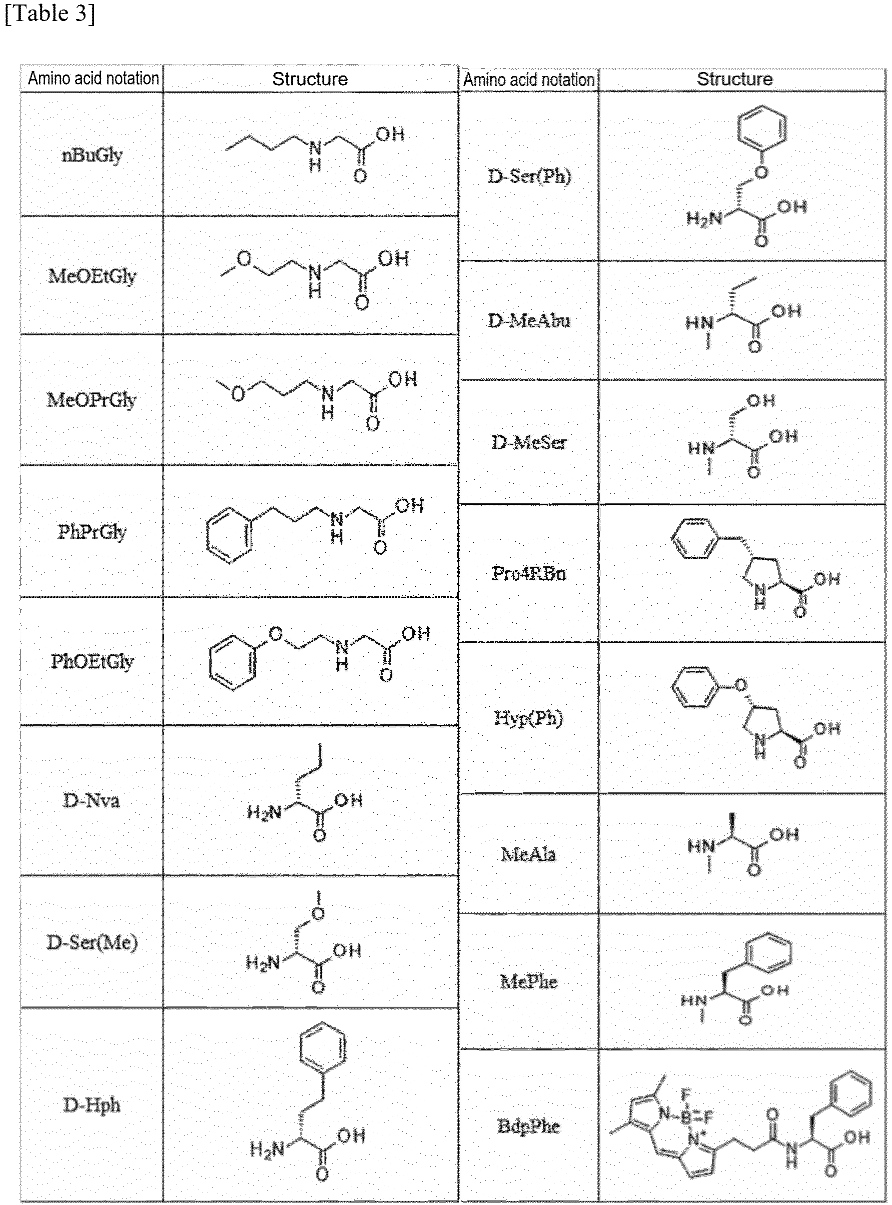

### Example 7. Preparation of mRNA

Template mRNA, sequence mR-1 or sequence mR-2 was synthesized from template DNA (SEQ ID NO: 4 or SEQ ID NO: 5) by in vitro transcription reaction using RiboMAX Large Scale RNA production System T7 (Promega Corporation, P1280), and purified by RNeasy Mini kit (QIAGEN N.V.).
Template DNA: D-1 (SEQ ID NO: 4) 
GTAATACGACTCACTATAGGGTTAACTTTAAGAAGGAGATATACATATGATTTTTAT
TGGTTTTATTATTctacagATTCCGGGTTAAGCTTCG
Template DNA: D-2 (SEQ ID NO: 5) 
GTAATACGACTCACTATAGGGTTAACTTTAAGAAGGAGATATACATATGATTTTTAT
TGGTTTTATTATTcagctaATTCCGGGTTAAGCTTCG
Template mRNA: mR-1 (SEQ ID NO: 6) 
GGGUUAACUUUAAGAAGGAGAUAUACAUAUGAUUUUUAUUGGUUUUAUUAUUcua
cagAUUCCGGGUUAAGCUUCG
Template mRNA: mR-2 (SEQ ID NO: 7)

GGGUUAACUUUAAGAAGGAGAUAUACAUAUGAUUUUUAUUGGUUUUAUUAUUcag
cuaAUUCCGGGUUAAGCUUCG

### Example 8. Translation synthesis of peptide

The translation system used was PURESYSTEM(R) (manufactured by BioComber Co., Ltd.), which is a reconstituted cell-free protein synthesis system derived from a prokaryote. Specifically, the translation system includes the following: 1 mM GTP, 1 mM ATP, 20 mM creatine phosphate, 50 mM HEPES-KOH pH 7.6, 100 mM potassium acetate, 2 mM spermidine, 1 mM dithiothreitol, 1.0 mg/ml E. coli MRE600 (RNase negative)-derived tRNA (F. Hoffmann-La Roche Ltd.)(Some of tRNA was removed by the method described in Non Patent Literature: (Yokogawa T, Kitamura Y, Nakamura D, Ohno S, Nishikawa K. 2010. Nucleic acids research 38: e89)), 3 µM in vitro transcribed E. coli tRNA AlalB, 0.26 µM EF-G, 4 µg/ml creatine kinase, 3 µg/ml myokinase, 2 unit/ml inorganic pyrophosphatase, 1.1 µg/ml nucleoside diphosphate kinase, 2.7 µM IF1, 0.4 µM IF2, 1.5 µM IF3, 40 µM EF-Tu, 35 µM EF-Ts, 1 µM EF-P-Lys, 0.4 unit/µl RNasein Ribonuclease inhibitor (Promega Corporation, N2111), 0.4 to 0.5 µM Penicillin G Amidase (PGA), 2.7 µM AlaRS, 1 µM GlyRS, 0.4 µM IleRS, 0.5 µM variant PheRS (WO 2016/148044), 0.16 µM ProRS, 0.09 µM ThrRS, 1 µM variant ValRS (WO 2016/148044), 1 µM variant SerRS (WO 2016/148044), 250 µM Gly, 250 µM Lys, 100 µM Ile, 250 µM Pro, 250 µM Thr, 5 mM N-methylalanine, 5 mM N-methylphenylalanine, 5 mM N-methylserine, and 5 mM N-methylvaline.

A translation reaction mixture was prepared by adding magnesium acetate to a translation solution such that the concentration of magnesium acetate was 4 mM, and 25 µM initiator aminoacylated tRNA (Compound AAtR-16) and 10 µM of each aminoacylated tRNA (any one of Compound AAtR-1 to Compound AAtR-13 and any one of Compound AAtR-14 and AAtR-15) were added. To the obtained mixture, 1 µM mRNA (mR-1 or mR-2) and 1.2 µM ribosomes were added, and the resulting mixture was allowed to stand at 37°C for 1 hour.

When mR-1 or mR-2 is used as the template mRNA, translation is initiated from the 29th-31st codon (AUG) to produce a translated peptide represented by BdpPhe-Ile-MePhe-Ile-Gly-MePhe-Ile-Ile-XI-X2-Ile-Pro-Gly (in one letter notation, BdpF-I-MeF-I-G-MeF-I-I-X1-X2-I-P-G, wherein X1 and X2 represent unnatural amino acid residues corresponding to the aminoacylated tRNA used).

### Example 9. Overview of Analysis

The solution containing the translated peptide obtained in Example 8 was diluted 10-fold and analyzed using a LC-FLR-MS device. For the analysis data, the peptide translation amount was evaluated by identifying the retention time of the translated peptide of interest based on the MS spectrum and calculating the peak area of the fluorescence absorption spectrum corresponding to the retention time. The evaluation of the peptide translation amount was calculated as the relative content based on the calibration curve prepared as the preparation using LCT12 synthesized in Example 2. LC-MS was performed under the analytical conditions shown in Table 4.

**[Table 4]**

| | |
|---|---|
| Device | Aquity UPLC-FLR-Xevo G2-XS Tof |
| Column | waters BEHC18 (2.1 x 50 mm, ϕ1.7 µm) |
| Mobile phase | A) 0.1% aqueous formic acid solution |
| | B) 0.1% formic acid acetonitrile solution |
| Gradient (A/B) | 0-0.2 min 10/90 |
| | 0.2-3.6 min 98/2 |
| | 3.6-4.0 min 10/90 |
| Flow rate (mL/min) | 0.5 |
| Column temperature (°C) | 40 |
| Detection wavelength (Ex/Em) | 491 nm/515 nm |
| MS mode | ESI- |

### Example 10. Translation results (1)

The translated peptide and translation amount each were evaluated using mR-1 as the template mRNA, and any one selected from Compounds AAtR-1 to AAtR-13 and Compound AAtR-14 as the aminoacylated tRNA. The results are shown in Table 5.

**[Table 5]**

| No. | Aminoacylated tRNA | Translated peptide compound | SEQ ID NO: | Translation amount (µM) | Retention time (min) | m/z [M-H] |
|---|---|---|---|---|---|---|
| 1 | AAtR-1 | BdpF-IMeFIGMeFII-nBuG-MeA-IPG | 8 | 0.26 | 3.27 | 1735.8 |
| | AAtR-14 | | | | | |
| 2 | AAtR-2 | BdpF-IMeFIGMeFII-MeOEtG-MeA-IPG | 9 | 0.88 | 3.1 | 1737.8 |
| | AAtR-14 | | | | | |
| 3 | AAtR-3 | BdpF-IMeFIGMeFII-MeOPrG-MeA-IPG | 10 | 0.11 | 3.11 | 1751.8 |
| | AAtR-14 | | | | | |
| 4 | AAtR-4 | BdpF-IMeFIGMeFII-PhPrG-MeA-IPG | 11 | 0.10 | 3.35 | 1797.9 |
| | AAtR-14 | | | | | |
| 5 | AAtR-5 | BdpF-IMeFIGMeFII-PhOEtG-MeA-IPG | 12 | 0.87 | 3.29 | 1799.9 |
| | AAtR-14 | | | | | |
| 6 | AAtR-6 | BdpF-IMeFIGMeFII-D-Nva-MeA-IPG | 13 | 0 | - | - |
| | AAtR-14 | | | | | |
| 7 | AAtR-7 | BdpF-IMeFIGMeFII-D-Ser(Me)-MeA-IPG | 14 | 0.17 | 3.01 | 1723.8 |
| | AAtR-14 | | | | | |
| 8 | AAtR-8 | BdpF-IMeFIGMeFII-D-Hph-MeA-IPG | 15 | 0.01 | 3.18 | 1783.9 |
| | AAtR-14 | | | | | |
| 9 | AAtR-9 | BdpF-IMeFIGMeFII-D-Ser(Ph)-MeA-IPG | 16 | 0.35 | 3.3 | 1785.9 |
| | AAtR-14 | | | | | |
| 10 | AAtR-10 | BdpF-IMeFIGMeFII-D-MeAbu-MeA-IPG | 17 | 0 | - | - |
| | AAtR-14 | | | | | |
| 11 | AAtR-11 | BdpF-IMeFIGMeFII-D-MeSer-MeA-IPG | 18 | 0.76 | 2.98 | 1723.8 |
| | AAtR-14 | | | | | |
| 12 | AAtR-12 | BdpF-IMeFIGMeFII-D-Pro(4R-Bn)-MeA-IPG | 19 | 0.01 | 3.28 | 1809.9 |
| | AAtR-14 | | | | | |
| 13 | AAtR-13 | BdpF-IMeFIGMeFII-Hyp(Ph)-MeA-IPG | 20 | 0.78 | 3.23 | 1811.9 |
| | AAtR-14 | | | | | |

### Example 11. Translation results (2)

The translated peptide and translation amount each were evaluated using mR-1 as the template mRNA, and any one selected from Compounds AAtR-1 to AAtR-13 and Compound AAtR-15 as the aminoacylated tRNA. The results are shown in Table 6.

**[Table 6]**

| No. | Aminoacylated tRNA | Translated peptide compound | SEQ ID NO: | Translation amount (µM) | Retention time (min) | m/z [M-H] |
|---|---|---|---|---|---|---|
| 14 | AAtR-1 | BdpF-IMeFIGMeFII-nBuG-MeF-IPG | 21 | 0.11 | 3.49 | 1811.9 |
| | AAtR-15 | | | | | |
| 15 | AAtR-2 | BdpF-IMeFIGMeFII-MeOEtG-MeF-IPG | 22 | 0.6 | 3.33 | 1813.9 |
| | AAtR-15 | | | | | |
| 16 | AAtR-3 | BdpF-IMeFIGMeFII-MeOPrG-MeF-IPG | 23 | 0.05 | 3.35 | 1827.9 |
| | AAtR-15 | | | | | |
| 17 | AAtR-4 | BdpF-IMeFIGMeFII-PhPrG-MeF-IPG | 24 | 0 | - | - |
| | AAtR-15 | | | | | |
| 18 | AAtR-5 | BdpF-IMeFIGMeFII-PhOEtG-MeF-IPG | 25 | 0.52 | 3.49 | 1875.9 |
| | AAtR-15 | | | | | |
| 19 | AAtR-6 | BdpF-IMeFIGMeFII-D-Nva-MeF-IPG | 26 | 0 | - | - |
| | AAtR-15 | | | | | |
| 20 | AAtR-7 | BdpF-IMeFIGMeFII-D-Ser(Me)-MeF-IPG | 27 | 0.17 | 3.22 | 1799.9 |
| | AAtR-15 | | | | | |
| 21 | AAtR-8 | BdpF-IMeFIGMeFII-D-Hph-MeF-IPG | 28 | 0 | - | - |
| | AAtR-15 | | | | | |
| 22 | AAtR-9 | BdpF-1MeFIGMeFII-D-Ser(Ph)-MeF-IPG | 29 | 0.44 | 3.53 | 1861.9 |
| | AAtR-5 | | | | | |
| 23 | AAtR-10 | BdpF-IMeFIGMeFII-D-MeAbu-MeF-IPG | 30 | 0 | - | - |
| | AAtR-15 | | | | | |
| 24 | AAtR-11 | BdpF-IMeFIGMeFII-D-MeSer-MeF-IPG | 31 | 0.38 | 3.17 | 1799.9 |
| | AAtR-15 | | | | | |
| 25 | AAtR-12 | BdpF-IMeFIGMeFII-D-Pro(4R-Bn)-MeF-IPG | 32 | 0 | - | - |
| | AAtR-15 | | | | | |
| 26 | AAtR-13 | BdpF-IMeFIGMeFII-Hyp(Ph)-MeF-IPG | 33 | 0.51 | 3.53 | 1887.9 |
| | AAtR-15 | | | | | |

### Example 12. Translation results (3)

The translated peptide and translation amount each were evaluated using mR-2 as the template mRNA, and Compound AAtR-4 or AAtR-5 and Compound AAtR-14 or AAtR-15 as the aminoacylated tRNA. The results are shown in Table 7.

**[Table 7]**

| No. | Aminoacylated tRNA | Translated peptide compound | SEQ ID NO: | Translation amount (µM) | Retention time (min) | m/z [M-H] |
|---|---|---|---|---|---|---|
| 27 | AAtR-4 | BdpF-IMeFIGMeFII-MeA-PhPrG-IPG | 34 | 0.87 | 3.25 | 1797.9 |
| | AAtR-14 | | | | | |
| 28 | AAtR-5 | BdpF-IMeFIGMeFII-MeA-PhOEtG-IPG | 35 | 0.86 | 3.2 | 1799.9 |
| | AAtR-14 | | | | | |
| 29 | AAtR-4 | BdpF-IMeFIGMeFII-MeF-PhPrG-IPG | 36 | 0.74 | 3.39 | 1874.0 |
| | AAtR-15 | | | | | |
| 30 | AAtR-5 | BdpF-IMeFIGMeFII-MeF-PhOEtG-IPG | 37 | 0.85 | 3.33 | 1875.9 |
| | AAtR-15 | | | | | |

The translation results of Examples 10 to 13 are shown in Fig. 1.

[Sequence Listing]

## Claims

1. A method for producing a peptide, comprising a step of translating a nucleic acid that comprises a codon encoding a first unnatural amino acid and a codon encoding a second unnatural amino acid which are adjacent to each other, wherein
at least one of the first unnatural amino acid and the second unnatural amino acid is an unnatural amino acid U1, and the unnatural amino acid U1 is an unnatural amino acid that meets the following requirement (A) and meets at least one selected from the group consisting of the following requirements (B) and (C):
(A) a nitrogen atom constituting a main-chain amino group (nitrogen atom N1) and an oxygen atom other than an oxygen atom constituting a main-chain carboxy group (oxygen atom O1) are bound via two atoms to each other;
(B) the oxygen atom O1 constitutes an alkoxy group or an aryloxy group;
(C) the nitrogen atom N1 is bound to a linear or branched C₁-C₆ alkyl group.

2. The method according to claim 1, wherein the oxygen atom O1, together with a linear or branched C₁-C₆ alkyl group, a C₃-C₁₀ cycloalkyl group, a C₆-C₁₀ aryl group, a C₇-C₁₂ arylalkyl group, or a C₇-C₁₂ alkylaryl group, constitutes a corresponding C₁-C₆ alkoxy group, a corresponding C₃-C₁₀ cycloalkoxy group, a corresponding C₆-C₁₀ aryloxy group, a corresponding C₇-C₁₂ arylalkoxy group, or a corresponding C₇-C₁₂ alkylaryloxy group.

3. The method according to claim 1 or 2, wherein the nitrogen atom N1 is substituted with a methyl group or an ethyl group.

4. The method according to any one of claims 1 to 3, wherein the unnatural amino acid U1 is an α-amino acid.

5. A method for producing a peptide, comprising a step of translating a nucleic acid that comprises a codon encoding a first unnatural amino acid and a codon encoding a second unnatural amino acid which are adjacent to each other, wherein
at least one of the first unnatural amino acid and the second unnatural amino acid is an unnatural amino acid U1, and the unnatural amino acid U1 is an unnatural amino acid represented by the following formula (I): 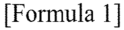 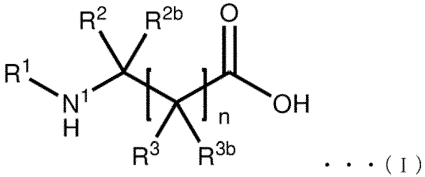 wherein n is an integer of 0 or more and 2 or less;
R¹ is a group represented by formula (II), a hydrogen atom or a linear or branched C₁-C₆ alkyl group; 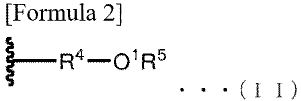
R² and R³ are each independently (a) a group represented by the formula (II), (b) a hydrogen atom, or (c) a linear or branched C₁-C₄ alkyl group;
R^{2b} and R^{3b} are each independently a hydrogen atom or a linear or branched C₁-C₄ alkyl group;
R² and R^{2b} or R³ and R^{3b} may form a ring together with a carbon atom to which they are bound;
any one or more of R¹, R², and R³ is a group represented by the formula (II);
N¹ (nitrogen atom N1) in the formula (I) and O¹ (oxygen atom O1) in the formula (II) are bound via two atoms to each other;
when R¹ is a substituent represented by the formula (II), R⁴ and R² may form a ring together with a carbon atom to which R² is bound and N¹;
when a plurality of R³ is present, they are each independently the same as or different from each other;
when a plurality of R^{3b} is present, they are each independently the same as or different from each other;
in the formula (II), 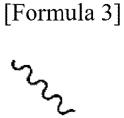 represents a binding position with N¹;
R⁴ is a C₁-C₂ alkylene group;
R⁵ is a hydrogen atom, a linear or branched C₁-C₆ alkyl group, a C₃-C₁₀ cycloalkyl group, a C₆-C₁₀ aryl group, a C₇-C₁₂ arylalkyl group, or a C₇-C₁₂ alkylaryl group; and
when R² is a substituent represented by the formula (II), at least one of R¹ in the formula (I) and R⁵ in the formula (II) is not a hydrogen atom.

6. The method according to claim 5, wherein the unnatural amino acid U1 is an unnatural amino acid represented by the following formula (III), (IV) or (V): 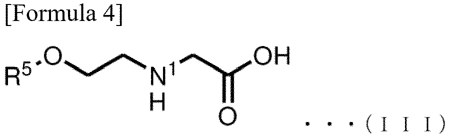 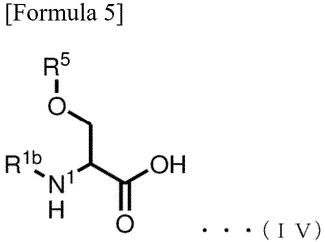 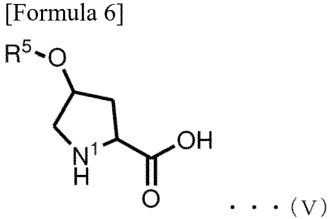 in the formulas (III), (IV) and (V), R^{1b} is a hydrogen atom or a linear or branched C₁-C₆ alkyl, and R⁵ has the same meaning as R⁵ in the formula (II).

7. The method according to claim 5 or 6, wherein R⁵ is a linear or branched C₁-C₆ alkyl group, a C₆-C₁₀ aryl group, or a C₇-C₁₂ arylalkyl group.

8. The method according to any one of claims 5 to 7, wherein R¹ and R^{1b} each are a methyl group or an ethyl group.

9. The method according to any one of claims 5 to 8, wherein n is 0.

10. The method according to any one of claims 1 to 9, wherein the first unnatural amino acid is the unnatural amino acid U1, and the second unnatural amino acid is an unnatural amino acid that is different from the unnatural amino acid U1 (unnatural amino acid U2).

11. The method according to any one of claims 1 to 10, wherein a codon encoding the first unnatural amino acid is translated before a codon encoding the second unnatural amino acid.

12. The method according to any one of claims 1 to 11, wherein the nitrogen atom N1 and the oxygen atom O1 are bound via two carbon atoms to each other.

13. The method according to any one of claims 1 to 12, wherein the unnatural amino acid U1 does not comprise a heteroatom other than a nitrogen atom and an oxygen atom.

14. The method according to claim 10, wherein the unnatural amino acid U2 is an unnatural amino acid represented by the following formula (VI): 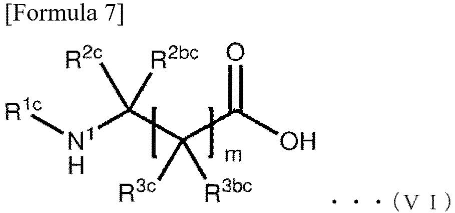 in the formula (VI),
m is an integer of 0 or more and 2 or less;
R^{1c} is a hydrogen atom or a linear or branched C₁-C₆ alkyl group;
R^{2c} is a hydrogen atom, a linear or branched C₁-C₆ alkyl group that may be substituted with a 5- to 10-membered aryl group, or a 5- to 10-membered aryl group that may be substituted with a linear or branched C₁-C₆ alkyl group;
R^{2bc} is a hydrogen atom or a linear or branched C₁-C₄ alkyl group;
R^{2c} and R^{2bc} may form a ring together with a carbon atom to which they are bound; and
a nitrogen atom to which R^{1c} is bound, R^{1c}, a carbon atom to which R^{2c} is bound, and R^{2c} may together form a ring.

15. A method for producing a peptide complex, comprising the method according to any one of claims 1 to 14.
